# FASCICULE DE BREVET EUROPEEN

(11) **EP 1 244 627 B1**
(45) Date de publication et mention de la délivrance du brevet: **20.09.2006**
(21) Numéro de dépôt: 01903877.7
(22) Date de dépôt: 05.01.2001
(51) Int. Cl.: C07D 213/81, A01N 43/40

(54) **DERIVES DE L'ACIDE PICOLINIQUE ET LEUR UTILISATION COMME FONGICIDES**
PICOLINSÄURE-DERIVATE UND IHRE VERWENDUNG ALS FUNGIZIDE
PICOLINIC ACID DERIVATIVES AND THEIR USE AS FUNGICIDES

(30) Priorité: 06.01.2000 FR 0000140
(43) Date de publication de la demande: 02.10.2002
(73) Titulaire: Bayer CropScience S.A., 69009 Lyon (FR)
(72) Inventeur: NIETO-ROMAN, Francisco, 34005 Palencia (ES); VORS, Jean-Pierre, 69009 Lyon (FR); VILLIER, Alain, 69370 Saint Didier au Mont d'Or (FR); LACHAISE, Hélène, 69009 Lyon (FR); MOUSQUES, Adeline, 69009 Lyon (FR); HARTMANN, Benoît, 69110 Sainte-Foy-Lès-Lyon (FR); HUTIN, Pierre, 69002 Lyon (FR); MOLINA, Jose Lorenzo, 69100 Villeurbanne (FR); MULLER, Benoît, 69002 Lyon (FR)
(74) Mandataire: Nowak, Alexander
(86) Numéro de dépôt international: PCT/FR2001/000033
(87) Numéro de publication internationale: WO 2001/049666

(56) Documents cités:
- WO-A-00/26191
- WO-A-01/05769
- WO-A-01/14339
- WO-A-95/25723
- DE-A- 19 958 166
- US-A- 3 228 950
- DATABASE WPI Section Ch, Week 199944 Derwent Publications Ltd., London, GB; Class B03, AN 1999-522674 XP002150334 & JP 11 228542 A (MEIJI SEIKA KAISHA LTD), 24 août 1999 (1999-08-24) cité dans la demande

## Description

La présente invention concerne de nouveaux dérivés de l'acide picolinique, leur procédé de préparation, leur utilisation comme fongicides, notamment sous forme de compositions fongicides, et les procédés de contrôle des champignons phytopathogènes des cultures à l'aide de ces composés ou de ces compositions.

Des dérivés de l'acide picolinique à action fongicide sont connus dans la littérature Ainsi, l'antimycine et certains de ses dérivés, décrits notamment dans la demande de brevet WO-A-99/11127 et par Kuzo SHIBATA et coll. (*The Journal of Antibiotics, **51*** (12), (1998), 1113-1116), sont présentés comme efficaces contre les champignons phytopathogènes des plantes, avec une bonne efficacité. Ces composés, de même que ceux décrits dans le brevet US-A-3,228,950 ne possèdent pas de substituants en position 4 du noyau pyridine.

La demande de brevet WO-A-00/26191 présente des dérivés de picolinamide éventuellement substitués en position 4 par le radical méthoxy. La demande de brevet WO-A-95/25723 propose quant à elle des dérivés de l'acide 3-pyridylcarboxylique.

Ces composés connus présentent toutefois le désavantage d'être des produits toxiques, ceci interdisant toute utilisation de ces composés en agriculture pour l'éradication des maladies phytopathogènes des cultures. De plus, ces composés sont issus de moûts de fermentation et possèdent des structures chimiques relativement complexes. Ainsi, les préparations et purifications des ces composés restent des opérations délicates et coûteuses, rendant toute synthèse industrielle et mise sur le marché peu rentables.

Des dérivés amides de l'acide picolinique sont également connus par la publication de la demande de brevet JP-11228542. Ces dérivés sont présents comme ayant des activités potentielles antifongiques et de faible toxicité pour être employés dans des produits pharmaceutiques. D'autres dérivés de l'acide picolinique sont également connus par la demande de brevet EP-A-0 690 061 dans laquelle de tels composés sont utilisés comme intermédiaires de synthèse pour la préparation de pyridothiadiazoles.

Ainsi, un premier objet de la présente invention consiste à proposer une nouvelle famille de dérivés de l'acide picolinique ne possédant pas les inconvénients cités précédemment.

Un autre objet de la présente invention est de proposer une nouvelle famille de composés ayant une activité améliorée, tant quantitativement que qualitativement, par rapport aux fongicides connus, antimycine ou dérivés. Par activité, on entend une activité fongicide ; par amélioration quantitative, on entend un meilleur contrôle des champignons phytopathogènes sur les cultures, et, par amélioration qualitative, un spectre d'action plus large, c'est-à-dire un contrôle d'une plus grande variété de champignons phytopathogènes des cultures.

Un autre objet de la présente invention est de proposer une nouvelle famille de composés ayant une toxicité et/ou phytotoxicité et/ou écotoxicité améliorées par rapport aux composés fongicides connus, notamment l'antimycine et ses dérivés.

Un autre objet de la présente invention est de proposer une nouvelle famille de composés possédant les caractéristiques présentées ci-dessus, notamment sur les cultures comme les céréales, le riz, le maïs, les arbres fruitiers, les arbres forestiers, la vigne, les cultures oléagineuses, les cultures protéagineuses, les cultures maraîchères, les solanées, la betterave, le lin, les agrumes, le bananier, les cultures ornementales et le tabac.

Un autre objet de la présente invention est de proposer une nouvelle famille de dérivés de l'acide picolinique possédant les caractéristiques présentées ci-dessus, notamment sur les cultures comme les céréales, le riz, le maïs, les arbres fruitiers, les arbres forestiers, la vigne, les cultures oléagineuses, les cultures protéagineuses, les cultures maraîchères, les solanées, la betterave, le lin, les agrumes, le bananier, les cultures ornementales et le tabac.

Un autre objet de la présente invention consiste également à proposer une nouvelle famille de dérivés de l'acide picolinique utile pour le traitement et la protection du bois de construction contre les maladies fongiques. En effet, le bois de construction utilisé par exemple pour la fabrication de meubles et bâtiments (charpentes, murs, plafond, sols, etc.), peut subir différents dommages, entre autres, de la part de champignons phytopathogènes. Les composés selon la présente invention permettent également de lutter contre ces attaques.

Un autre objet de la présente invention consiste à proposer une nouvelle famille de dérivés de l'acide picolinique utile en thérapeutique humaine et animale. En effet, de part leurs propriétés antifongiques, les dérivés de l'acide picolinique objets de la présente invention peuvent s'avérer utiles pour le traitement, chez l'homme comme chez l'animal, des maladies fongiques, comme par exemple les mycoses et les candidoses.

De façon surprenante, il a été trouvé que ces objets peuvent être réalisés en totalité ou en partie, par des dérivés de l'acide picolinique tels que ceux décrits dans la présente invention.

### Définition générale de l'invention

L'invention concerne des dérivés de l'acide picolinique, de formule générale (I) : 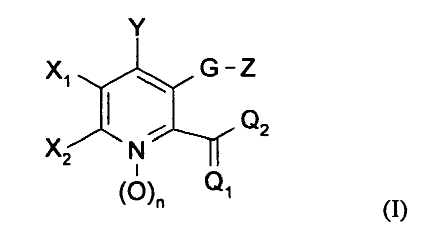 dans lesquels :
- G représente un atome d'oxygène ou de soufre,
- n représente 0 ou 1,
- Q₁ est un d'oxygène,
- Q₂ est le groupe -NR₄R₅,
- Z est choisi parmi l'atome d'hydrogène, le radical cyano, un radical alkyle, allyle, aryle, arylalkyle, propargyle, cycloalkyle, halocycloalkyle, alcényle, alcynyle, cyanoalkyle, haloalkyle, alkoxyalkyle, haloalkoxyalkyle, alkylthioalkyle, haloalkylthioalkyle, N-alkylaminoalkyle, N,N-dialkylaminoalkyle, acylaminoalkyle, alkoxycarbonylaminoalkyle, aminocarbonylaminoalkyle, alkoxycarbonyle, N-alkylamino-carbonyle, N,N-dialkylaminocarbonyle, acyle, thioacyle, alkoxythiocarbonyle, N-alkylaminothiocarbonyle, N,N-dialkylaminothiocarbonyle, alkylsulfinyle, haloalkylsulfinyle, alkylsulfonyle, haloalkylsulfonyle, alkoxysulfonyle, aminosulfonyle, N-alkylaminosulfonyle, N,N-dialkylaminosulfonyle, arylsulfinyle, arylsulfonyle, aryloxysulfonyle, N-arylaminosulfonyle, N,N-diarylaminosulfonyle, et N,N-arylalkylaminosulfonyle ;
- Y est choisi parmi un atome d'halogène, le radical hydroxy, mercapto, nitro, thiocyanato, azido, cyano, pentafluorosulfonyle, un radical alkyle, haloalkyle, alkylthio, haloalkylthio, alkoxyalkyle, haloalkoxyalkyle, alkylthioalkyle, haloalkylthioalkyle, cyanoalkyle, cyanoalkoxy, cyanoalkylthio, alkylsulfinyle, haloalkylsulfinyle, alkylsulfonyle, haloalkylsulfonyle, alkoxysulfonyle, un groupe cycloalkyle, halocycloalkyle, alcényle, alcynyle, alcényloxy, alcynyloxy, alcénylthio, alcynylthio, un groupe amino, N-alkylamino, N,N-dialkylamino, -NHCOR₁₀, -NHCSR₁₀, N-alkylaminocarbonylamino, N,N-dialkylaminocarbonyl-amino, aminoalkyle, N-alkyl-aminoalkyle, N,N-dialkylaminoalkyle, acylaminoalkyle, thioacylamino, alkoxythiocarbonylamino, N-alkyl-aminothiocarbonylamino, N,N-dialkylaminothiocarbonylamino, N,N-arylalkylaminocarbonylamino, N-alkylsulfinylamino, N-alkyl-sulfonylamino, N-alkyl-(alkylsulfonyl)amino, N-arylsulfinylamino, N-arylsulfonylamino, N-alkoxysulfonylamino, N-alkoxysulfinylamino, N-haloalkoxysulfinyl-amino, N-haloalkoxysulfonylamino, N-arylamino, N,N-diarylamino, arylcarbonylamino, alkoxy-carbonylamino, N-arylaminocarbonylamino, N,N-diarylamino-carbonylamino, arylthiocarbonylamino, aryloxythiocarbonylamino, N-arylaminothiocarbonylamino, N,N-diarylaminothiocarbonylamino, N,N-arylalkylaminothiocarbonylamino, un radical acyle, carboxy, carbamoyle, N-alkylcarbamoyle, N,N-dialkylcarbamoyle, alkoxycarbonyle inférieur, N-arylcarbamoyle, N,N-diarylcarbamoyle, aryloxycarbonyle, N,N-arylalkylcarbamoyle, et un groupe imino de formule : 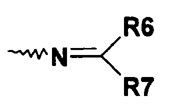
- X₁ et X₂ sont identiques ou différents et sont choisis indépendamment l'un de l'autre parmi l'atome d'hydrogène, un atome d'halogène, le radical hydroxy, mercapto, nitro, thiocyanato, azido, cyano, pentafluorosulfonyle, un radical alkyle, haloalkyle, alkoxy, haloalkoxy, alkylthio, haloalkylthio, alkoxyalkyle, haloalkoxyalkyle, alkylthioalkyle, haloalkylthioalkyle, cyanoalkyle, cyanoalkoxy, cyanoalkylthio, alkylsulfinyle, haloalkylsulfinyle, alkylsulfonyle, haloalkylsulfonyle, et alkoxysulfonyle, ou bien
- X₁ et X₂ peuvent également être joints ensemble, formant ainsi un cycle de 4 à 8 chaînons, saturé, partiellement insaturé ou totalement insaturé, et comportant éventuellement un ou plusieurs hétéroatomes choisis parmi le soufre, l'oxygène, l'azote et le phosphore,
- R₄ et R₅ sont identiques ou différents et sont choisis indépendamment l'un de l'autre parmi l'atome d'hydrogène et un radical aryle éventuellement substitué par un ou plusieurs radicaux choisi indépendemment les uns des autres parmi R₉, arylalkyle et un groupement -T-R₈,
- T représente une liaison directe ou un radical divalent choisi parmi un radical -(CH₂)ₘ-, m prenant une valeur comprise entre 1 et 12, bornes incluses, le dit radical étant éventuellement interrompu ou borné par un ou deux hétéroatomes choisis parmi l'azote, l'oxygène et/ou le soufre, un radical oxyalkylène, alkoxyalkylène, carbonyle (-CO-), oxycarbonyle(-O-CO-), carbonyloxy(-CO-O-), sulfinyle (-SO-), sulfonyle (-SO₂-) oxysulfonyle (-O-SO₂-) sulfonyloxy (-SO₂-O-), oxysulfinyle (-O-SO-), sulfinyloxy (-SO-O-), thio (-S-), oxy (-O-), vinyle (-C=C-), éthinyle (-C≡C-), -NR₉-, -NR₉O-, -ONR₉-, -N=N-, -NR₉-NR₁₀-, -NR₉-S-, -NR₉-SO-, -NR₉-SO₂-, -S-NR₉-, -SO-NR₉-, -SO₂-NR₉-, -CO-NR₉-O-, et -O-NR₉-CO-,
- R₈ est choisi parmi l'atome d'hydrogène, un radical aryle, ou hétérocyclyle,
- R₉ et R₁₀, identiques ou différents, sont choisis indépendamment l'un de l'autre parmi l'atome d'hydrogène, un atome d'halogène, le radical hydroxy, mercapto, nitro, thiocyanato, azido, cyano ou pentafluorosulfonyle, un radical alkyle, haloalkyle, alkoxy, haloalkoxy, alkylthio, haloalkylthio, alkoxyalkyle, haloalkoxyalkyle, alkylthioalkyle, haloalkylthioalkyle, arylalkyle, cyanoalkyle, cyanoalkoxy, cyanoalkylthio, alkylsulfinyle, haloalkylsulfinyle, alkylsulfonyle, haloalkylsulfonyle, et alkoxysulfonyle,
ainsi que les éventuels N-oxydes, isomères géométriques et/ou optiques, énantiomères et/ou diastéréoisomères, formes tautomères, les sels, complexes métalliques et métalloïdiques des composés de formule (I) tels qu'ils viennent d'être définis,
avec la restriction que lorsque G-Z est un groupe hydroxy, R₄ est un atome d'hydrogène et R₅ représente le groupe 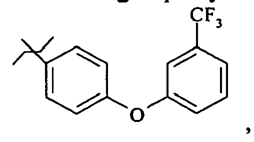 alors Y ne peut être ni un groupe methyl ni un groupe methylthio.

Les formes tautomères des composés de formule (I) tels que définis précédemment, sont également comprises dans l'invention. Par formes tautomères, on entend toutes formes isomériques décrites dans l'ouvrage "The tautomerism of heterocycles, Advances in Heterocyclic Chemistry, Supplement 1" par J. Elguero, C. Martin, A.R. Katritzky et P. Linda, édité par Academic Press, New York, 1976, pages 1-4.

Par ailleurs, les termes génériques suivants sont utilisés avec les significations suivantes :
- l'atome d'halogène signifie l'atome de fluor, de chlore, de brome ou d'iode,
- les radicaux alkyle, ainsi que les groupements comportant ces radicaux alkyle (alkoxy, alkylcarbonyle ou acyle, etc.) comportent, sauf indication contraire, de 1 à 6 atomes de carbone en chaîne linéaire ou ramifiée et sont éventuellement substitués,
- les radicaux alkyle, alkoxy et halocycloalkyle halogénés peuvent comporter un ou plusieurs atomes d'halogènes identiques ou différents,
- les radicaux cycloalkyle comportent de 3 à 6 atomes de carbone et sont éventuellement substitués,
- les radicaux alcényle et alcynyle, ainsi que les groupements comportant ces radicaux, comportent, sauf indication contraire, de 2 à 6 atomes de carbone en chaîne linéaire ou ramifiée et sont éventuellement substitués,
- le radical acyle signifie alkylcarbonyle, ou cycloalkylcarbonyle, la partie alkyle contenant de 1 à 6 atomes de carbone et la partie cycloalkyle comprenant de 3 à 6 atomes de carbone, sauf précisions contraires et sont éventuellement substitués,
- le radical alkylène désigne le radical bivalent -(CH₂)ₘ- où m représente un entier égal à 1,2, 3, 4, 5 ou 6,
- le terme "aryl", dans "aryle" et "arylalkyle" signifie phényle ou naphtyle, éventuellement substitué,
- le terme "hétérocyclyl" dans "hétérocyclyle" et "hétérocyclylalkyle" signifie un cycle de 4 à 10 chaînons saturé, partiellement insaturé ou insaturé, éventuellement substitué, comprenant un ou plusieurs hétéroatomes, identiques ou différents, choisis parmi azote, oxygène, soufre, silicium et phosphore,
- lorsque le radical amino est disubstitué, les deux substituants sont identiques ou différents ou bien peuvent constituer ensemble avec l'atome d'azote qui les porte un hétérocycle azoté saturé, partiellement insaturé ou insaturé, de 5 ou 6 atomes au total,
- lorsque le radical carbamoyle est disubstitué, les deux substituants sont identiques ou différents ou bien peuvent constituer ensemble avec l'atome d'azote qui les porte un hétérocycle azoté saturé, partiellement insaturé ou insaturé, de 5 ou 6 atomes au total,
- sauf précision contraire, l'expression "éventuellement substitué" qualifiant un groupe organique s'applique aux différents radicaux constituant ce groupe et signifie que les différents radicaux sont éventuellement substitués par un ou plusieurs radicaux R₉ et/ou aryle et/ou arylalkyle, identiques ou différents.

Selon une variante de la présente invention, celle-ci concerne des dérivés de l'acide picolinique, de formule générale (I) telle que définie ci-dessus et pour lesquels X₁ et X₂ représentent chacun un atome d'hydrogène.

Selon une autre variante de la présente invention, celle-ci concerne des dérivés de l'acide picolinique, de formule générale (I) telle que définie ci-dessus et pour lesquels Q₁ est choisi parmi l'atome d'oxygène et de soufre.

Selon une troisième variante de la présente invention, celle-ci concerne des dérivés de l'acide picolinique, de formule générale (I) telle que définie ci-dessus et pour lesquels Z est choisi parmi un radical alkyle et l'atome d'hydrogène ou un radical clivable susceptible de redonner l'hydrogène, par exemple, alkoxyalkyle, haloalkoxy-alkyle, alkylthioalkyle, haloalkylthioalkyle, N-alkylamino-alkyle, N,N-dialkylamino-alkyle, acylamino-alkyle, acyle, thioacyle, cyanoalkyle, alkoxythiocarbonyle, N-alkylaminothiocarbonyle, N,N-dialkylaminothiocarbonyle, ou alkylsulfinyle.

Une autre variante de la présente invention concerne des dérivés de l'acide picolinique, de formule générale (I) telle que définie ci-dessus et pour lesquels Y est choisi parmi un atome d'halogène, le radical hydroxy, mercapto, nitro, thiocyanato, azido, cyano, pentafluorosulfonyle, un radical alkyle, haloalkyle, alkylthio, haloalkylthio, alkoxyalkyle, haloalkoxyalkyle, alkylthioalkyle, haloalkylthioalkyle, alkylsulfinyle, haloalkylsulfinyle, alkylsulfonyle, haloalkylsulfonyle, alkoxysulfonyle, un groupe amino, N-alkylamino, N,N-dialkylamino, -NHCOR₁₀, -NHCSR₁₀, N-arylamino, N,N-diarylamino, arylcarbonylamino, arylthiocarbonylamino, aryloxythiocarbonylamino, et N,N-arylalkylaminothiocarbonylamino.

Plus particulièrement, la présente invention concerne des dérivés de l'acide picolinique, de formule générale (I) telle que définie ci-dessus possédant les caractéristiques suivantes prises isolément ou en combinaison :
- X₁ et X₂ représentent chacun un atome d'hydrogène,
- Z est choisi parmi un radical alkyle et l'atome d'hydrogène ou un radical clivable susceptible de redonner l'hydrogène, par exemple, alkoxyalkyle, haloalkoxy-alkyle, alkylthioalkyle, haloalkylthioalkyle, N-alkylaminoalkyle, N,N-dialkylaminoalkyle, acylaminoalkyle, acyle, thioacyle, cyanoalkyle, alkoxythiocarbonyle, N-alkylaminothiocarbonyle, N,N-dialkylaminothiocarbonyle, ou alkylsulfinyle,
- Y est choisi parmi un atome d'halogène, le radical hydroxy, mercapto, nitro, thiocyanato, azido, cyano, pentafluorosulfonyle, un radical alkyle, haloalkyle, alkylthio, haloalkylthio, alkoxyalkyle, haloalkoxyalkyle, alkylthioalkyle, haloalkylthioalkyle, alkylsulfinyle, haloalkylsulfinyle, alkylsulfonyle, haloalkylsulfonyle, alkoxysulfonyle, un groupe amino, N-alkylamino, N,N-dialkylamino, -NHCOR₁₀, -NHCSR₁₀, N-arylamino, N,N-diarylamino, arylcarbonylamino, arylthiocarbonylamino, aryloxythiocarbonylamino, N,N-arylalkylaminothiocarbonylamino,
- Q₁ est choisi parmi l'atome d'oxygène et de soufre.

Plus particulièrement encore, la présente invention concerne des dérivés de l'acide picolinique, de formule générale (I) telle que définie ci-dessus possédant les caractéristiques suivantes :
- X₁ et X₂ représentent chacun un atome d'hydrogène,
- Z est choisi parmi un radical alkyle et l'atome d'hydrogène ou un radical clivable susceptible de redonner l'hydrogène, par exemple, alkoxyalkyle, haloalkoxy-alkyle, alkylthioalkyle, haloalkylthioalkyle, N-alkylaminoalkyle, N,N-dialkylaminoalkyle, acylaminoalkyle, acyle, thioacyle, cyanoalkyle, alkoxythiocarbonyle, N-alkylaminothiocarbonyle, N,N-dialkylaminothiocarbonyle, ou alkylsulfinyle,
- Y est choisi parmi un atome d'halogène, le radical hydroxy, mercapto, nitro, thiocyanato, azido, cyano, pentafluorosulfonyle, un radical alkyle, haloalkyle, alkylthio, haloalkylthio, alkoxyalkyle, haloalkoxyalkyle, alkylthioalkyle, haloalkylthioalkyle, alkylsulfinyle, haloalkylsulfinyle, alkylsulfonyle, haloalkylsulfonyle, alkoxysulfonyle, un groupe amino, N-alkylamino, N,N-dialkylamino, -NHCOR₁₀, -NHCSR₁₀, N-arylamino, N,N-diarylamino, arylcarbonylamino, arylthiocarbonylamino, aryloxythiocarbonylamino, N,N-arylalkylaminothiocarbonylamino,
- Q₁ est choisi parmi l'atome d'oxygène et de soufre.

Plus spécifiquement encore, la présente invention concerne des dérivés de l'acide picolinique, de formule générale (I) telle que définie ci-dessus possédant les caractéristiques suivantes :
- X₁ et X₂ représentent chacun un atome d'hydrogène,
- Z est choisi parmi un radical alkyle et l'atome d'hydrogène ou un radical clivable susceptible de redonner l'hydrogène, par exemple, alkoxyalkyle, haloalkoxy-alkyle, alkylthioalkyle, haloalkylthioalkyle, N-alkylaminoalkyle, N,N-dialkylaminoalkyle, acylaminoalkyle, acyle, thioacyle, cyanoalkyle, alkoxythiocarbonyle, N-alkylaminothiocarbonyle, N,N-dialkylaminothiocarbonyle, ou alkylsulfinyle,
- Y est choisi parmi un atome d'halogène, le radical hydroxy, azido, alkylthio, alkylsulfonyle, un groupe amino, -NHCOR₁₀, et -NHCSR₁₀,
- Q₁ représente l'atome d'oxygène.

Dans le cadre de la présente invention, le terme "aryle" signifie phényle ou naphtyle, le terme "arylalkyle" signifiant alors phénylalkyle ou naphtylalkyle, plus particulièrement benzyle, phénéthyle, phénylpropyle, phénylbutyle, naphtylméthyle, naphtyléthyle, naphtylpropyle, ou naphtylbutyle. Il est entendu que ces différents radicaux peuvent être éventuellement substitués par un ou plusieurs radicaux R₉ et/ou aryle et/ou arylalkyle, identiques ou différents.

Les termes hétérocyclyle et hétérocyclylalkyle sont définis de façon similaire, étant entendu que "hétérocycle" signifie un monocycle ou un bicycle de 4 à 10 chaînons saturé, partiellement insaturé ou insaturé, comprenant au moins un hétéroatome choisi parmi azote, oxygène, soufre, silicium et phosphore.

Plus particulièrement le terme "hétérocycle" s'entend comme étant un des cycles (i) à (v) suivants :
- un cycle à 5 maillons décrit par la formule (i) : 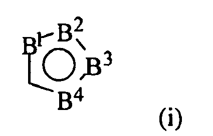
dans laquelle chacun des groupes de la liste B¹, B², B³, B⁴, est choisi parmi les atomes de carbone, d'azote, d'oxygène et de soufre de telle sorte que ladite liste comprenne de 0 à 3 atomes de carbone, de 0 à 1 atome de soufre, de 0 à 1 atome d'oxygène et de 0 à 4 atomes d'azote ;
- un cycle à 6 maillons décrit par la formule (ii) : 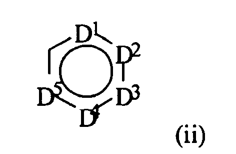
dans laquelle chacun des groupes de la liste D¹, D², D³, D⁴, D⁵, est choisi parmi les atomes de carbone ou d'azote de telle sorte que ladite liste comprenne de 1 à 4 atomes de carbone et de 1 à 4 atomes d'azote ;
- deux cycles fusionnés à 6 maillons chacun, décrits par la formule (iii) : 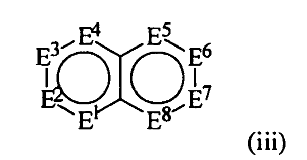
dans laquelle chacun des groupes de la liste E¹, E², E³, E⁴, E⁵, E⁶, E⁷, E⁸ est choisi parmi les atomes de carbone ou d'azote de telle sorte que ladite liste comprenne de 4 à 7 atomes de carbone et de 1 à 4 atomes d'azote ;
- un cycle à 6 maillons et un cycle à 5 maillons fusionnés décrits par la formule (iv) : 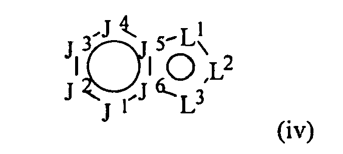 dans laquelle :
   * chacun des groupes de la liste J¹, J², J³, J⁴, J⁵, J⁶ est choisi parmi les atomes de carbone ou d'azote de telle sorte que ladite liste comprenne de 3 à 6 atomes de carbone, et de 0 à 3 atomes d'azote ; et
   * chacun des groupes de la liste L1, L2, L3 est choisi parmi les atomes de carbone, d'azote, d'oxygène ou de soufre de telle sorte que ladite liste comprenne de 0 à 3 atomes de carbone, de 0 à 1 atome de soufre, de 0 à 1 atome d'oxygène et de 0 à 3 atomes d'azote ; et
   * chacun des groupes de la liste J¹, J², J³, J⁴, J⁵, J⁶, L¹, L², L³ est choisi de telle façon que ladite liste comprenne de 3 à 8 atomes de carbone ;
- deux cycles fusionnés à 5 maillons chacun décrits par la formule (v) : 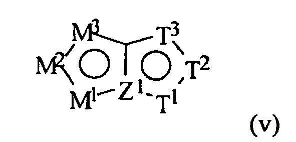 dans laquelle :
   chacun des groupes de la liste M¹, M², M³, représente les atomes de carbone, d'azote, d'oxygène ou de soufre de telle sorte que ladite liste comprenne de 0 à 3 atomes de carbone, de 0 à 1 atome de soufre, de 0 à 1 atome d'oxygène et de 0 à 3 atomes d'azote ;
   chacun des groupes de la liste T¹, T², T³ représentent les atomes de carbone, d'azote, d'oxygène ou de soufre de telle sorte que ladite liste comprenne de 0 à 3 atomes de carbone, de 0 à 1 atome de soufre, de 0 à 1 atome d'oxygène et de 0 à 3 atomes d'azote ;
   Z1 représente l'atome de carbone ou d'azote ;
   chacun des groupes de la liste M¹, M², M³, T¹, T², T³ est choisi de telle façon que ladite liste comprenne de 0 à 6 atomes de carbone.

Plus particulièrement encore le terme "hétérocycle" signifie dans la présente invention : furanyle, pyrolyle, thiophényle, pyrazolyle, imidazolyle, oxazolyle, isoxazolyle, thiazolyle, isothiazolyle, 1,2,3-oxadiazolyle, 1,2,4-oxadiazolyle, 1,2,5-oxadiazolyle, 1,3,4-oxadiazolyle, 1,2,3-thiadiazolyle, 1,2,4-thiadiazolyle, 1,2,5-thiadiazolyle, 1,3,4-thiadiazolyle, 1,2,3-triazolyle, 1,2,4-triazolyle, tétrazolyle, pyridyle, pyrimidinyle, pyrazinyle, pyridazinyle, 1,2,3-triazinyle, 1,2,4-triazinyle, 1,3,5-triazinyle, 1,2,3,4-tétrazinyle, 1,2,3,5-tétrazinyle, 1,2,4,5-tétrazinyle, benzimidazolyle, indazolyle, benzotriazolyle, benzoxazolyle, 1,2-benzisoxazolyle, 2,1-benzisoxazolyle, benzothiazolyle, 1,2-benzisothiazolyle, 2,1-benzisothiazolyle, 1,2,3-benzoxadiazolyle, 1,2,5-benzoxadiazolyle, 1,2,3-benzothiadiazolyle, 1,2,5-benzothiadiazolyle, quinoléinyle, isoquinoléinyle, quinoxazolinyle, quinazolinyle, cinnolyle ou phtalazyle, ptéridinyle, benzotriazinyle, 1,5-naphthyridinyle, 1,6-naphthyridinyle, 1,7-naphthyridinyle, 1,8-naphthyridinyle, imidazo[2,1-b]thiazolyle, thieno[3,4-b]pyridyle, purine, ou encore pyrolo[1,2-b]thiazolyle.

De manière tout à fait particulière, la présente invention concerne des dérivés de l'acide picolinique, de formule générale (I) telle que définie ci-dessus, qui sont :
- le 4-amino-3-hydroxy-N-[4-(4-méthylphénoxy)phényl]-2-pyridine carboxamide,
- le 4-(formylamino)-3-hydroxy-N-{4-[3-(trifluorométhyl)phénoxy] phényl}-2-pyridine carboxamide,
- le 4-amino-3-hydroxy-N-{4-[4-(trifluorométhyl)phénoxy]phényl}-2-pyridine carboxamide,
- le N-[4-(4-chlorophénoxy)phényl]-4-(formylamino)-3-hydroxy-2-pyridine carboxamide,
- le 4-(formylamino)-3-hydroxy-N-{4-[4-(trifluorométhyl)phénoxy] phényl}-2-pyridine carboxamide, et
- le N-[4-(benzyloxy)phényl]-4-(formylamino)-3-hydroxy-2-pyridine carboxamide.
ainsi que les éventuels N-oxydes, isomères géométriques et/ou optiques, énantiomères et/ou diastéréoisomères, formes tautomères, les sels, complexes métalliques et métalloïdiques.

Les composés de formule générale (I) et les composés éventuellement utilisables à titre d'intermédiaires dans les procédés de préparation, et qui seront définis à l'occasion de la description de ces procédés, peuvent exister sous une ou plusieurs formes d'isomères géométriques selon le nombre de doubles liaisons du composé. Les composés de formule générale (I) où Q₁ est -NR₁ ou -N-NR₄R₅ peuvent comporter 2 isomères géométriques différents notés (*E*) ou (*Z*) suivant la configuration des deux doubles liaisons. La notation *E* et *Z* peut être remplacée respectivement par les termes "syn" et "anti", ou "cis" et "trans". On se rapportera notamment à l'ouvrage de E. Eliel et S. Wilen "Stereochemistry of Organic Compounds", Ed. Wiley (1994), pour la description et l'utilisation de ces notations.

Les composés de formule générale (I) et les composés éventuellement utilisables à titre d'intermédiaires dans les procédés de préparation, et qui seront définis à l'occasion de la description de ces procédés, peuvent exister sous une ou plusieurs formes d'isomères optiques ou chiraux selon le nombre de centres asymétriques du composé. L'invention concerne donc aussi bien tous les isomères optiques que leurs mélanges racémiques ou scalémiques (on désigne par scalémique un mélange d'énantiomères dans des proportions différentes), ainsi que les mélanges de tous les stéréoisomères possibles en toutes proportions, y compris le mélange racémique. La séparation des diastéréoisomères et/ou des isomères optiques peut s'effectuer selon les méthodes connues en soi (E. Eliel *ibid.*).

La présente invention concerne également le procédé de préparation des composés de formule générale (I) et des composés éventuellement utilisables à titre d'intermédiaires dans les procédés de préparation. Ces composés peuvent être préparés selon la méthode de préparation générale décrite ci-dessous. Bien que générale, cette méthode de préparation fournit l'ensemble des conditions opératoires à mettre en oeuvre pour la synthèse des composés de formule (I) selon la présente invention. Il est bien entendu cependant que l'homme du métier saura adapter cette méthode en fonction des spécifités de chacun des composés qu'il voudra synthétiser.

La préparation des réactifs utilisés dans l'une ou l'autre des méthodes de préparation générale, est habituellement connue en soi et est habituellement décrite spécifiquement dans l'art antérieur ou d'une manière telle que l'homme de l'art peut l'adapter au but souhaité. L'art antérieur utilisable par l'homme de l'art pour établir les conditions de préparation des réactifs, peut être trouvé dans de nombreux ouvrages généraux de chimie comme "Advanced Organic Chemistry" de J.March, Ed. Wiley (1992), "Methoden der organischen Chemie" (Houben-Weyl), Ed. Georg Thieme Verlag ou les "Chemical Abstracts" Ed. American Chemical Society ainsi que dans les bases de données informatiques accessibles au public.

Les composés de formule générale (I), dans lesquels G représente l'oxygène, Z représente l'hydrogène et n est égal à 0, peuvent être préparés à partir d'un composé de formule (IIa) (préparé par exemple selon la méthode décrite dans la publication de R.H. Dodd, *Heterocycles, **47**,* (1998), 811) : 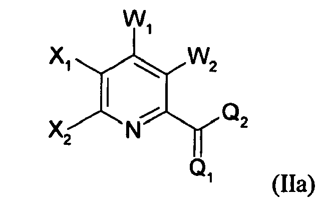 dans lesquels X₁, X₂, Q₁ et Q₂ sont tels que définis précédemment, et W₁ et W₂, identiques ou différents, représentent indépendamment l'un de l'autre un atome d'halogène choisi parmi fluor, chlore, brome ou iode,
qui est mis au contact d'un sel de l'acide azothydrique, plus particulièrement, mais non exclusivement, l'azoture de sodium, cette réaction étant conduite préférentiellement dans un solvant polaire aprotique comme la diméthylformamide, la diméthylacétamide, la N-méthylpyrrolidone, la diméthylpropylène-urée, le diméthylsulfoxide, au reflux ou à une température comprise entre 20°C et 200°C, pour conduire aux composés de formule (IIIa) : 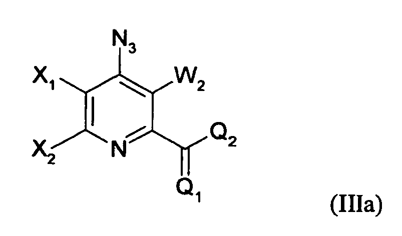 dans lesquels X₁, X₂, Q₁ et Q₂ sont tels que définis précédemment, et W₂ représente un atome d'halogène choisi parmi fluor, chlore, brome ou iode.

Les azotures de formule (IIIa) ci-dessus sont alors éventuellement réduits en dérivés aminés correspondants de formule (IVa) : 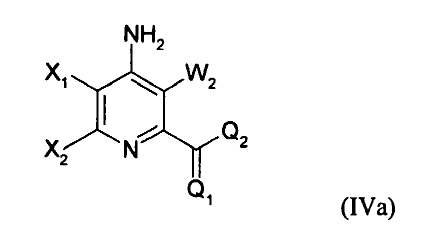 dans lesquels X₁, X₂, Q₁ et Q₂ sont tels que définis précédemment, et W₂ représente un atome d'halogène choisi parmi fluor, chlore, brome ou iode,
par action d'un agent réducteur, tel que par exemple la triphénylphosphine, le borohydrure de sodium ou l'hydrogène en présence d'un catalyseur, ou encore comme décrit J. March, *ibid,* pages 1219-1220.

Les composés de formule (IVa) peuvent alors être hydrolysés en dérivés 3-hydroxypicoliniques de formule (Va) : 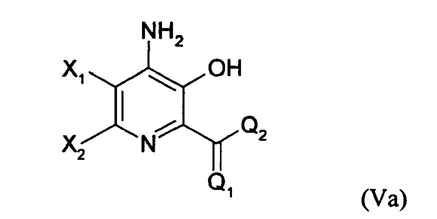 dans lesquels X₁, X₂, Q₁ et Q₂ sont tels que définis précédemment,
par action d'une base inorganique, tel que, mais non exclusivement, les hydroxydes de métaux alcalins et alcalino-terreux comme l'hydroxyde de sodium, de potassium, de césium ou de calcium. Cette réaction est généralement effectuée à une température comprise entre 0°C et la température d'ébullition du solvant, dans un solvant polaire aprotique dipolaire comme la diméthylformamide, la diméthylacétamide, la N-méthylpyrrolidone, la diméthylpropylène-urée, le diméthylsulfoxide ou l'eau.

Les composés de formule (Va) peuvent éventuellement soumis à diverses réactions d'alkylation bien connues de l'homme du métier afin de conduire aux composés de formule (VIa) : 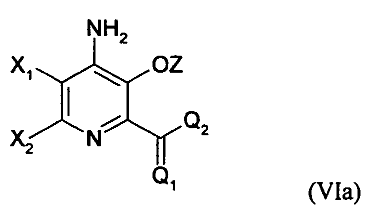 dans lesquels X₁, X₂, Z, Q₁ et Q₂ sont tels que définis précédemment.

Les composés de formule générale (I) dans lesquels G représente le soufre peuvent également être avantageusement préparés à partir d'un composé de formule (IIb) (préparé par exemple selon la méthode décrite dans la publication de R.H. Dodd, *Heterocycles, **47,*** (1998), page 811): 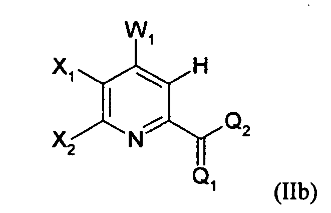 dans lesquels X₁, X₂, Q₁ et Q₂ sont tels que définis précédemment, et W₁ représente un atome d'halogène choisi parmi fluor, chlore, brome ou iode,
par réaction d'une base organique, tel que, mais non exclusivement, du di-isopropylamidure de lithium, et de soufre, pour conduire aux composés de formule (IIIb) : 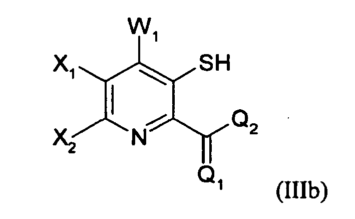 dans lesquels X₁, X₂, W₁, Q₁ et Q₂ sont tels que définis précédemment.

Le solvant approprié pour cette réaction peut être un hydrocarbure aliphatique tel que le pentane, l'hexane, l'heptane, l'octane; un hydrocarbure aromatique tel que le benzène, le toluène; un éther comme le diéthyléther, le di-isopropyléther, le tétrahydrofurane. La réaction est généralement réalisée à une température comprise entre -100°C et 0°C.

Les thiols de formule (IIIb) peuvent être ensuite transformés en composés de formule (IVb) : 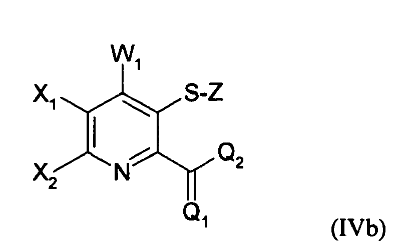 dans lesquels X₁, X₂, W₁, Q₁ et Q₂ sont tels que définis précédemment,
par réaction avec un agent alkylant, tel que, mais non spécifiquement, l'iodure de méthyle ou le chlorure de 4-méthoxybenzyle.

Cette réaction nécessite la présence d'une base organique ou inorganique, tel que l'hydroxyde de sodium, de potassium, de césium ou de calcium, les alcoolates de métaux alcalins et alcalino-terreux comme le *tertio*-butylate de potassium, les hydrures de métaux alcalins et alcalino-terreux, comme l'hydrure de sodium, de potassium ou de césium, les carbonates et bicarbonates de métaux alcalins et alcalino-terreux comme le carbonate de sodium, de potassium ou de calcium, les bases organiques, de préférences azotées, comme la pyridine, les alkylpyridines, les alkylamines comme la triméthylamine, la triéthylamine ou la di-isopropyléthylamine, les dérivés aza comme le 1,5-diazabicyclo[4.3.0]non-5-ène ou le 1,8-diazabicyclo[5.4.0]undèc-7-ène. La réaction est généralement effectuée à une température comprise entre -80°C et 180°C (de préférence entre 0°C et 150°C) ou au point d'ébullition du solvant utilisé. Le solvant approprié pour cette réaction peut être un hydrocarbure aliphatique tel que le pentane, l'hexane, l'heptane, l'octane; un hydrocarbure aromatique tel que le benzène, le toluène, les xylènes; un éther comme le diéthyléther, le di-isopropyléther, le tétrahydrofurane, le dioxane, le diméthoxyéthane; un hydrocarbure halogéné comme le dichlorométhane, le chloroforme, le 1,2-dichloroéthane; un nitrile comme l'acétonitrile, le propionitrile, le benzonitrile; un solvant aprotique dipolaire comme la diméthylformamide, la diméthylacétamide, la N-méthylpyrrolidone, la diméthylpropylène-urée, le diméthylsulfoxide ou l'eau.

Les composés de formule (IVb) peuvent ensuite être transformés en composés 4-aminés de formule (Vb) : 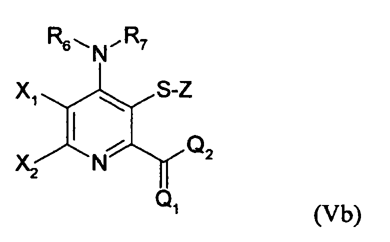 dans lesquels X₁, X₂, Q₁, Q₂, Z, R₆ et R₇ sont tels que définis précédemment,
en faisant réagir un composé de formule R₆R₇NH, ou un sel alcalin ou alcalino-terreux correspondant, en l'absence de solvant ou dans un solvant aprotique polaire, comme la diméthylformamide, la diméthylacétamide, la N-méthylpyrrolidine, la diméthylpropylène-urée, le diméthylsulfoxide, à une température comprise entre 0°C et la température d'ébullition du solvant.

Les composés de formules (Vb) dans lesquels X₁, X₂, Q₁, Q₂, R₆ et R₇ sont tels que définis précédemment, et Z représente un groupement 4-méthoxybenzilique, peuvent être transformés en 3-thiopyridine correspondant par réaction avec un acide organique, tel que, mais non exclusivement, l'acide trifluoroacétique, cette réaction étant conduite préférentiellement dans un solvant polaire protique comme les alcools, tel que l'éthanol, le méthanol, le propanol, ou le crésol, au reflux ou à une température comprise entre 20°C et 200°C,
pour conduire aux composés de formule (VIb) : 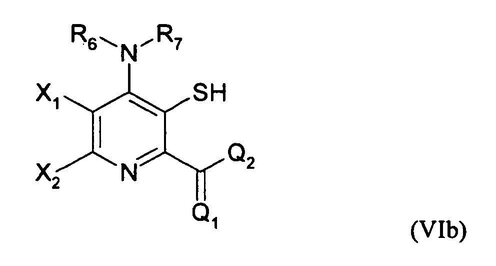 dans lesquels X₁, X₂, Q₁, Q₂, R₆ et R₇ sont tels que définis précédemment.

Les composés de formule (IVb) peuvent être transformés également en azotures de formule (VIIb) : 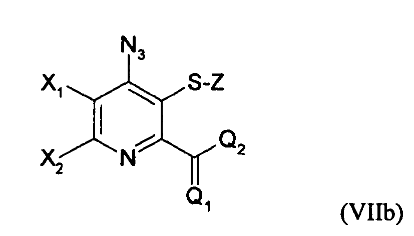 dans lesquels X₁, X₂, Q₁, Q₂, et Z sont tels que définis précédemment,
par réaction avec un sel de l'acide azothydrique, plus particulièrement, mais non exclusivement, l'azoture de sodium, préférentiellement dans un solvant polaire aprotique comme la diméthylformamide, la diméthylacétamide, la N-méthylpyrrolidone, la diméthylpropylène-urée, le diméthylsulfoxide, au reflux ou à une température comprise entre 20°C et 200°C.

Les composés de formule (VIIb) peuvent être ensuite hydrolysés en composés de formule (VIIIb) : 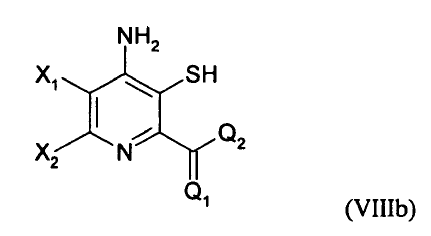 dans lesquels X₁, X₂, Q₁ et Q₂ sont tels que définis précédemment,
par action d'un agent réducteur, tel que par exemple la triphénylphosphine ou l'hydrogène, en présence d'un catalyseur, ou encore comme décrit J. March, *ibid,* pages 1219-1220.

Les composés de formules (Va), (VIa) et (VIIIb) définies précédemment peuvent éventuellement être mis au contact d'un agent acylant en présence d'un solvant et éventuellement d'une base. Par agent acylant, on entend préférentiellement mais de manière non limitative, un halogénure d'acyle, un anhydride, un acide, un ester, un amide primaire, et leurs homologues thio-, comme décrit dans J. March, *ibid,* pages 417-424, afin de conduire aux composés de formules (IX₁) et (IX₂) : 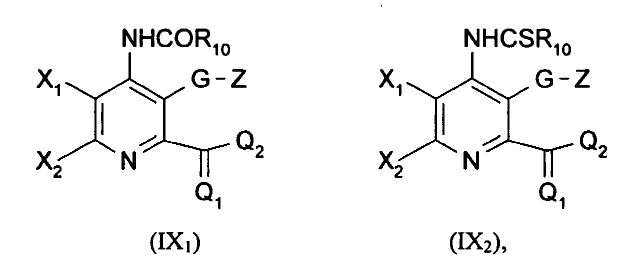 dans lesquels G, X₁, X₂, Q₁, Q₂, Z et R₁₀ sont tels que définis précédemment.

Les composés de formules (VIa) et (IVb) peuvent également être éventuellement soumis à diverses réactions de substitution et/ou d'addition bien connues de l'homme du métier pour conduire à l'ensemble des composés de formule (X): 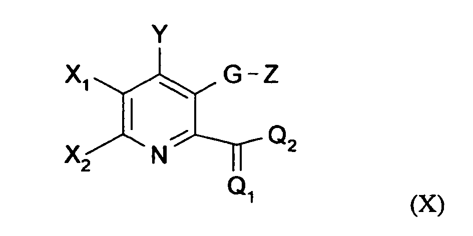 dans lesquels G, X₁, X₂, Q₁, Q₂, Y et Z sont tels que définis précédemment, cas particulier des composés de formule (I) pour lesquels n représente zéro.

Les composés de formule générale (XI) : 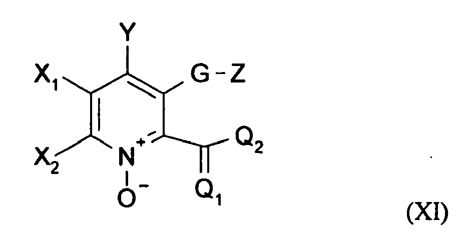 cas particuliers des composés de formule (I) pour lesquels n est égal à 1,
peuvent être obtenus par un procédé consistant à mettre en contact un composé de formule (X), cas particulier des composés de formule (I) pour lesquels n est égal à zéro,
avec un agent oxydant comme décrit dans J. March, *ibid.,* page 1200, en particulier l'eau oxygénée ou les peracides carboxyliques, boroniques, sulfurique.

Il doit être entendu que les réactions décrites dans les paragraphes précédents peuvent être effectuées dans tout ordre différent et convenable pour obtenir les composés de formule (I) souhaités. L'ordre des réactions sera tout particulièrement fixé par les impératifs de compatibilité des différents substituants du noyau pyridinique. Les compatibilités des différents radicaux et réactifs utilisés sont bien connues de l'homme du métier qui pourra de plus se référer aux exemples de préparation des composés de formule (I) exposés plus loin dans cette description.

L'invention concerne également des compositions fongicides comportant une quantité efficace d'au moins une matière active de formule (I). Les compositions fongicides selon l'invention comprennent, outre la matière active de formule (I), les supports solides ou liquides, acceptables en agriculture et/ou les agents tensioactifs également acceptables en agriculture. En particulier sont utilisables les supports inertes et usuels et les agents tensioactifs usuels. Ces compositions recouvrent non seulement les compositions prêtes à être appliquées sur la plante ou semence à traiter au moyen d'un dispositif adapté, tel qu'un dispositif de pulvérisation ou de poudrage, mais également les compositions concentrées commerciales qui doivent être diluées avant application sur la culture.

Ces compositions fongicides selon l'invention peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... Plus généralement les matières actives peuvent être combinées à tous les additifs solides ou liquides correspondant aux techniques habituelles de la mise en formulation.

D'une façon générale, les compositions selon l'invention contiennent habituellement de 0,05 à 99 % (en poids) de matière active, un ou plusieurs supports solides ou liquides et, éventuellement, un ou plusieurs agents tensioactifs.

Par le terme "support", dans le présent exposé, on désigne une matière organique ou minérale, naturelle ou synthétique, avec laquelle la matière active est combinée pour faciliter son application sur les parties de la plante. Ce support est donc généralement inerte et il doit être acceptable en agriculture. Le support peut être solide (argiles, silicates naturels ou synthétiques, silice, résines, cires, engrais solides, etc...) ou liquide (eau, alcools, notamment le butanol etc..., solvants organiques, huiles minérales et végétales et leurs dérivés). Des mélanges de tels supports peuvent être également utilisés.

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque la matière active et/ou le support inerte ne sont pas solubles dans l'eau et que l'agent vecteur de l'application est l'eau.

Ainsi donc, les compositions à usage agricole selon l'invention peuvent contenir la matière active dans de très larges limites, allant de 0,05 % à 99 % (en poids). Leur teneur en agent tensioactif est avantageusement comprise entre 5 % et 40 % en poids. Sauf indication contraire les pourcentages donnés dans cette description sont des pourcentages pondéraux.

Ces compositions selon l'invention sont elles-mêmes sous des formes assez diverses, solides ou liquides. Comme formes de compositions solides, on peut citer les poudres pour poudrage (à teneur en matière active pouvant aller jusqu'à 100 %) et les granulés, notamment ceux obtenus par extrusion, atomisation, compactage, imprégnation d'un support granulé, granulation à partir d'une poudre (la teneur en matière active dans ces granulés étant entre 0,5 et 80% pour ces derniers cas).

Les compositions fongicides selon l'invention peuvent encore être utilisées sous forme de poudres pour poudrage ; on peut aussi utiliser des compositions comprenant 50 g de matière active et 950 g de talc ; on peut aussi utiliser des compositions comprenant 20 g de matière active, 10 g de silice finement divisée et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les poudres mouillables (ou poudre à pulvériser).

Les suspensions concentrées, applicables en pulvérisation, sont préparées de manière à obtenir un produit fluide stable ne se déposant pas et conduisant à une bonne bio-disponibilité de la ou des matière(s) active(s). Ces suspensions contiennent habituellement de 5 % à 75 % de matière active, de préférence de 10 % à 25 %, de 0,5 à 75 % d'agents tensioactifs, de préférence de 5 % à 50 %, de 0 à 10 % d'additifs appropriés, comme des agents épaississants d'origine organique ou minérale, des agents anti-mousses, des inhibiteurs de corrosion, des adhésifs, des conservateurs, comme par exemple le Proxel GXL®, des antigels et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau. Dans certains cas, et notamment pour les formulations destinées au traitement de semences, un ou plusieurs colorants pourront être ajoutés.

Pour les applications foliaires, le choix des tensioactifs est primordial pour assurer une bonne bio disponibilité de la ou des matière(s) active(s) ; ainsi, on utilisera de préférence une combinaison d'un tensioactif à caractère hydrophile (HLB > 10) et d'un tensioactif à caractère lipophile (HLB < 5). De telles combinaisons d'agents tensioactifs sont par exemples décrites dans la demande de brevet français non encore publiée n° 00 04015.

À titre d'exemple, voici 3 formulations possibles de type suspension concentrée, adaptées à différentes cultures:

### Exemple SC1 (en g/kg):

Cet exemple est plutôt adapté aux cultures monocotylédones (céréales, riz,......)
- matière active 150
- tensioactif à caractère hydrophile (par exemple Rhodasurf 860P) 300
- tensioactif à caractère lipophile (par exemple Plurafac LF 700) 150
- phosphate de tristyrylphénol éthoxylé 50
- antimousse 5
- propylène glycol 30
- aérosil 200 20
- attagel 50 40
- eau (q.s.p. 1 kg) 255

### Exemple SC2 (en g/kg)

Cet exemple est plutôt adapté aux cultures dicotylédones (vigne, arbre fruitier......)
- matière active 150
- tensioactif à caractère hydrophile (par exemple Rhodasurf 860P) 150
- phosphate de tristyrylphénol éthoxylé 50
- antimousse 5
- propylène glycol 30
- aérosil 200 20
- attagel 50 40
- eau (q.s.p. 1 kg) 555

### Exemple SC3 (en g/kg)

Cet exemple est plus spécifiquement adapté au traitement de semences.
- matière active 50
- tensioactif à caractère hydrophile (par exemple Rhodasurf 860P) 5
- phosphate de tristyrylphénol éthoxylé 15
- antimousse 1
- propylène glycol 30
- colorant 20
- rhodopol G 1,5
- proxel GXL 1,5
- eau (q.s.p. 1 kg) 876

Pour réaliser ces formulations, on utilisera de préférence le mode opératoire suivant : À la quantité d'eau nécessaire, au moyen d'un agitateur à turbine, on mélange les tensioactifs sélectionnés (tensioactif à caractère hydrophile + tensioactif à caractère lipophile + phosphate de tristylphénol éthoxylé); après homogénéisation, on mélange ensuite les autres constituants de la formule en dehors de la matière active.
Puis on ajoute la matière active et éventuellement l'épaississant d'origine minérale (aérosil 200 et attagel 50) afin d'obtenir un milieu de consistance visqueuse.
Le mélange obtenu est ensuite broyé au moyen d'une turbine broyeuse à grande vitesse puis d'un broyeur à billes jusqu' à obtenir un D50 de l'ordre de 1 à 3 µm et un D90 compris entre 3 et 8 µm.
Dans le cas ou on n'a pas utilisé d'épaississant d'origine minérale, on rajoute ensuite l'épaississant d'origine naturelle (Rhodopol G) et on agite jusqu'à obtenir une viscosité adéquate.

Les poudres mouillables (ou poudre à pulvériser) sont habituellement préparées de telle sorte qu'elles contiennent de 20 % à 95 % de matière active, et elles contiennent habituellement, en plus du support solide, de 0 % à 30 % d'un agent mouillant, de 3 % à 20 % d'un agent dispersant, et, quand c'est nécessaire, de 0,1 % à 10 % d'un ou plusieurs stabilisants et/ou autres additifs, comme des agents de pénétration, des adhésifs, ou des agents antimottants, colorants, etc...

Pour obtenir les poudres à pulvériser ou poudres mouillables, on mélange intimement les matières actives dans les mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables très avantageusement en particulier pour l'application par exemple sur les feuilles des végétaux ou sur les semences.

A titre d'exemple, voici diverses compositions de poudres mouillables (ou poudres à pulvériser) :

### Exemple PM1

- matière active 50%
- alcool gras éthoxylé (agent mouillant) 2,5%
- phényléthylphénol éthoxylé (agent dispersant) 5%
- craie (support inerte) 42,5%

### Exemple PM2 :

- matière active 10%
- alcool synthétique oxo de type ramifié, en C13 éthoxylé par 8 à 10 oxyde d'éthylène (agent mouillant) 0,75%
- lignosulfonate de calcium neutre (agent dispersant) 12%
- carbonate de calcium (charge inerte) q.s.p. 100 %

### Exemple PM3 :

Cette poudre mouillable contient les mêmes ingrédients que dans l'exemple précédent, dans les proportions ci-après :
- matière active 75%
- agent mouillant 1,50%
- agent dispersant 8%
- carbonate de calcium (charge inerte) q.s.p. 100%

### Exemple PM4 :

- matière active 90%
- alcool gras éthoxylé (agent mouillant) 4%
- phényléthylphénol éthoxylé (agent dispersant) 6%

### Exemple PM5 :

- matière active 50%
   mélange de tensio-actifs anioniques et non ioniques (agent mouillant) 2,5%
- lignosulfonate de sodium (agent dispersant) 5%
- argile kaolinique (support inerte) 42,5%

Les dispersions et émulsions aqueuses, par exemple les compositions obtenues en diluant à l'aide d'eau une poudre mouillable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les compositions fongicides selon l'invention peuvent être formulées sous la forme de granulés dispersables dans l'eau également compris dans le cadre de l'invention. Ces granulés dispersables, de densité apparente généralement comprise entre environ 0,3 et 0,6 ont une dimension de particules généralement comprise entre environ 150 et 2000 et de préférence entre 300 et 1500 microns.

La teneur en matière active de ces granulés est généralement comprise entre environ 1% et 90%, et de préférence entre 25% et 90%. Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20% en poids du granulé) dont plus de la moitié est, par exemple, constituée par au moins un agent dispersant, essentiellement anionique, tel qu'un polynaphtalène sulfonate alcalin ou alcalino terreux ou un lignosulfonate alcalin ou alcalino-terreux, le reste étant constitué par des mouillants non ioniques ou anioniques tel qu'un alcoyle naphtalène sulfonate alcalin ou alcalino-terreux. Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc...). On termine généralement par un concassage suivi d'un tamisage à la dimension de particule choisie dans les limites mentionnées ci-dessus. On peut encore utiliser des granulés obtenus comme précédemment puis imprégnés avec une composition contenant la matière active.

De préférence, il est obtenu par extrusion, en opérant comme indiqué dans les exemples ci-après.

### Exemple GD1 : Granulés dispersables

Dans un mélangeur, on mélange 90 % en poids de matière active et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

### Exemple GD2 : Granulés dispersables

Dans un mélangeur, on mélange les constituants suivants :
- matière active 75%
- agent mouillant (alkylnaphtalène sulfonate de sodium) 2%
- agent dispersant (polynaphtalène sulfonate de sodium) 8%
- charge inerte insoluble dans l'eau (kaolin) 15%

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

Les composés de l'invention peuvent aussi être mélangés avec un ou plusieurs insecticides, fongicides, bactéricides, acaricides attractants ou phéromones ou autres composés à activité biologique. Les mélanges ainsi obtenus ont une activité à spectre élargi. En particulier, les composés selon la présente invention ne présente pas de phénomène de résistance croisée avec les dérivés de strobilurine. En effet, les composés selon l'invention sont actifs sur un site biochimique différent de celui des dérivés de strobilurine.

Les mélanges avec d'autres fongicides sont particulièrement avantageux, notamment les mélanges avec l'acibenzolar-S-méthyl, l'azoxystrobine, le bénalaxyl, le bénomyl, la blasticidine-S, le bromuconazole, le captafol, le captane, le carbendazim, la carboxine, le carpropamide, le chlorothalonil, les compositions fongicides à base de cuivre, les dérivés du cuivre tels que l'hydroxyde de cuivre et l'oxychlorure de cuivre, la cyazofamide, le cymoxanil, le cyproconazole, le cyprodinyl, le dichloran, le diclocymet, le diethofencarb, le difénoconazole, le diflumétorim, le diméthomorphe, le diniconazole, la discostrobine, le dodémorphe, la dodine, l'édifenphos, l'époxyconazole, l'éthaboxam, l'éthirimol, la famoxadone, la fénamidone, le fénarimol, le fenbuconazole, le fenhexamide, le fenpiclonil, la fenpropidine, le fenpropimorphe, la ferimzone, le fluazinam, le fludioxonil, le flumétover, le fluquinconazole, le flusilazole, le flusulfamide, le flutolanil, le flutriafol, le folpel, le furalaxyl, le furametpyr, la guazatine, l'hexaconazole, l'hymexazol, l'imazalil, l'iprobenphos, l'iprodione, l'isoprothiolane, la kasugamycin, le krésoxim-méthyl, le mancozèbe, le manèbe, le méfénoxam, le mépanipyrim, le métalaxyl et ses formes énatiomériques telles que le métalaxyl-M, le metconazole, le métiram-zinc, la métominostrobine, l'oxadixyl, le péfurazoate, le penconazole, le pencycuron, l'acide phosphoreux et ses dérivés tels que le fosétyl-Al, le phtalide, la picoxystrobine, le probénazole, le prochloraz, la procymidone, le propamocarbe, le propiconazole, la pyraclostrobine, le pyriméthanil, le pyroquilon, le quinoxyfène, le silthiofam, le siméconazole, la spiroxamine, le tébuconazole, le tétraconazole, le thiabendazole, la thifluzamide, le thiophanate, par exemple le thiophanate-méthyl, le thiram, le triadiméfon, le triadiménol, le tricyclazole, le tridémorphe, la trifloxystrobine, le triticonazole, les dérivés de la valinamide tels que, par exemple, l'iprovalicarb, la vinclozoline, le zinèbe et la zoxamide.

L'invention a pour autre objet un procédé de lutte, à titre curatif ou préventif, contre les champignons phytopathogènes des cultures, caractérisé en ce que l'on applique sur le sol où poussent ou où sont susceptibles de pousser les végétaux, sur les feuilles et/ou les fruits des végétaux ou sur les semences des végétaux, une quantité efficace (agronomiquement efficace) et non phytotoxique d'une matière active de formule (I), de préférence sous forme d'une composition fongicide selon l'invention.

Par "quantité efficace et non phytotoxique", on entend une quantité de composition selon l'invention suffisante pour permettre le contrôle ou la destruction des champignons présents ou susceptibles d'apparaître sur les cultures, et n'entraînant pour lesdites cultures aucun symptôme notable de phytotoxicité. Une telle quantité est susceptible de varier dans de larges limites selon le champignon à combattre, le type de culture, les conditions climatiques, et les composés compris dans la composition fongicide selon l'invention. Cette quantité peut être déterminée par des essais systématiques au champ, à la portée de l'homme du métier.

L'invention concerne enfin une méthode de protection à titre préventif ou curatif des produits de multiplication des végétaux, ainsi que des végétaux en résultant, contre les maladies fongiques, caractérisée en ce que l'on recouvre lesdits produits d'une dose efficace et non phytotoxique d'une composition selon l'invention.

Les compositions selon l'invention sont utiles pour traiter les semences de céréales (blé, seigle, triticale et orge notamment), de pomme de terre, de coton, de pois, de colza, de maïs, de lin ou encore les semences d'arbres forestiers (notamment de résineux), ou encore les semences génétiquement modifiées de ces végétaux.

On notera à ce propos que dans le jargon de l'homme de métier, le terme traitement de semences se rapporte en fait au traitement des graines. Les techniques d'application sont bien connues de l'homme de métier et elles peuvent être utilisées sans inconvénient dans le cadre de la présente invention. On pourra citer par exemple le pelliculage ou l'enrobage. Parmi les produits de multiplications des végétaux concernés, on peut citer notamment les semences ou graines, et les tubercules.

Comme cela a été indiqué précédemment, les modalités de recouvrement des produits de multiplication des végétaux, notamment des semences, sont bien connues dans l'art et font appel en particulier aux techniques de pelliculage ou d'enrobage.

Les produits et compositions selon l'invention peuvent aussi s'appliquer en application foliaire sur les cultures végétales, c'est-à-dire sur les feuillages, les fleurs, les fruits et/ou les troncs des végétaux concernés.

Parmi les végétaux visés par la méthode selon l'invention, on peut citer le riz, le maïs, le coton, les céréales, comme le blé, l'orge, le triticale, les arbres fruitiers, en particulier pommiers, poiriers, pêchers, vigne, bananiers, orangers, citronniers, etc..., les cultures oléagineuses, par exemple, colza, tournesol, les cultures maraîchères et légumières, tomates, salades, les cultures protéagineuses, le pois, les solanées, par exemple la pomme de terre, la betterave, le lin, et les arbres forestiers, ainsi que les homologues génétiquement modifiés de ces cultures.

Parmi les végétaux visés par la méthode selon l'invention, on peut citer :
- le blé, en ce qui concerne la lutte contre les maladies suivantes des semences : les fusarioses (*Microdochium nivale* et *Fusarium roseum*), les caries (*Tilletia caries, Tilletia controversa* ou *Tilletia indica*), la septoriose (*Septoria nodorum*) ; le charbon nu (*Ustilago tritici*) ;
- le blé, en ce qui concerne la lutte contre les maladies suivantes des parties aériennes de la plante : le piétin-verse (*Tapesia yallundae, Tapesia acuiformis*), le piétin-échaudage (*Gaeumannomyces graminis*), la fusariose du pied (*F. culmorum, F. graminearum*), la fusariose des épis (*F. culmorum, F. graminearum, Microdochium nivale*), le rhizoctone (*Rhizoctonia cerealis*), l'oïdium (*Erysiphe graminis forma specie tritici*), les rouilles (*Puccinia striiformis* et *Puccinia recondita*) et les septorioses (*Septoria tritici* et *Septoria nodorum*), l'helminthosporiose (*Drechslera tritici-repentis*) ;
- le blé et l'orge, en ce qui concerne la lutte contre les maladies bactériennes et virales, par exemple la jaunisse nanisante de l'orge ;
- l'orge, en ce qui concerne la lutte contre les maladies suivantes des semences : les helminthosporioses (*Pyrenophora graminea, Pyrenophora teres* et *Cochliobolus sativus*), le charbon nu (*Ustilago nuda*) et les fusarioses (*Microdochium nivale* et *Fusarium roseum*) ;
- l'orge, en ce qui concerne la lutte contre les maladies suivantes des parties aériennes de la plante : le piétin-verse (*Tapesia yallundae*), les helminthosporioses (*Pyrenophora teres* et *Cochliobolus sativus*), l'oïdium (*Erysiphe graminis forma specie hordei*), la rouille naine (*Puccinia hordei*) et la rhynchosporiose (*Rhynchosporium secalis*) ;
- la pomme de terre, en ce qui concerne la lutte contre les maladies du tubercule (notamment *Helminthosporium solani, Phoma tuberosa, Rhizoctonia solani, Fusarium solani*), le mildiou (*Phytopthora infestans*) et certaines viroses (virus Y) ;
- la pomme de terre en ce qui concerne la lutte contre les maladies du feuillage suivantes : l'alternariose (*Alternaria solani*), le mildiou (*Phytophthora infestans*) ;
- le coton, en ce qui concerne la lutte contre les maladies suivantes des jeunes plantes issues des semences : les fontes de semis et les nécroses du collet (*Rhizoctonia solani, Fusarium oxysporum*), la pourriture noire des racines (*Thielaviopsis basicola*) ;
- les cultures protéagineuses, par exemple le pois, en ce qui concerne la lutte contre les maladies suivantes des semences : l'anthracnose (*Ascochyta pisi, Mycosphaerella pinodes*), la fusariose (*Fusarium oxysporum*), la pourriture grise (*Botrytis cinerea*)*,* le mildiou (*Peronospora pisi*) ;
- les cultures oléagineuses, par exemple le colza, en ce qui concerne la lutte contre les maladies suivantes des semences : *Phoma lingam, Alternaria brassicae* et *Sclerotinia sclerotiorum* ;
- le maïs, en ce qui concerne la lutte contre les maladies des semences : (*Rhizopus* sp., *Penicillium* sp., *Trichoderma* sp., *Aspergillus* sp. et *Gibberella fujikuroï*) ;
- le lin, en ce qui concerne la lutte contre la maladie des semences : *Alternaria linicola ;*
- les arbres forestiers, en ce qui concerne la lutte contre les fontes de semis (*Fusarium oxysporum, Rhizoctonia solani*) ;
- le riz en ce qui concerne la lutte contre les maladies suivantes des parties aériennes : la pyriculariose (*Magnaporthe grisea*), le rhizoctone (*Rhizoctonia solani*) ;
- les cultures légumières en ce qui concerne la lutte contre les maladies suivantes des semis ou des jeunes plants issus de semences : les fontes de semis et les nécroses du collet (*Fusarium oxysporum, Fusarium roseum, Rhizoctonia solani, Pythium sp.*) ;
- les cultures légumières en ce qui concerne la lutte contre les maladies suivantes des parties aériennes : la pourriture grise (Botrytis sp.), les oïdiums (notamment *Erysiphe cichoracearum, Sphaerotheca fuliginea, Leveillula taurica*), les fusarioses (*Fusarium oxysporum, Fusarium roseum*), les cladosporioses (*Cladosporium sp.*), les alternarioses (*Alternaria sp.*), les anthracnoses (*Colletotrichum sp.*), les septorioses (*Septoria sp.*), le rhizoctone (*Rhizoctonia solani*), les mildious (par exemple *Bremia lactucae, Peronospora sp., Pseudoperonospora sp, Phytophthora sp*) ;
- les arbres fruitiers en ce qui concerne les maladies des parties aériennes : la moniliose (*Monilia fructigenae, M. laxa*), la tavelure (*Venturia inaequalis*), l'oïdium (*Podosphaera leucotricha*) ;
- la vigne en ce qui concerne les maladies du feuillage : notamment la pourriture grise (*Botrytis cinerea*), l'oïdium (*Uncinula necator*), le black-rot (*Guignardia biwelli*), le mildiou (*Plasmopara viticola*) ;
- la betterave en ce qui concerne les maladies suivantes des parties aériennes : la cercosporiose (*Cercospora beticola*), l'oïdium (*Erysiphe beticola*), la ramulariose (*Ramularia beticola*).

Le blé et le riz sont les végétaux préférés pour la mise en oeuvre de la méthode selon l'invention, bien que l'ensemble des cultures, plantes, produits de multiplication des végétaux, fleurs, bois, et, en règle générale, tous les végétaux pouvant subir des attaques de la part de champignons phytopathogènes, peuvent être avantageusement traités selon la méthode de l'invention, par utilisation d'une ou plusieurs matières actives, compositions fongicides selon la présente invention.

Dans le cas des traitements de végétaux, la dose de composition appliquée est, en général et de façon avantageuse, comprise entre 10 et 800 g / ha, de préférence 50 à 300 g / ha de matière active pour des applications en traitement foliaire.
La dose de composition appliquée est, en général, de façon avantageuse telle que la dose de matière active est comprise entre 2 et 200 g de matière active par 100 kg de semence, de préférence entre 3 et 150 g par 100 kg dans le cas des traitements de semences. Il est bien entendu que les doses indiquées ci-dessus le sont à titre d'exemples d'illustration de l'invention. L'homme du métier saura adapter les doses d'application en fonction de la nature de la culture à traiter.

La présente invention concerne également une méthode de traitement, à titre curatif ou préventif, à l'aide d'un ou plusieurs composés selon l'invention, ou d'une composition selon la présente invention contre les maladies fongiques susceptibles de se développer sur ou à l'intérieur de bois de construction. Par bois de construction, on entend tous types d'essence de bois, et tous types de mise en forme de ce bois dédié à la construction, par exemple bois massif, aggloméré, plaqué, contre-plaqué, etc.

Ainsi, la méthode de traitement de bois de construction selon l'invention, consiste à mettre en contact un ou plusieurs composés de la présente invention, ou une composition selon l'invention. Cette mise en contact peut revêtir les formes les plus diverses, comme par exemple application directe, badigeonnage, trempage, injection ou tout autre moyen approprié.

La présente invention concerne également le traitement des plantes génétiquement modifiées avec les composés selon l'invention ou les compositions agrochimiques selon l'invention. Les plantes génétiquement modifiées sont des plantes dans le génome desquelles un gène hétérologue codant pour une protéine d'intérêt a été intégré de manière stable.

Par gène hétérologue codant pour une protéine d'intérêt on entend essentiellement selon l'invention les gènes conférant à la plante transformée de nouvelles propriétés agronomiques, ou les gènes d'amélioration de la qualité agronomique de la plante transformée.

Parmi les gènes conférant de nouvelles propriétés agronomiques aux plantes transformées, on peut citer les gènes conférant une tolérance à certains herbicides, ceux conférant une résistance à certains insectes, ceux conférant une tolérance à certaines maladies, etc. De tels gènes sont notamment décrits dans les demandes de brevet WO 91/02071 et WO 95/06128.

Parmi les gènes conférant une tolérance à certains herbicides, on peut citer le gène *Bar* conférant une tolérance au bialaphos, le gène codant pour une EPSPS appropriée conférant une résistance aux herbicides ayant l'EPSPS comme cible comme le glyphosate et ses sels (US 4,535,060, US 4,769,061, US 5,094,945, US 4,940,835, US 5,188,642, US 4,971,908, US 5,145,783, US 5,310,667, US 5,312,910, US 5,627,061, US 5,633,435, FR 2 736 926), le gène codant pour la glyphosate oxydoréductase (US 5,463,175), ou encore un gène codant pour une HPPD conférant une tolérance aux herbicides ayant pour cible l'HPPD comme les isoxazoles, notamment l'isoxafutole (FR 95 06800, FR 95 13570), les dicétonitriles (EP 496 630, EP 496 631) ou les tricétones, notamment la sulcotrione (EP 625 505, EP 625 508, US 5,506,195). De tels gènes codant pour une HPPD conférant une tolérance aux herbicides ayant pour cible l'HPPD sont décrits dans la demande de brevet WO 96/38567.

Dans les cas des gènes codant pour EPSPS ou HPPD, et plus particulièrement pour les gènes ci-dessus, la séquence codant pour ces enzymes est avantageusement précédée par une séquence codant pour un peptide de transit, en particulier pour le peptide de transit dit peptide de transit optimisé décrit dans le brevet US 5,510,471.

Parmi les gènes conférant de nouvelles propriétés de résistance aux insectes, on citera plus particulièrement les gènes codant pour les protéines *Bt* largement décrites dans la littérature et bien connues de l'homme du métier. On citera aussi les gènes codant pour les protéines extraites de bactéries comme *Photorabdus* (WO 97/17432 et WO 98/08932).

Parmi les gènes conférant de nouvelles propriétés de résistance aux maladies on citera notamment les gènes codant pour les chitinases, les glucanases, l'oxalate oxydase, toutes ces protéines et leurs séquences codantes étant largement décrites dans la littérature, ou encore les gènes codant pour des peptides antibactériens et/ou antifongiques, en particulier des peptides de moins de 100 acides aminés riches en cystéines comme les thionines ou défensines de plantes, et plus particulièrement les peptides lytiques de toutes origines comprenant un ou plusieurs ponts disulfures entre les cystéines et des régions comprenant des acides aminés basiques, notamment les peptides lytiques suivants : l'androctonine (WO 97/30082 et PCT/FR98/01814, déposée le 18 août 1998) ou la drosomicine (PCT/FR98/01462, déposée le 8 juillet 1998). On citera également les gènes codant pour des peptides éliciteurs fongiques, en particulier les élicitines (Kamoun & al., 1993 ; Panabières & al., 1995).

Parmi les gènes modifiant la constitution des plantes modifiées, on peut citer en particulier les gènes modifiant la teneur et la qualité de certains acides gras essentiels (EP 666 918) ou encore la teneur et la qualité des protéines, en particuliers dans les feuilles et/ou les graines desdites plantes. On citera en particulier les gènes codant pour des protéines enrichies en acides aminés soufrés (WO 98/20133 ; WO 97/41239 ; WO 95/31554 ; WO 94/20828 ; WO 92/14822).

La présente invention concerne plus particulièrement le traitement des plantes génétiquement modifiées comprenant un gène hétérologue conférant à la plante des propriétés de résistance aux maladies. De manière préférentielle, le gène hétérologue confère à la plante génétiquement modifiée un spectre d'activité complémentaire du spectre d'activité des composés selon l'invention. Par spectre complémentaire, on entend selon l'invention un spectre d'activité pour le gène hétérologue distinct du spectre d'activité des composés selon l'invention, ou un spectre d'activité portant sur des agents infectieux identiques mais permettant un contrôle identique ou amélioré pour de moindre doses d'application en composés selon l'invention.

Enfin, l'invention concerne l'utilisation des composés selon l'invention utiles en thérapeutique humaine et animale pour le traitement des maladies fongiques, comme par exemple les mycoses, dermatoses, maladies à trichophyton et les candidoses ou encore les maladies à *Aspergillus spp.,* par exemple *Aspergillus fumigatus.*

Les exemples suivants illustrent de manière non limitative quelques exemples de composés fongicides selon l'invention. Dans les exemples qui suivent, "PF" signifie "Point de Fusion" et est exprimé en ° Celsius (°C).

### Exemple a) :

### Préparation de la 2-cyano-3-méthoxy-4-nitropyridine

Un mélange de 12,5 g (12,5 moles) du N-oxyde de la 3-méthoxy-4-nitropyridine, 7,72 mL (1,1 éq.) de sulfate de méthyle et 70 mL de 1,2-dichoroéthane est chauffé à 70°C pendant 2,5 heures. On laisse refroidir et on ajoute 70 mL d' eau. On refroidit dans un bain de glace et de sel et on additionne, par portions, 7,55 g (2,1 moles) de cyanure de sodium en contrôlant que la température ne dépasse pas 10°C. Après 4 heures d'agitation, le mélange réactionnel est extrait avec l'éther éthylique, la phase organique est lavée à l'eau, concentrée et le résidu chromatographié (acétate d'éthyle/dichlorométhane). On obtient 7,06 g d' une huile jaune (Rendement 53 %).

### Exemple b) :

### Préparation de 4-bromo-2-cyano-3-méthoxypyridine

Un mélange de 6 g (0,0335 moles) de 2-cyano-3-méthoxy-4-nitropyridine obtenue dans l'exemple a), 12,37 g (0,100 moles) de bromure d'acétyle et 36 mL de diméthoxy-1,2-éthane est chauffé à 85°C pendant 1,5 heures. On laisse refroidir et on verse la réaction sur 100 g de glace pilée. On ajoute 30 mL de 1,2-dichloroéthane et on neutralise doucement jusqu' à pH = 8 avec une solution aqueuse d'ammoniac à 28%. Après extraction par du 1,2-dichloroéthane, lavage à l'eau, séchage et concentration, le résidu est chromatographié (acétate d'éthyle/heptane, 3:7) pour obtenir 5,32 g (rendement 75%) d'un solide blanc (PF = 116°C).
De la même manière, en remplaçant le bromure d'acétyle par le chlorure d'acétyle, est obtenue la 4-chloro-2-cyano-3-méthoxypyridine (rendement 83%) sous forme d'un solide blanc (PF = 91 °C).

### Exemple c) :

### Préparation de 4-azido-2-cyano-3-méthoxypyridine

Sur 1 g (0,0155 moles) de nitrure de sodium dans 25 mL de diméthylformamide à 0°C, on ajoute doucement, 3 g (0,0141 moles) de 4-bromo-2-cyano-3-méthoxypyridine de l'exemple b), dissous dans 40 mL de diméthylformamide. On laisse le mélange sous agitation pendant 6 jours à température ambiante. On dilue la réaction dans 200 mL d'eau glacée et l'on extrait au dichlorométhane. On lave la phase organique deux fois à l'eau, on sèche, on concentre et on chromatographie le résidu (acétate d'éthyle/heptane, 3:7). On obtient 0,87g (rendement 35%) d'un solide blanc (PF = 102°C).

### Exemple d) :

### Préparation de l'acide 4-chloro-3-hydroxypicolinique

Un mélange de 2 g (0,012 moles) de 4-chloro-2-cyano-3-méthoxypyridine obtenue dans l'exemple b), et 7 mL d'acide chlorhydrique à 37% est chauffé à 100°C pendant 12 heures. Après refroidissement le solide formé est filtré, lavé une fois a l'eau et trois fois avec de l'acétone et séché sous vide pendant 8 heures. On obtient 1,78 g (rendement 86%) d'un solide jaune (PF = 228°C).

De la même manière, sont obtenus les hydroxy-acides suivants :

| Y | Hydracide | Rdt, PF (°C) |
|---|---|---|
| acide 4-bromo-3-hydroxypicolinique | HBr | solide jaune, 82%, 230°C |
| acide 4-azido-3-hydroxypicolinique | HCl | solide violet, 63% |
| acide 3,4-dihydroxypicolinique | HBr | solide blanc, 74%, 264°C |

### Exemple e) :

### Préparation de 2-cyano-3, 4-diméthoxypyridine

3 g (0,017 moles) de 2-cyano-3-méthoxy-4-nitropyridine obtenue dans l'exemple a) et une solution de méthylate de sodium préparée avec 0,77g (0,033 moles) de sodium et 65 mL de méthanol sont agités à température ambiante pendant 4 h. On ajoute 100 mL d' eau, on élimine le méthanol et la phase aqueuse est extraite avec le dichlorométhane. La phase organique est lavée à l'eau, séchée, concentrée et le résidu chromatographié (acétate d'éthyle/heptane, 1:1) pour obtenir 1,96 g (rendement 72%) d'un solide blanc (PF = 133°C).

### Exemple f) :

### Préparation de 2-cyano-3-hydroxy-4-thiométhoxypyridine

2 g de 4-bromo-2-cyano-3-méthoxypyridine obtenue dans l'exemple b) et 2,16 g de thiométhylate de sodium dans 40ml de diméthylformamide anhydre sont chauffés à 85°C pendant 5 heures. Après refroidissement et ajout de 20ml d'eau, le mélange réactionnel est concentré à sec. Le résidu est extrait trois fois avec du méthanol chaud. La phase méthanolique refroidie est filtrée et concentrée. On obtient 1,51 g (rendement 97%) d'un miel marron utilisé brut.

### Exemple g) :

### Préparation de l'acide 3-hydroxy-4-thiométhoxypicolinique

2,5 g (0,015 moles) de 2-cyano-3-hydroxy-4-thiométhoxypyridine de l'exemple f), 8,5 g d'hydroxyde de potassium et 25 mL d'eau sont chauffés à reflux pendant 2,5 heures. On laisse refroidir et dans un bain glacé on neutralise doucement avec de l'acide chlorhydrique 1N jusqu'à pH = 2-3. On filtre le solide formé. On lave le solide une fois à l'eau et trois fois avec de l'acétone ; on sèche sous vide pendant 8 heures. On obtient 1,81 g (rendement 68%) d'un solide blanc (PF = 247°C).

### Exemple h) :

### Préparation de l'acide 3,4-diméthoxypicolinique

1 g de 3,4-diméthoxy-2-cyanopyridine obtenue dans l'exemple e) et 3,5 g d'hydroxyde de potassium dans 15 mL d'eau sont chauffés à 85°C pendant une demi-heure. On laisse refroidir et dans un bain glacé, on ajoute doucement de l'acide chlorhydrique jusqu'à pH = 2-3. Après concentration à sec, le résidu est extrait trois fois avec du méthanol chaud ; on laisse refroidir, on filtre et on concentre. On obtient un solide utilisé brut.

### Exemple i) :

### Préparation du N-oxyde de l'acide 3-hydroxypicolinique

À un mélange de 20ml d'acide acétique et 20ml d'eau oxygénée, sont ajoutés 2 g d'acide 3-hydroxypicolinique ; le tout est porté à 80°C pendant 5 heures. Après élimination des solvants sous vide, le solide obtenu est lavé avec de l'alcool chaud pour obtenir 2,02 g de composé attendu sous la forme d'un solide blanc (PF = 182°C).

### Exemple de préparation :

### 3-hydroxy-4-méthoxy-N-paraphénoxyphénylpicolinamide

0,046g de paraphénoxyaniline, 0,04g d'acide 3-hydroxy-4-méthoxypicolinique (obtenu selon un procédé similaire à celui décrit dans l'exemple g)), 0,034g de 1-hydroxybenzotriazole et 0,060 g de chlorhydrate de 1-(3-diméthylaminopropyl)-3-éthylcarbodi-imide sont chauffés dans 2 mL de pyridine entre 75 et 85°C pendant 1 à 2 heures. Après refroidissement, le résidu est repris dans une mélange de dichlorométhane et 2ml d'acide chlorhydrique 1N . Après extraction par le dichlorométhane, concentration et chromatographie sur silice on obtient 0,057g du composé en titre, solide jaune (PF = 186°C).

### Exemple 1 : Composé n° 76

### 4-amino-3-hydroxy-N-paraphénoxyphénylpicolinamide

À 0,14g de 4-azido-3-hydroxy-N-paraphénoxyphénylpicolinamide (obtenue à partir du composé de l'exemple de préparation selon les modes opératoires décrits dans les exemples a) à g))) dissous dans un mélange éthanol/acétate d'éthyle, 1:2, on ajoute une pointe de spatule de palladium sur charbon à 10%. L'hydrogénation est conduite à 20 bars de pression et à température ambiante pendant 4-5 heures. Après filtration, concentration et chromatographie du résidu dans l'acétate d'éthyle, on obtient 0,099g d'un solide blanc (PF : 197°C).

### Exemple 2 : Composé n° 111

### 4-formamido-3-hydroxy-N-paraphénoxyphénylpicolinamide

On chauffe à reflux 61,2 mg d'anhydride acétique et 27,6 mg d' acide formique pendant 4 heures et on ajoute 46 mg de 4-amino-3-hydroxy-N-paraphénoxy-phénylpicolinamide de l'exemple 1, dissous dans 5 mL de tétrahydrofurane. Après 8 heures de reflux, le mélange réactionnel est concentré et purifié par chromatographie pour donner 39 mg d'un solide jaune PF 208°C.

### Exemple 3 : Composé n° 108

### 4-amino-3-hydroxy-N-para-[4-(trifluorométhyl)phénoxy]phénylpicolinamide

### Étape 1 :

### 4-azido-3-iodo-N-para[4-(trifluorométhyl)phénoxy]phénylpicolinamide

Un mélange de 25,9 g (0,05 mol) de 4-chloro-3-iodo-N-*para-*[4-(trifluorométhyl)phénoxy]phénylpicolinamide (préparée à partir de l'acide picolinique selon la méthode décrite dans *Heterocycles, **47,*** (1998), 811) et 6,5 g (0,1 mol) d'azoture de sodium dissous dans 250 mL de diméthylsulfoxyde, est chauffé à 70°C pendant 8 heures. Après refroidissement, le mélange est versé sur 1 litre d'eau. Le précipité obtenu est filtré et lavé à l'éther. On obtient 22,5 g (rendement 85%) d'un solide marron. Rf (heptane/acétate d'éthyle 50/50) : 0,45.

### Étape 2:

### 4-amino-3-iodo-N-para-[(4-(trifluorométhyl)phénoxy]phénylpicolinamide

Un mélange de 21,0 g (0,04 mol) de 4-azido-3-iodo-N-*para-*[4-(trifluorométhyl)phénoxy]phénylpicolinamide et de 21,0 g (0,08 mol) de triphénylphosphine dans 80 mL de tétrahydrofurane, est agité à température ambiante pendant 15 heures. Le mélange est hydrolysé pendant 1 heure avec 100 mL d'une solution de chlorure d'hydrogène 1N. Le milieu est ensuite versé sur 200 mL d'eau et neutralisé par ajout de 100 mL d'hydroxyde de sodium 1N. Après extraction par l'acétate d'éthyle, séchage et concentration, le résidu est chromatographié sur gel de silice (acétate d'éthyle/heptane 1:1) pour obtenir 13,4 g (rendement 55%) d'un solide jaune. Rf (heptane/acétate d'éthyle 50/50) : 0,29.

### Étape 3 :

### 4-amino-3-hydroxy-N-para-[4-(trifluorométhyl)phénoxy)phénylpicolinamide

Un mélange de 9,15 g (0,018 mol) de 4-amino-3-iodo-N-*para-*[4-(trifluorométhyl)phénoxy]phénylpicolinamide dissous dans une solution de 82 mL d'hydroxyde de potassium 50% aqueux et 20 mL de diméthylsulfoxyde est chauffé pendant 8 heures à 90°C. Le milieu est versé sur 100 mL d'eau et extrait à l'éther. La phase organique est séchée et évaporée. Après recristallisation dans le méthanol, on obtient 6,15 g (rendement 85%) d'un solide blanc (PF = 202°C).

Les composés décrits dans le tableau 1 suivant sont préparés de manière analogue :

**Tableau 1**

| | | | | |
|---|---|---|---|---|
| 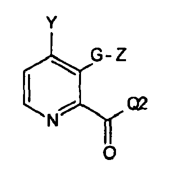 | | | | |

| N° | Q2 | G - Z | Y | PF |
|---|---|---|---|---|
| 1 | 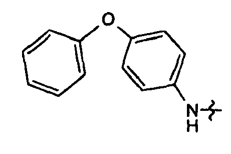 | 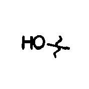 | 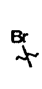 | 140 |
| 2 | 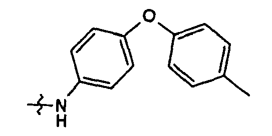 | 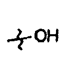 | 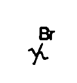 | 168 |
| 3 | 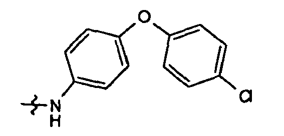 | 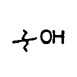 | 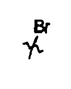 | 155 |
| 4 | 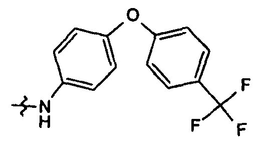 | 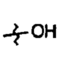 | 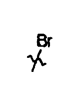 | 145 |
| 5 | 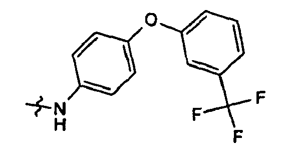 | 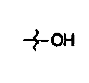 | 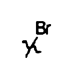 | 118 |
| 6 | 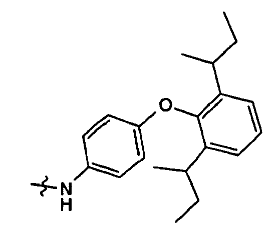 | 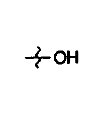 | 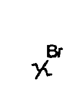 | 86 |
| 7 | 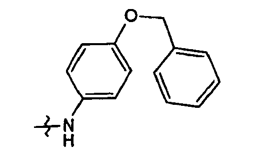 | 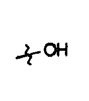 | 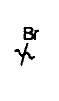 | 165 |
| 8 | 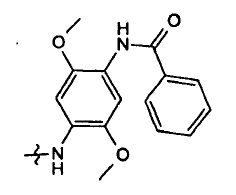 | 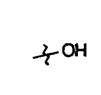 | 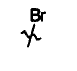 | 250 |
| 9 | 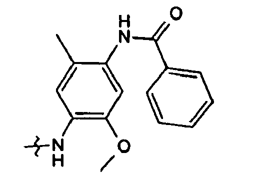 | 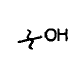 | 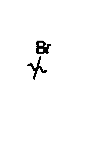 | 255 |
| 10 | 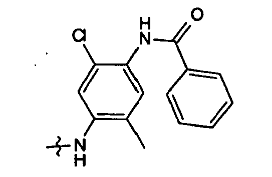 | 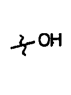 | 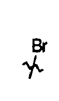 | 235 |
| 11 | 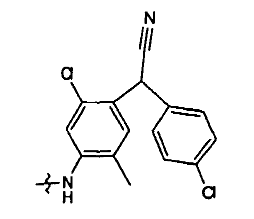 | 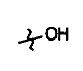 | 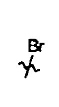 | 162 |
| 12 | 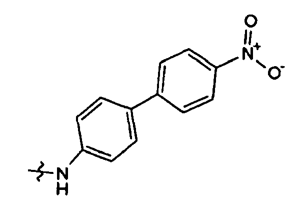 | 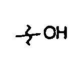 | 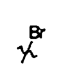 | 292 |
| 13 | 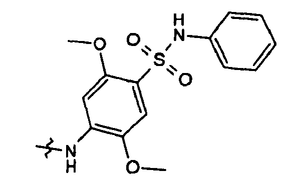 | 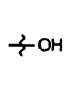 | 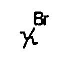 | 168 |
| 14 | 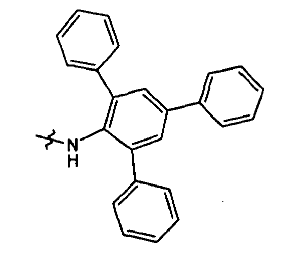 | 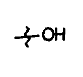 | 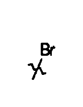 | 135 |
| 15 | 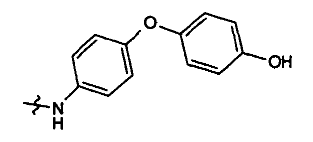 | 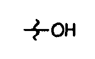 | 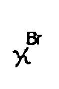 | 165 |
| 16 | 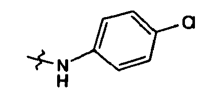 | 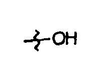 | 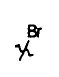 | 161 |
| 17 | 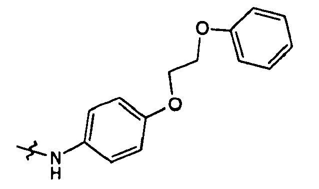 | 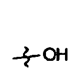 | 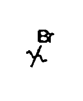 | 160 |
| 18 | 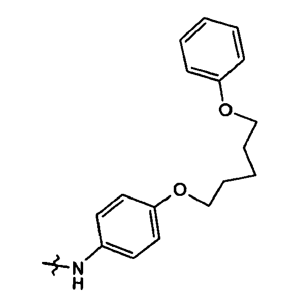 | 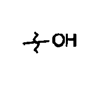 | 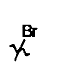 | 122 |
| 19 | 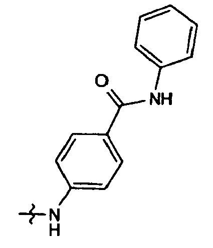 | 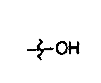 | 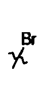 | 256 |
| 20 | 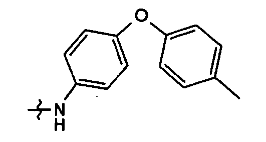 | 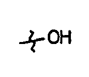 | 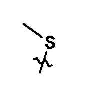 | 198 |
| 21 | 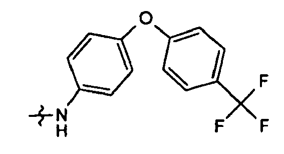 | 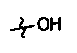 | 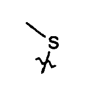 | 162 |
| 22 | 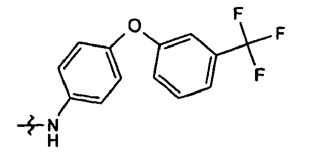 | 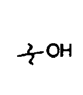 | 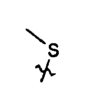 | 139 |
| 23 | 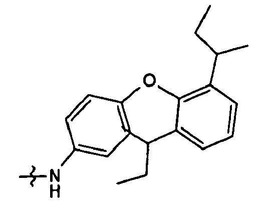 | 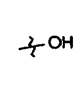 | 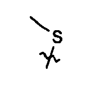 | 150 |
| 24 | 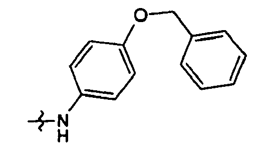 | 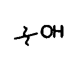 | 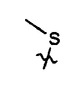 | 208 |
| 25 | 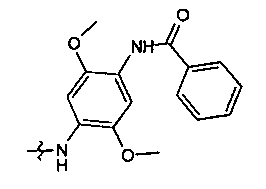 | 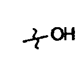 | 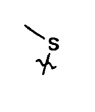 | 210 |
| 26 | 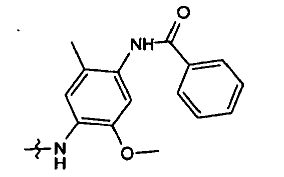 | 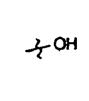 | 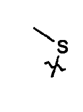 | 242 |
| 27 | 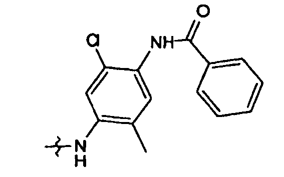 | 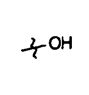 | 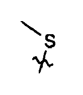 | 243 |
| 28 | 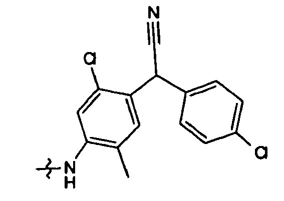 | 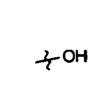 | 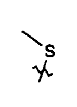 | 212 |
| 29 | 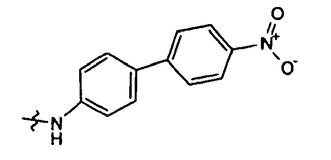 | 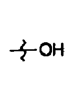 | 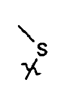 | |
| 30 | 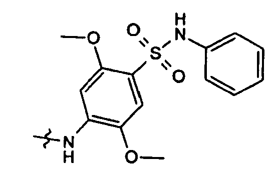 | 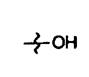 | 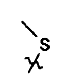 | 185 |
| 31 | 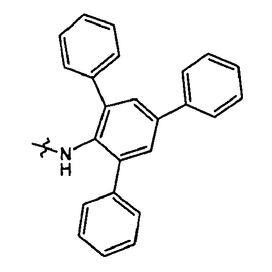 | 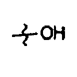 | 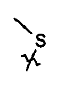 | 118 |
| 32 | 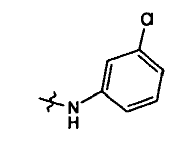 | 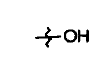 | 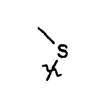 | 172 |
| 33 | 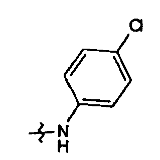 | 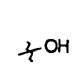 | 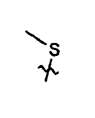 | 214 |
| 34 | 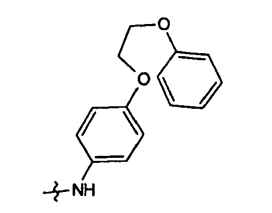 | 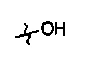 | 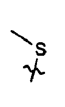 | 172 |
| 35 | 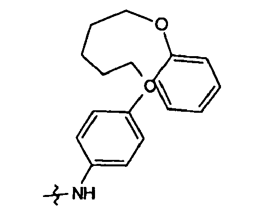 | 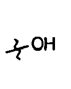 | 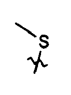 | 122 |
| 36 | 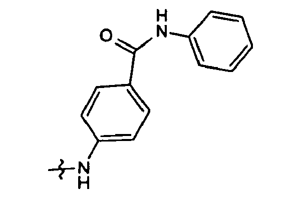 | 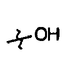 | 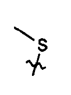 | 248 |
| 37 | 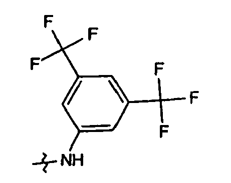 | 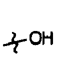 | 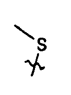 | 168 |
| 38 | 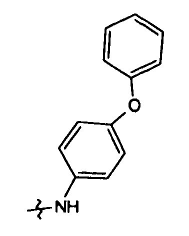 | 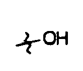 | 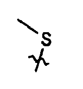 | 186 |
| 39 | 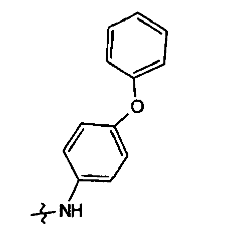 | 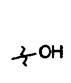 | 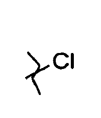 | 120 |
| 40 | 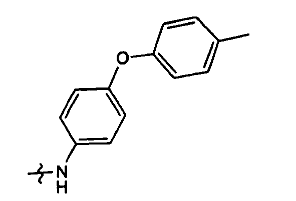 | 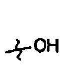 | 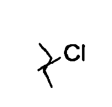 | 146 |
| 41 | 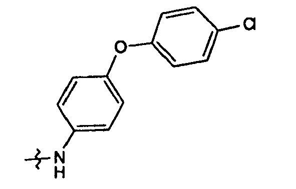 | 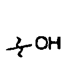 | 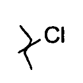 | 148 |
| 42 | 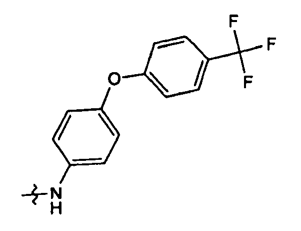 | 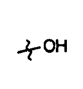 | 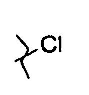 | 147 |
| 43 | 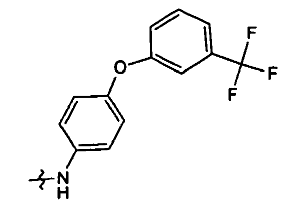 | 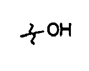 | 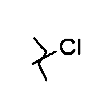 | 110 |
| 44 | 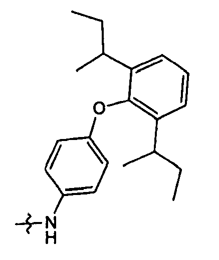 | 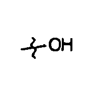 | 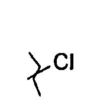 | 66 |
| 45 | 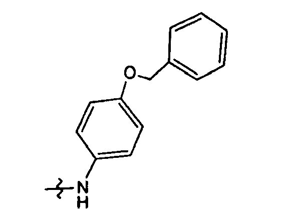 | 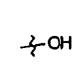 | 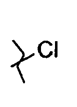 | 150 |
| 46 | 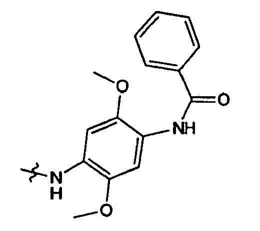 | 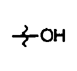 | 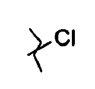 | 246 |
| 47 | 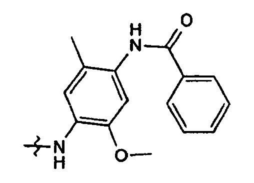 | 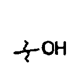 | 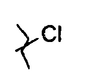 | 260 |
| 48 | 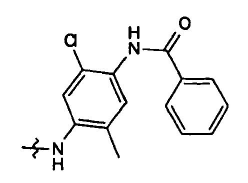 | 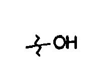 | 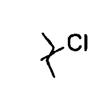 | 226 |
| 49 | 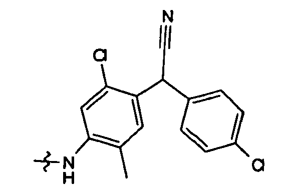 | 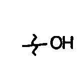 | 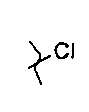 | 140 |
| 50 | 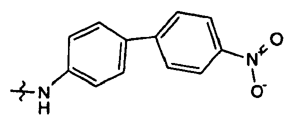 | 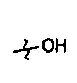 | 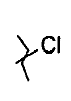 | |
| 51 | 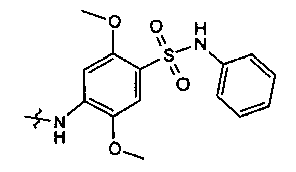 | 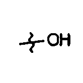 | 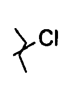 | 166 |
| 52 | 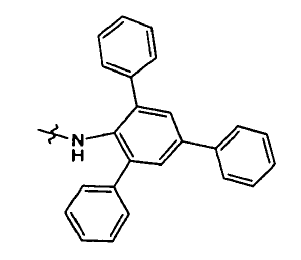 | 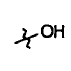 | 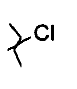 | 124 |
| 53 | 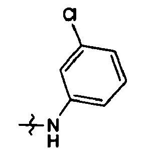 | 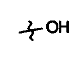 | 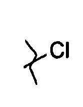 | 174 |
| 54 | 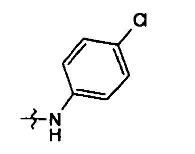 | 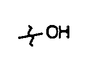 | 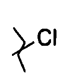 | 166 |
| 55 | 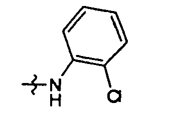 | 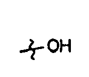 | 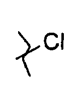 | 164 |
| 56 | 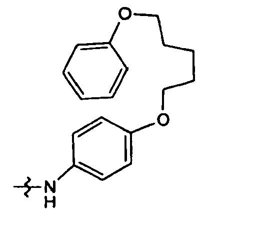 | 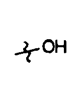 | 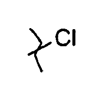 | 120 |
| 57 | 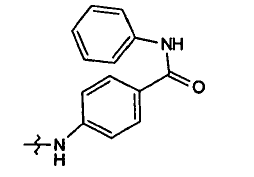 | 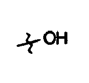 | 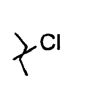 | 279 |
| 58 | 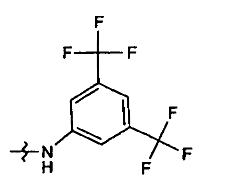 | 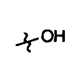 | 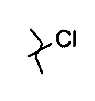 | 76 |
| 59 | 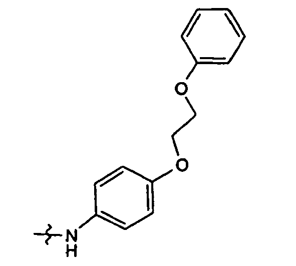 | 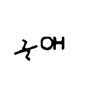 | 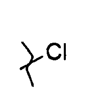 | 156 |
| 60 | 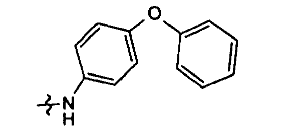 | 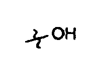 | 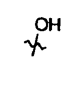 | 284 |
| 61 | 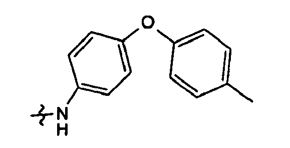 | 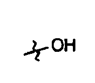 | 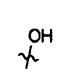 | 265 |
| 62 | 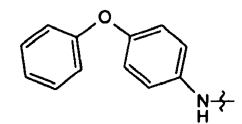 | 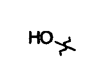 | 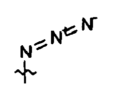 | 138 |
| 63 | 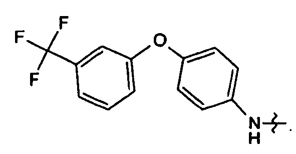 | 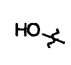 | 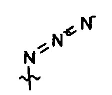 | |
| 64 | 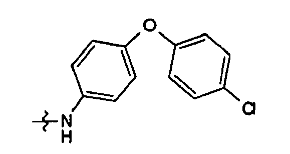 | 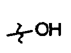 | 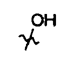 | 271 |
| 65 | 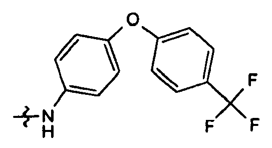 | 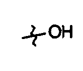 | 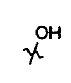 | 274 |
| 66 | 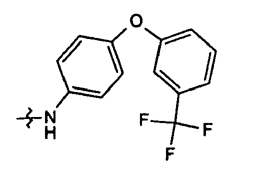 | 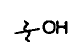 | 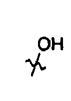 | 252 |
| 67 | 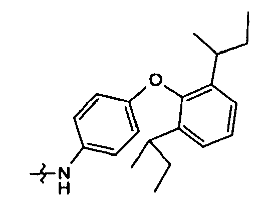 | 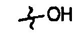 | 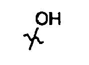 | 272 |
| 68 | 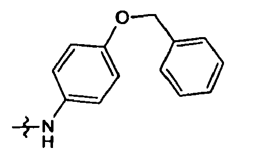 | 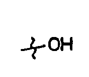 | 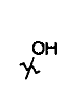 | 294 |
| 69 | 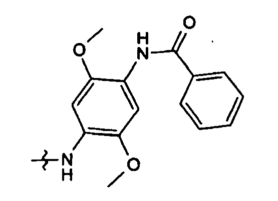 | 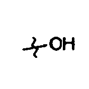 | 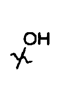 | 296 |
| 70 | 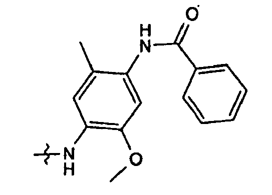 | 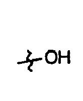 | 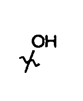 | |
| 71 | 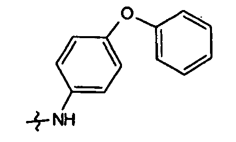 | 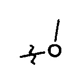 | 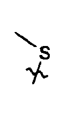 | |
| 72 | 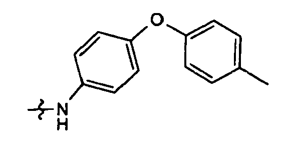 | 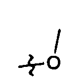 | 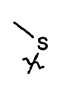 | 102 |
| 73 | 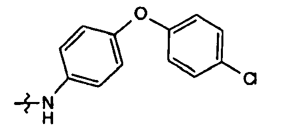 | 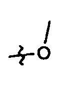 | 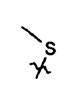 | 114 |
| 74 | 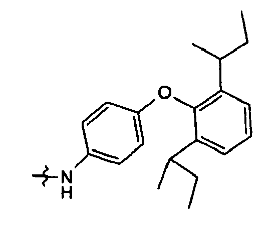 | 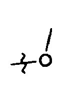 | 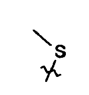 | |
| 75 | 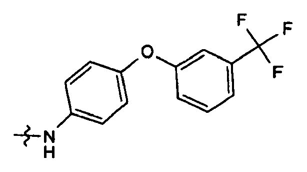 | 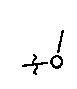 | 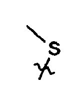 | 136 |
| 76 | 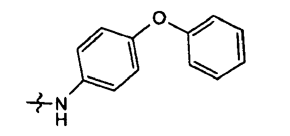 | 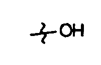 | 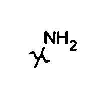 | 197 |
| 77 | 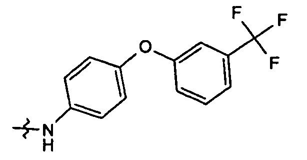 | 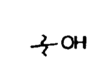 | 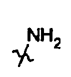 | 199 |
| 78 | 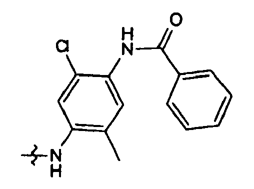 | 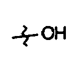 | 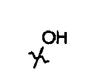 | |
| 79 | 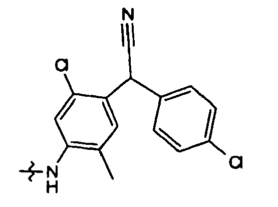 | 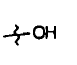 | 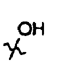 | 148 |
| 80 | 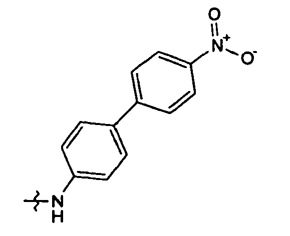 | 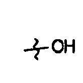 | 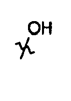 | |
| 81 | 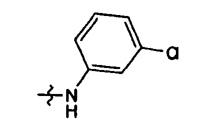 | 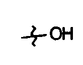 | 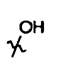 | 277 |
| 82 | 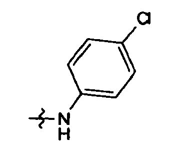 | 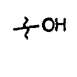 | 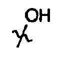 | 288 |
| 83 | 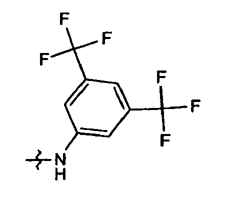 | 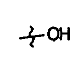 | 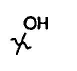 | 278 |
| 84 | 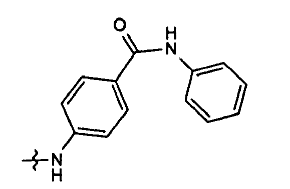 | 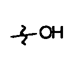 | 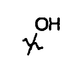 | |
| 85 | 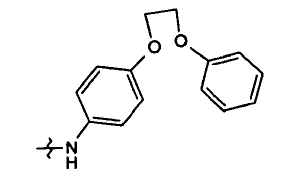 | 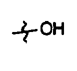 | 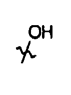 | 276 |
| 86 | 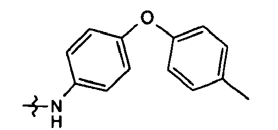 | 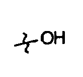 | 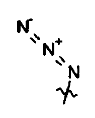 | 121 |
| 87 | 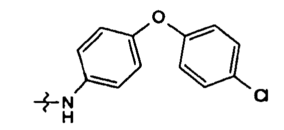 | 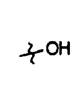 | 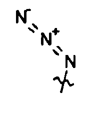 | 113 |
| 88 | 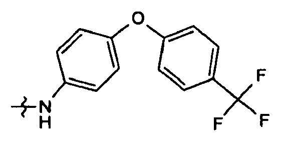 | 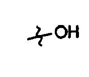 | 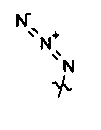 | 105 |
| 89 | 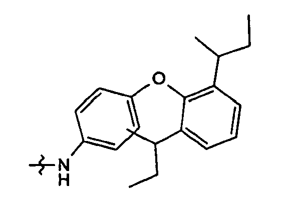 | 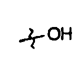 | 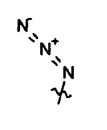 | 106 |
| 90 | 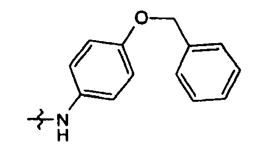 | 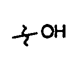 | 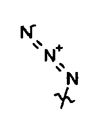 | 135 |
| 91 | 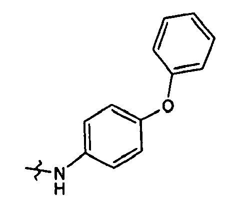 | 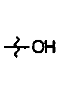 | 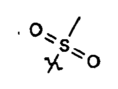 | |
| 92 | 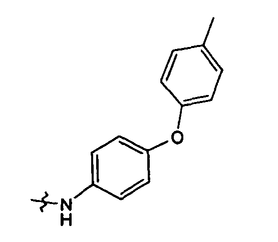 | 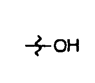 | 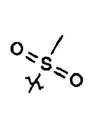 | |
| 93 | 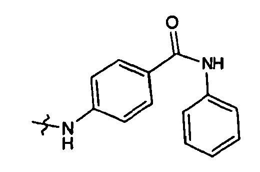 | 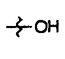 | 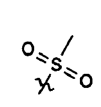 | |
| 94 | 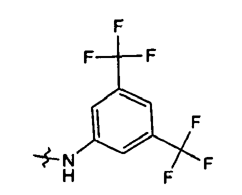 | 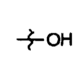 | 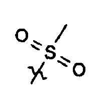 | 170 |
| 95 | 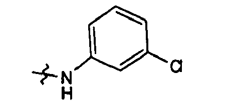 | 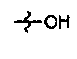 | 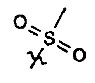 | 142 |
| 96 | 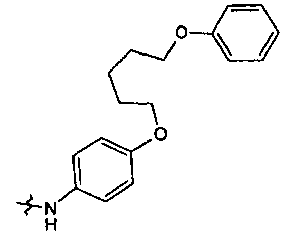 | 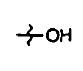 | 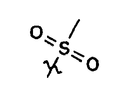 | |
| 97 | 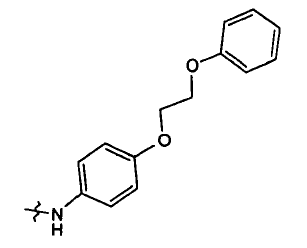 | 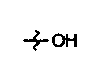 | 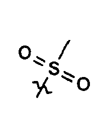 | |
| 98 | 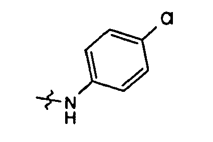 | 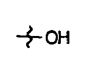 | 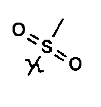 | |
| 99 | 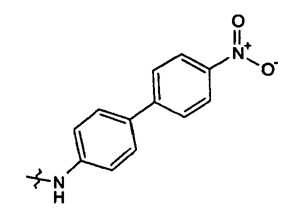 | 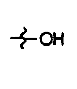 | 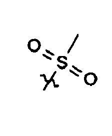 | |
| 100 | 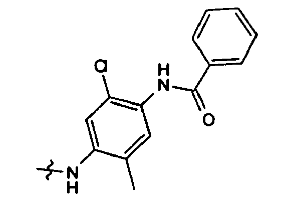 | 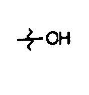 | 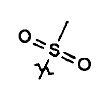 | |
| 101 | 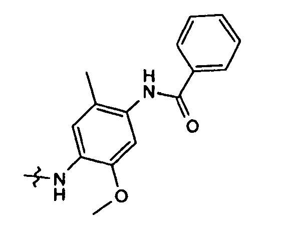 | 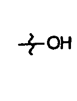 | 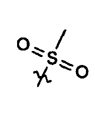 | |
| 102 | 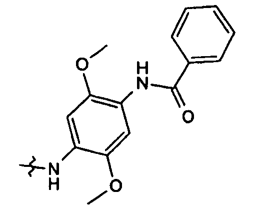 | 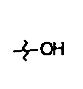 | 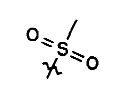 | |
| 103 | 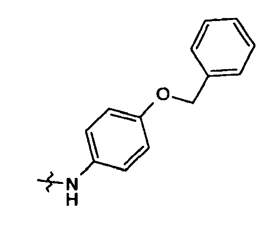 | 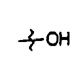 | 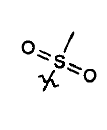 | |
| 104 | 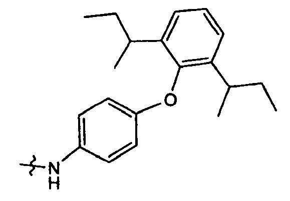 | 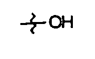 | 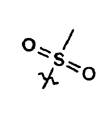 | 117 |
| 105 | 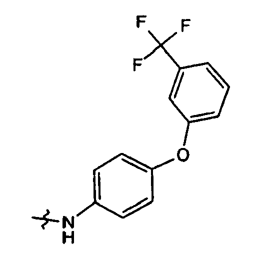 | 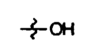 | 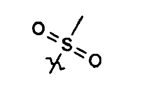 | |
| 106 | 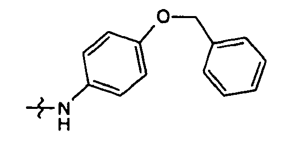 | 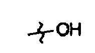 | 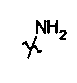 | 214 |
| 107 | 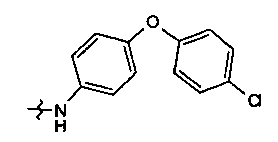 | 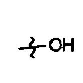 | 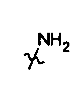 | 252 |
| 108 | 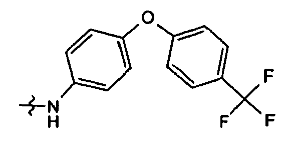 | 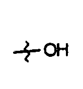 | 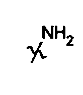 | 232 |
| 109 | 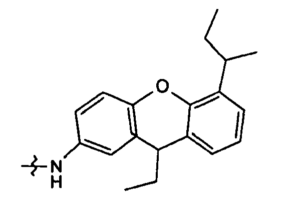 | 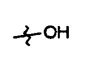 | 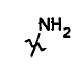 | 246 |
| 110 | 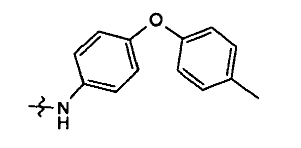 | 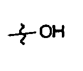 | 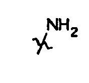 | 227 |
| 111 | 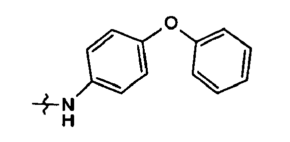 | 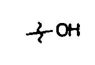 | 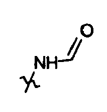 | 208 |
| 112 | 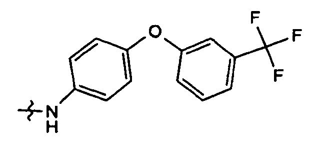 | 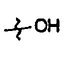 | 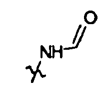 | 208 |
| 113 | 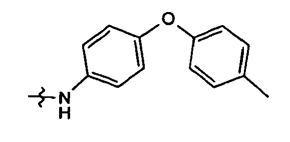 | 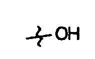 | 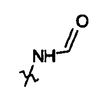 | 185 |
| 114 | 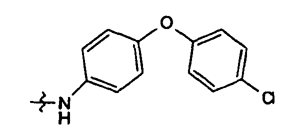 | 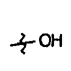 | 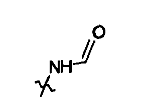 | 198 |
| 115 | 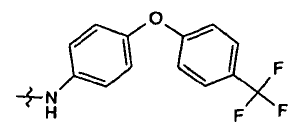 | 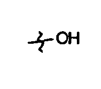 | 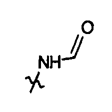 | 173 |
| 116 | 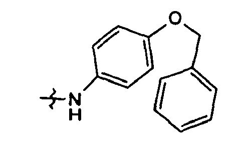 | 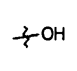 | 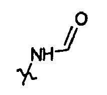 | 170 |
| 117 | 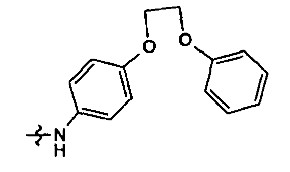 | 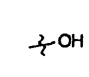 | 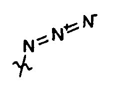 | 143 |
| 118 | 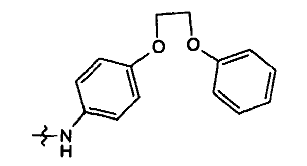 | 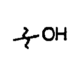 | 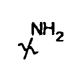 | 230 |
| 119 | 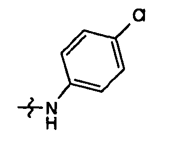 | 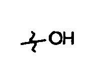 | 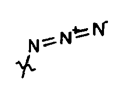 | 128 |
| 120 | 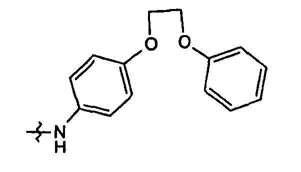 | 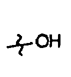 | 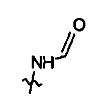 | 232 |
| 121 | 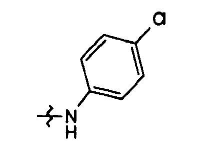 | 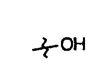 | 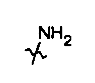 | |
| 122 | 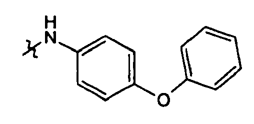 | 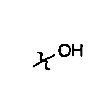 | 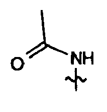 | |
| 123 | 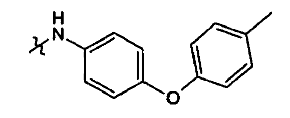 | 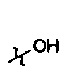 | 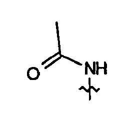 | |
| 124 | 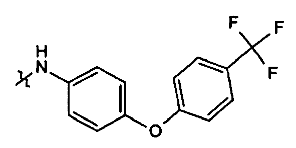 | 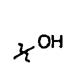 | 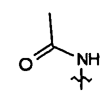 | |
| 125 | 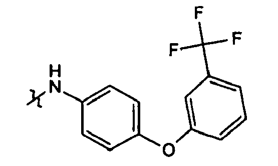 | 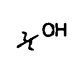 | 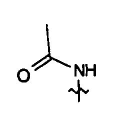 | |
| 126 | 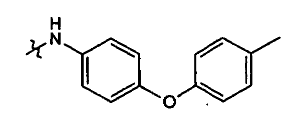 | 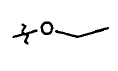 | 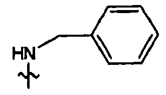 | |
| 127 | 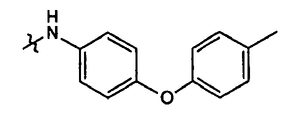 | 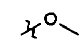 | 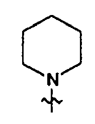 | |
| 128 | 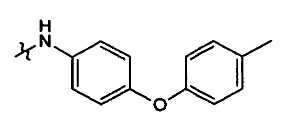 | 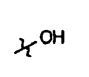 | 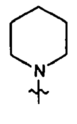 | |
| 129 | 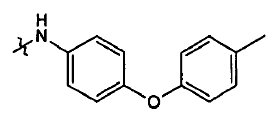 | 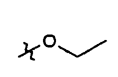 | 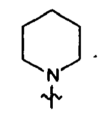 | |
| 130 | 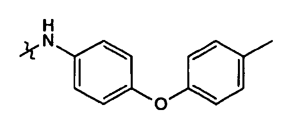 | 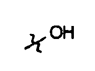 | 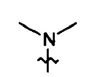 | |
| 131 | 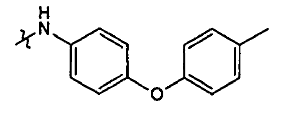 | 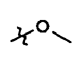 | 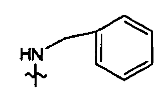 | |
| 132 | 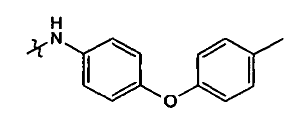 | 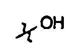 | 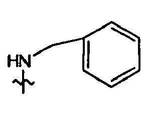 | |
| 133 | 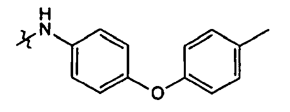 | 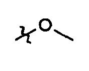 | 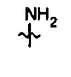 | |

### Exemple 4: Composé n° 145

### 4-chloro-3-mercapto-N-para-[3-(trifluorométhyl)phénoxy]phénylpicolinamide

À une solution de 100 mg (0,25 mmol) de 4-chloro-*N-para-*[3-(trifluorométhyl)phénoxy]phénylpicolinamide (préparée a partir de l'acide picolinique selon la méthode décrite dans *Heterocycles,* 47, (1998), 811) dans 2 mL de tétrahydrofurane anhydre à -78 °C, est ajouté en 15 minutes 0,32 mL d'une solution commerciale 2M de di-isopropylamidure de lithium. Après une heure d'agitation à -78 °C, 103 mg (0,40 mmol) de souffre sont ajoutés. Le mélange est agité encore 3 heures à -78 °C puis traité par une solution aqueuse saturée de chlorure d'ammonium. Le bain froid est ensuite retiré. Les deux phases sont séparées et la phase aqueuse est extraite avec du dichlorométhane (2x 1 mL). Les phases organiques réunies sont séchées sur sulfate de magnésium anhydre et concentrées. Le résidu est purifié par chromatographie sur gel de silice (dichlorométhane/méthanol, 97:3). On obtient 61 mg (rendement 57 %) d'un solide orange (APCI-, 423, M-1).

### Exemple 5: Composé n° 160

### 4-chloro-3-{[(4-méthoxyphényl)méthyl]thio}-N-para-[3-(trifluorométhyl)phénoxy]phénylpicolinamide

À une solution de 3,71 g (8,73 mmol) du 4-chloro-3-mercapto-*N-para-*[3-(trifluorométhyl)phénoxy]phénylpicolinamide et de 1,21 mL (1 éq.) de triéthylamine dans 75 mL de tétrahydrofurane, on ajoute lentement 1,38 mL de chlorure de 4-méthoxybenzyle. Après 24 heures d'agitation à température ambiante, le mélange réactionnel est lavé avec de l'eau, la phase organique est séchée sur sulfate de magnésium anhydre et évaporée. Le résidu est purifié par chromatographie sur gel de silice (acétate d'éthyle/heptane, 50:50). On obtient 2,94 g (rendement 62 %) d'une huile brune (APCI+, 545, M+1).

### Exemple 6 : Composé n° 173

### 4-azido-3-{[(4-méthoxyphényl)méthyl]thio}-N-para-[3-(trifluorométhyl)phénoxy]phénylpicolinamide

Un mélange de 50 mg (0,092 mmol) du 4-chloro-3-{[(4-méthoxyphényl)méthyl]thio}-*N-para*-[3-(trifluorométhyl)phénoxy]phénylpicolinamide, de 30 mg (5 éq.) d'azoture de sodium et de 1 mL de diméthylformamide est chauffé à 60 °C pendant 5 jours. Après refroidissement, le solvant est évaporé sous vide et le résidu est purifié par chromatographie sur gel de silice (acétate d'éthyle/heptane, 50:50). On obtient 29 mg (rendement 57 %) d'une huile jaune (APCI+, 552, M+1).

### Exemple 7 : Composé n° 181

### Préparation de la 4-amino-3-mercapto-N-para-[3-(trifluorométhyl)phénoxy]phénylpicolinamide

À une solution de 200 mg (0,36 mmol) de 4-azido-3-{[(4-méthoxyphényl)méthyl]thio}-*N*-*para*-[3-(trifluorométhyl)phénoxy]phénylpicolinamide dans 4 mL de tétrahydrofurane, est ajouté 228 mg (2,4 éq.) de triphénylphosphine. Le mélange est agité 18 heures à température ambiante, puis traité par 1 mL d'une solution aqueuse à 5% en acide chlorhydrique. Après 10 minutes, les deux phases sont séparées et la phase organique est lavée avec de l'eau, séchée sur sulfate de magnésium anhydre et concentrée. Le résidu est purifié par chromatographie sur gel de silice (acétate d'éthyle/heptane, 50:50). On obtient 99 mg (rendement 68 %) d'un solide blanc (APCI-, 404, M-1).

### Exemple 8 : Composé n° 164

### 4-iso-butylamino-3-{[(4-méthoxyphényl)méthyl]thio}-N-para-[3-(trifluorométhyl)phénoxy]phénylpicolinamide

Une solution de 50 mg (0,092 mmol) de 4-chloro-3-{[(4-méthoxyphényl)méthyl]thio}-*N*-*para*-[3-(trifluorométhyl)phénoxy]phénylpicolinamide dans 1 mL de *iso*-butylamine est chauffée à 60 °C pendant 24 heures. Après refroidissement, l'amine en excès est évaporée et le résidu est purifié par chromatographie sur gel de silice (acétate d'éthyle/heptane, 50:50). On obtient 29 mg (rendement 54 %) d'une huile incolore (APCI+, 582, M+1).

### Exemple 9 : Composé n° 172

### 4-iso-butylamino-3-mercapto-N-para-[3-(trifluorométhyl)phénoxy] phénylpicolinamide

Une solution de 25 mg (0,043 mmol) de 4-*iso*-butylamino-3-{[(4-méthoxyphényl)méthyl]thio}-*N*-*para*-[3-(trifluorométhyl)phénoxy]phénylpicolinamide et de 45 mL (10 équivalents) de *méta*-crésol dans 0.6 mL d'acide trifluoroacétique est chauffée à 70 °C pendant 24 heures. Après refroidissement, l'acide trifluoroacétique est évaporé et le résidu est légèrement basifié avec une solution aqueuse saturée en bicarbonate de sodium. Le mélange est neutralisé jusqu'à pH = 7 avec une solution aqueuse saturée en chlorure d'ammonium et extrait avec du dichlorométhane. Les phases organiques réunies sont séchées sur sulfate de magnésium anhydre et concentrées. Le résidu est purifié par chromatographie sur gel de silice (acétate d'éthyle/heptane, 50:50). On obtient 12 mg (rendement 38%) d'un solide jaune (APCI+, 462, M+1).

Les composés décrits dans le tableau 2 suivant sont préparés de manière analogue :

**Tableau 2**

| | | | | | |
|---|---|---|---|---|---|
| 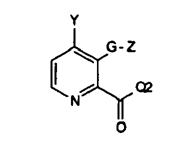 | | | | | |

| N° | Q2 | G-Z | Y | Massé théorique | Masse observée |
|---|---|---|---|---|---|
| 134 | 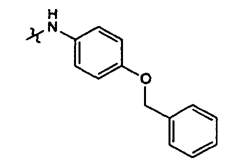 | 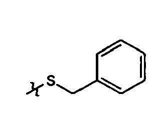 | 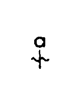 | 460 | 461 (M+1) |
| 135 | 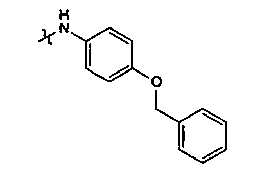 | 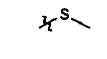 | 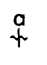 | 384 | 385 (M+1) |
| 136 | 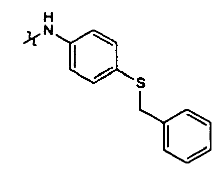 | 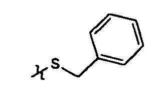 | 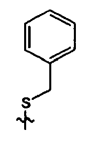 | 564 | |
| 137 | 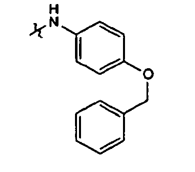 | 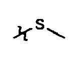 | 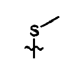 | 396 | |
| 138 | 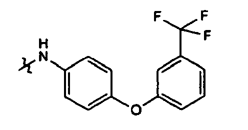 | 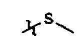 | 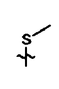 | 450 | |
| 139 | 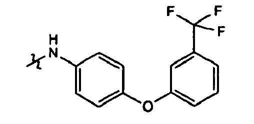 | 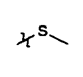 | 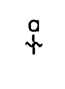 | 438 | |
| 140 | 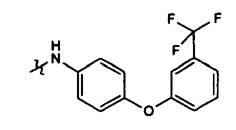 | 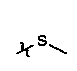 | 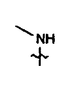 | 433 | 432 (M-1) |
| 141 | 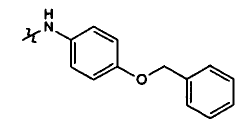 | 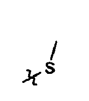 | 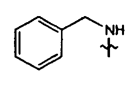 | 455 | 456 (M+1) |
| 142 | 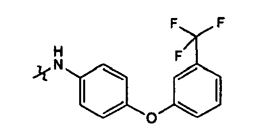 | 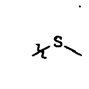 | 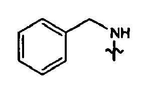 | 509 | 510 (M+1) |
| 143 | 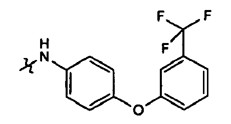 | 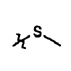 | 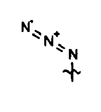 | 445 | |
| 144 | 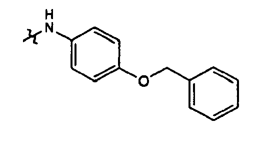 | 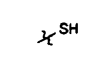 | 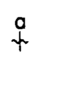 | 370 | 369 (M-1) |
| 145 | 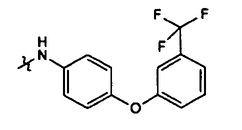 | 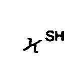 | 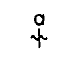 | 424 | 424 (M+1) |
| 146 | 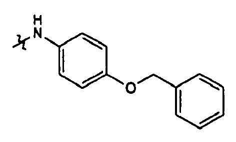 | 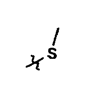 | 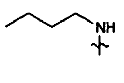 | 421 | 422 (M+1) |
| 147 | 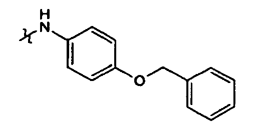 | 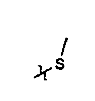 | 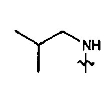 | 421 | |
| 148 | 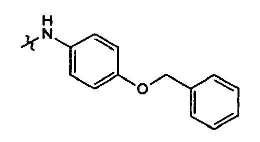 | 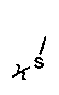 | 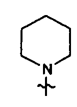 | 433 | |
| 149 | 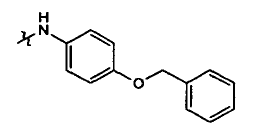 | 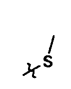 | 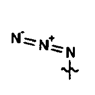 | 391 | 392 (M+1) |
| 150 | 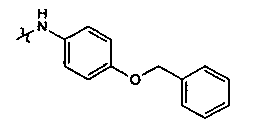 | 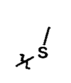 | 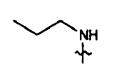 | 407 | 408 (M+1) |
| 151 | 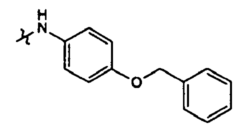 | 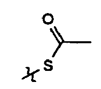 | 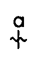 | 412 | 413 (M+1) |
| 152 | 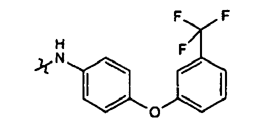 | 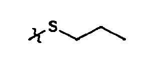 | 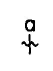 | 466 | 467 (M+1) |
| 153 | 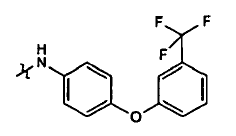 | 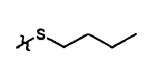 | 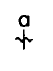 | 480 | 479 (M-1) |
| 154 | 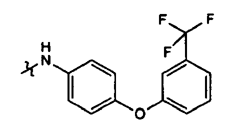 | 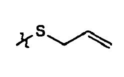 | 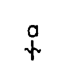 | 464 | 465 (M+1) |
| 155 | 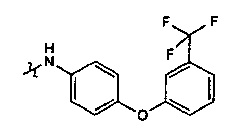 | 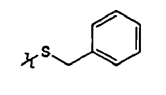 | 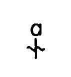 | 514 | 515 (M+1) |
| 156 | 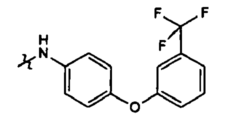 | 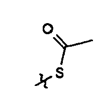 | 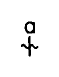 | 466 | |
| 157 | 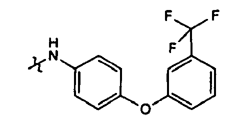 | 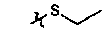 | 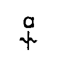 | 452 | 453 (M+1) |
| 158 | 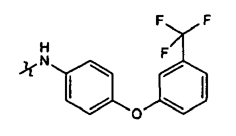 | 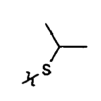 | 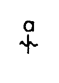 | 466 | 467 (M+1) |
| 159 | 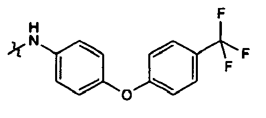 | 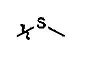 | 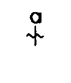 | 438 | 439 (M+1) |
| 160 | 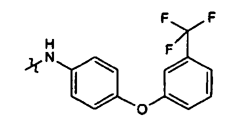 | 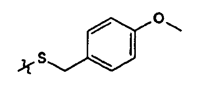 | 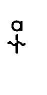 | 544 | 545 (M+1) |
| 161 | 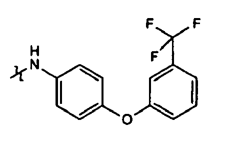 | 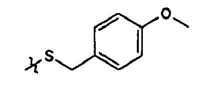 | 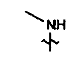 | 539 | 540 (M+1) |
| 162 | 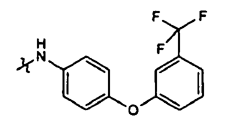 | 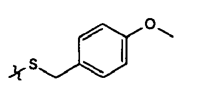 | 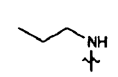 | 567 | 568 (M+1) |
| 163 | 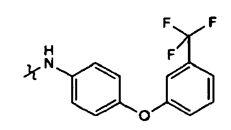 | 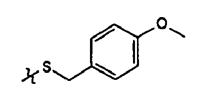 | 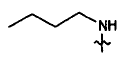 | 581 | 582 (M+1) |
| 164 | 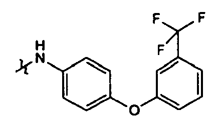 | 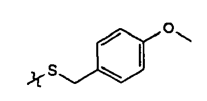 | 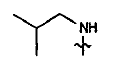 | 581 | 582 (M+1) |
| 165 | 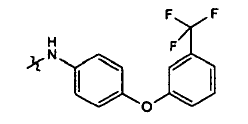 | 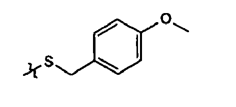 | 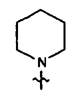 | 593 | 592 (M-1) |
| 166 | 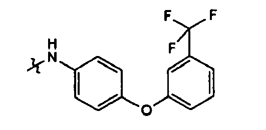 | 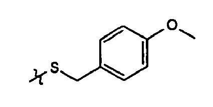 | 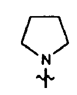 | 579 | 580 (M+1) |
| 167 | 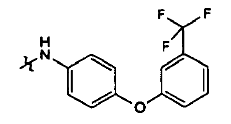 | 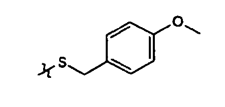 | 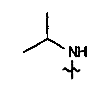 | 567 | 568 (M+1) |
| 168 | 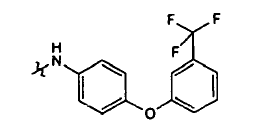 | 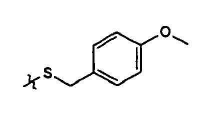 | 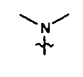 | 553 | 554 (M+1) |
| 169 | 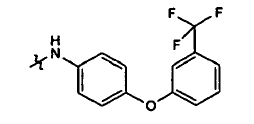 | 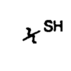 | 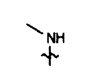 | 419 | 418 (M-1) |
| 170 | 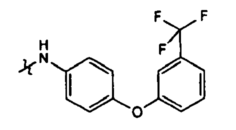 | 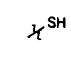 | 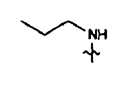 | 447 | 448 (M+1) |
| 171 | 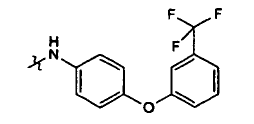 | 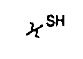 | 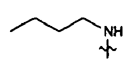 | 461 | |
| 172 | 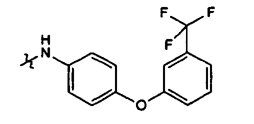 | 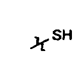 | 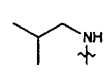 | 461 | 460 (M-1) |
| 173 | 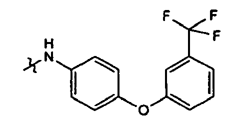 | 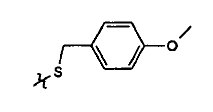 | 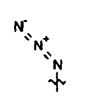 | 551 | 552 (M+1) |
| 174 | 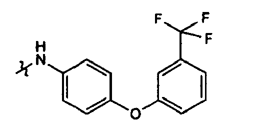 | 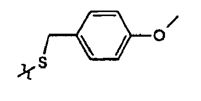 | 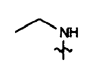 | 553 | 554 (M+1) |
| 175 | 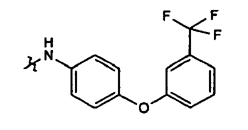 | 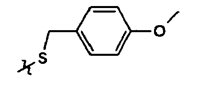 | 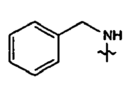 | 615 | 616 (M+1) |
| 176 | 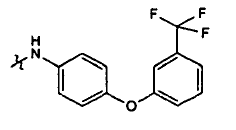 | 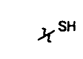 | 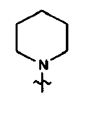 | 473 | |
| 177 | 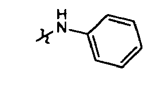 | 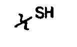 | 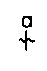 | 264 | 265 (M+1) |
| 178 | 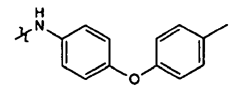 | 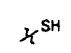 | 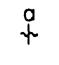 | 370 | 371 (M+1) |
| 179 | 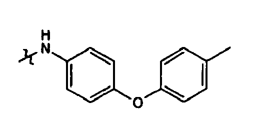 | 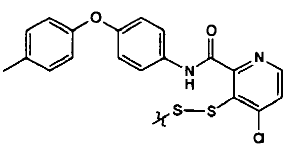 | 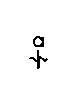 | 738 | 369 (M/2) |
| 180 | 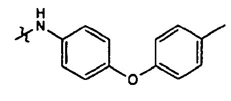 | 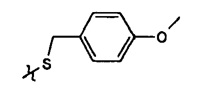 | 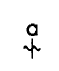 | 490 | 491 (M+1) |
| 181 | 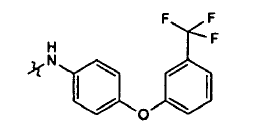 | 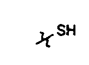 | 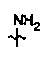 | 405 | 404 (M-1) |
| 182 | 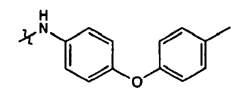 | 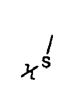 | 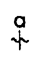 | 384 | 385 (M+1) |
| 183 | 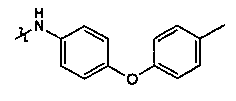 | 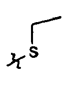 | 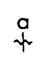 | 398 | 399 (M+1) |
| 184 | 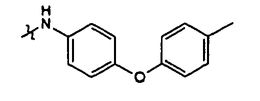 | 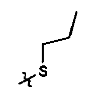 | 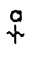 | 412 | 413 (M+1) |
| 185 | 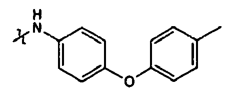 | 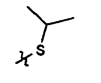 | 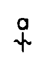 | 412 | 413 (M+1) |
| 186 | 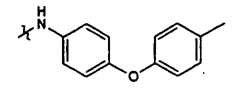 | 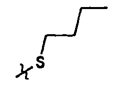 | 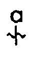 | 426 | 427 (M+1) |
| 187 | 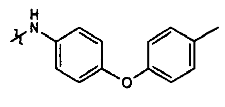 | 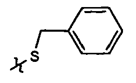 | 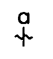 | 460 | 461 (M+1) |
| 188 | 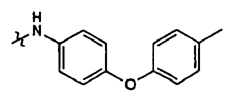 | 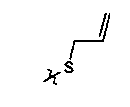 | 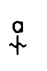 | 410 | 411 (M+1) |
| 189 | 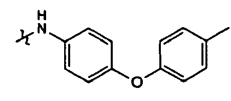 | 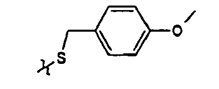 | 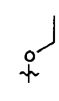 | 500 | 501 (M+1) |
| 190 | 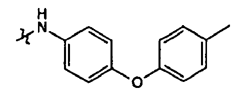 | 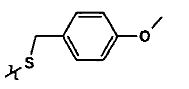 | 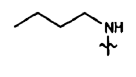 | 527 | 528 (M+1) |
| 191 | 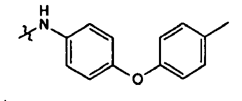 | 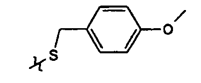 | 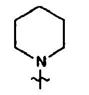 | 539 | 554 (M+1) |
| 192 | 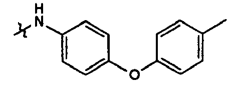 | 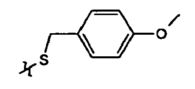 | 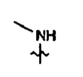 | 485 | 486 (M+1) |
| 193 | 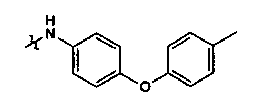 | 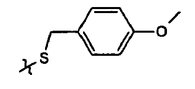 | 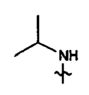 | 513 | 513 (M+1) |
| 194 | 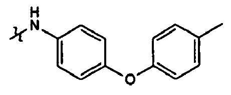 | 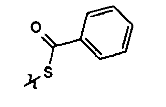 | 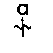 | 474 | 474 (M+1) |
| 195 | 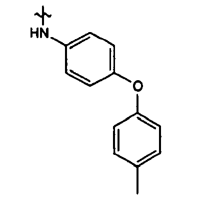 | 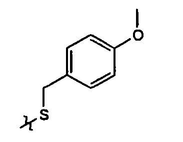 | 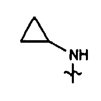 | 511 | 511 (M+1) |
| 196 | 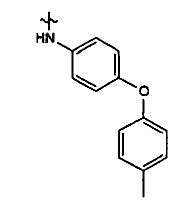 | 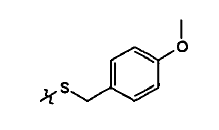 | 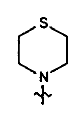 | 557 | 557 (M+1) |
| 197 | 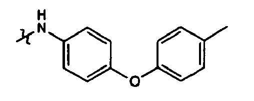 | 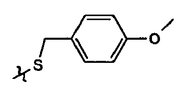 | 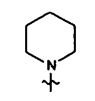 | 539 | 540 (M+1) |
| 198 | 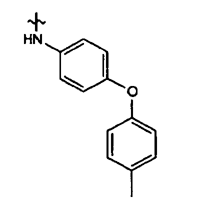 | 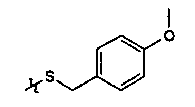 | 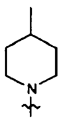 | 553 | 553 (M+1) |
| 199 | 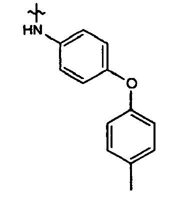 | 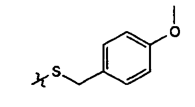 | 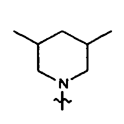 | 567 | 567 (M+1) |
| 200 | 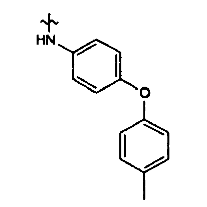 | 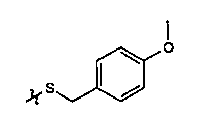 | 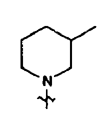 | 553 | 553 (M+1) |
| 201 | 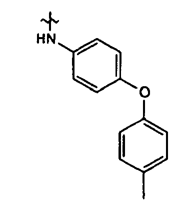 | 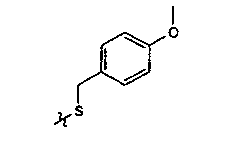 | 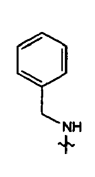 | 561 | 561 (M+1) |
| 202 | 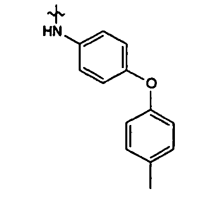 | 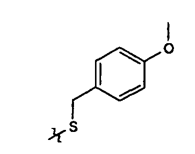 | 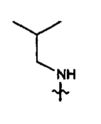 | 527 | 527 (M+1) |
| 203 | 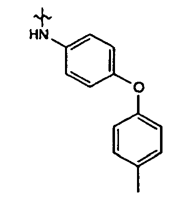 | 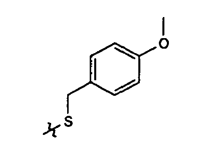 | 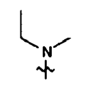 | 513 | 513 (M+1) |
| 204 | 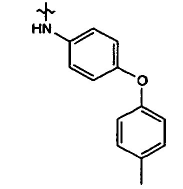 | 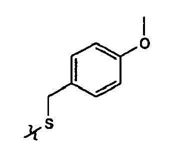 | 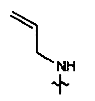 | 511 | 511 (M+1) |
| 205 | 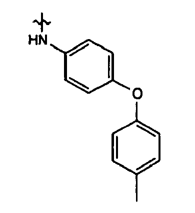 | 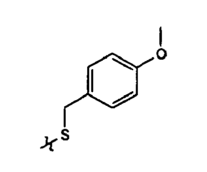 | 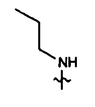 | 513 | 513 (M+1) |
| 206 | 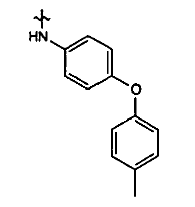 | 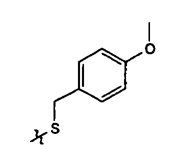 | 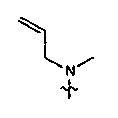 | 525 | 525 (M+1) |
| 207 | 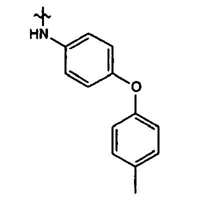 | 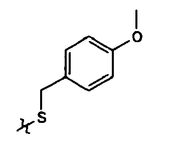 | 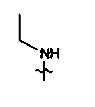 | 499 | 499 (M+1) |
| 208 | 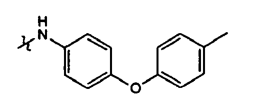 | 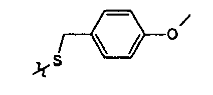 | 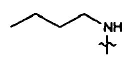 | 527 | 528 (M+1) |
| 209 | 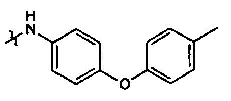 | 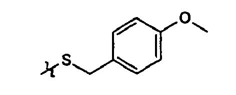 | 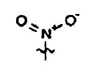 | 501 | |
| 210 | 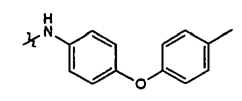 | 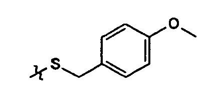 | 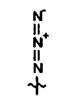 | 497 | 470 (M+1-28) |
| 211 | 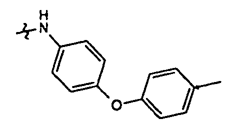 | 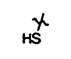 | 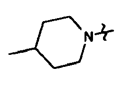 | 433 | 432 (M-1) |
| 212 | 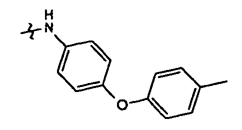 | 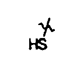 | 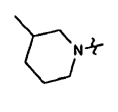 | 433 | 432 (M-1) |
| 213 | 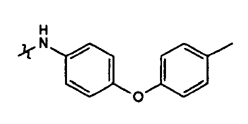 | 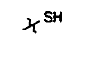 | 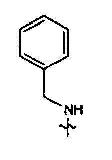 | 441 | 442 (M+1) |
| 214 | 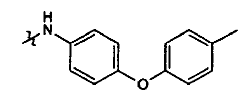 | 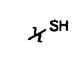 | 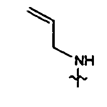 | 391 | 392 (M+1) |
| 215 | 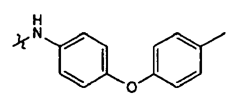 | 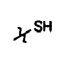 | 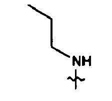 | 393 | 394 (M+1) |

### Exemples d'activités biologiques des composés de l'invention

### Exemple A : Test in vivo sur Alternaria brassicae (Alternariose des crucifères) :

Une suspension aqueuse, de concentration 2 g/L, de la matière active testée est obtenue par broyage fin de celle-ci dans le mélange suivant :
- eau
- agent tensioactif Tween 80 (oléate de dérivé polyoxyéthyléné du sorbitan) dilué à 10% dans l'eau : 5 mL/mg de matière active
- argile : support inerte q.s.p. 100%.

Cette suspension aqueuse est ensuite diluée par de l'eau de manière à obtenir la concentration désirée en matière active.

Des plants de radis (variété Pernot) en godets, semés sur un substrat tourbe terre-pouzzolane 50/50 et cultivés à 18-20°C, sont traités au stade cotylédons par pulvérisation de la suspension aqueuse décrite ci-dessus.

Des plants, utilisés comme témoins sont traités par une solution aqueuse ne contenant pas la matière active.

Après 24 heures, les plants sont contaminés par pulvérisation d'une suspension aqueuse de spores (40 000 spores par cm³) *d'Alternaria brassicae.* Les spores sont récoltées sur une culture âgée de 12-13 jours.

Les plants de radis contaminés sont mis en incubation pendant 6-7 jours à 18°C environ, en atmosphère humide.

La notation est effectuée 6 à 7 jours après la contamination, en comparaison avec les plants témoins.

Dans ces conditions, on observe à la dose de 250 g/ha, une protection bonne (au moins 50%) ou totale avec les composés suivants : 108, 110, 112, 115, 116, 130, 133.

### Exemple B : Test in vivo sur Septoria nodorum (septoriose du blé) :

Une suspension aqueuse, de concentration 2 g/L de la matière active testée est obtenue par broyage fin de celle-ci dans le mélange suivant :
- eau,
- agent tensioactif Tween 80 (oléate de dérivé polyoxyéthyléné du sorbitan) dilué à 10% dans l'eau : 5 mL/mg de matière active,
- argile : support inerte q.s.p. 100%.

Cette suspension aqueuse est ensuite diluée par de l'eau de manière à obtenir la concentration désirée en matière active.

Des plants blé (variété Scipion) en godets, semés sur un substrat tourbe terre-pouzzolane 50/50 et cultivés à 12°C, sont traités au stade 1 feuille (10 cm de hauteur) par pulvérisation de la suspension aqueuse décrite ci-dessus.

Des plants, utilisés comme témoins, sont traités par une solution aqueuse ne contenant pas la matière active.

Après 24 heures, les plants sont contaminés par pulvérisation d'une suspension aqueuse de spores (500 000 spores par cm³) de *Septoria nodorum.* Les spores sont récoltées sur une culture âgée de sept jours.

Les plants de blé contaminés sont mis en incubation pendant 72 heures à 18°C environ, en atmosphère humide, puis pendant 14 jours à 90 % d'humidité relative.

La notation est effectuée 15 à 20 jours après la contamination, en comparaison avec les plants témoins.

Dans ces conditions, on observe, à la dose de 250 g/ha, une protection bonne (au moins 50%) ou totale avec les composés suivants : 108, 110, 112, 133.

### Exemple C : Test in vivo sur Magnaporthe grisea (pyriculariose du riz) :

Une suspension aqueuse, de concentration 50 mg/L, de la matière active testée est obtenue par broyage fin de celle-ci dans le mélange suivant :
- eau,
- acétone 2%.

Des plants de riz (variété Koshihirakari), semés sur du sol de KUREHA et cultivés en pot de plastique de 33 cm² jusqu'au stade 3-4 feuilles, sont traités par pulvérisation de la suspension aqueuse précédemment décrite.

Des plants, utilisés comme témoins, sont traités par une solution aqueuse ne contenant pas de matière active

24 heures après traitement, les plants sont contaminés par pulvérisation d'une suspension aqueuse de spores (500000 spores par cm³) de *Magnaporthe grisea.*

Les plants de riz contaminés sont placés en incubateur pendant 24 heures à 25°C en atmosphère humide, puis pendant 5 à 7 jours dans une chambre d'incubation à 20-25°C et 70-90% d'humidité relative

La notation est effectuée 5 à 7 jours après la contamination par comptage des lésions sur la première feuille du plant.

Dans ces conditions, on observe, à la dose de 50 mg/l, une protection bonne (au moins 50%) ou totale avec les composés suivants : 62, 114, 115.

### Exemple D : Test in vivo sur Erisyphe graminis f. sp. tritici (oïdium du blé) :

Une suspension aqueuse, de concentration 2 g/L, de la matière active testée est obtenue par broyage fin de celle-ci dans le mélange suivant :
- eau,
- agent tensioactif Tween 80 (oléate de dérivé polyoxyéthyléné du sorbitan) dilué à 10% dans l'eau : 5 mL/mg de matière active,
- argile : support inerte q.s.p. 100%.

Cette suspension aqueuse est ensuite diluée par de l'eau de manière à obtenir la concentration désirée en matière active.

Des plants blé (variété Audace) en godets, semés sur un substrat tourbe terre - pouzzolane 50/50 et cultivés à 12°C, sont traités au stade 1 feuille (10 cm de hauteur) par pulvérisation de la suspension aqueuse décrite ci-dessus.

Des plants, utilisés comme témoins sont traités par une solution aqueuse ne contenant pas la matière active.

Après 24 heures, les plants sont contaminés par saupoudrage avec des spores d'*Erisyphe graminis* f. sp. *tritici ,* le saupoudrage étant effectué à l'aide de plants malades.

La notation est effectuée 7 à 14 jours après la contamination, en comparaison avec les plants témoins.

Dans ces conditions, on observe à la dose de 500 g/ha, une protection bonne (au moins 50%) ou totale avec le composé décrit dans l'exemple : 108.

### Exemple E: Test in vivo sur Rhizoctonia solani (Rhizoctone du riz) :

Une suspension aqueuse, de concentration 100 mg/L, de la matière active testée est obtenue par broyage fin de celle-ci dans le mélange suivant :
- eau,
- acétone 2%

Des plants de riz (variété Koshihirakari), semés sur du sol de KUREHA et cultivés en pot de plastique de 33 cm² jusqu'au stade 7-8 feuilles, sont traités par pulvérisation de la suspension aqueuse précédemment décrite.

Des plants, utilisés comme témoins, sont traités par une solution aqueuse ne contenant pas de matière active.

Après 24 heures, chaque plant est contaminé par dépôt d'un disque gélosé de culture fongique, prélevé sur une culture de *Rhizoctonia solani* sur PDA, entre la tige et la gaine.

Les plants contaminés sont ensuite incubés pendant 24 heures à 25°C dans une atmosphère à 100% d'humidité et ensuite incubés pendant 5-7 jours à une humidité relative comprise entre 70-90%.

La notation est effectuée 7 jours après la contamination, par la mesure de la hauteur de la lésion sur le plant en comparaison avec les contrôles.

Dans ces conditions, à une dose de 100 ppm, une bonne protection (au moins 50%) est observée avec les composés décrits comme exemple : 62, 133.

### Exemple F : Test in vivo sur Septoria tritici (septoriose du blé) :

Une suspension aqueuse, de concentration 1,5%, de la matière active testée est obtenue par broyage fin de celle-ci dans une formulation de type suspension concentrée, par exemple telle que celle décrite au paragraphe [0061] ci-dessus (formulation SC1, SC2 ou SC3).

Cette suspension aqueuse est ensuite diluée dans de l'eau de manière à obtenir la concentration désirée en matière active, soit 2 g/L.

Des plants de blé (variété Scipion) en godets, semés sur un substrat tourbe - pouzzolane 50/50 et cultivés à 12°C, sont traités au stade 1 feuille (10 cm de hauteur) par pulvérisation de la suspension aqueuse décrite précédemment.

Des plants, utilisés comme témoins, sont traités par une solution aqueuse ne contenant pas la matière active.

Après 24 heures, les plants sont contaminés par pulvérisation d'une suspension aqueuse de spores (500000 spores par mL) de *Septoria tritici.* Les spores sont récoltées sur une culture âgée de 15 jours et mises en suspension dans une solution nutritive composée de :
- 1.5 g/L de gélatine
- 0.5 g/L d'oléate de sodium
- 24 g/L de PDB

Les plants de blé contaminés sont mis en incubation pendant 72 heures à 20°C environ, à 100% d'humidité relative, puis pendant 15 jours à 80% d'humidité relative.

La notation est effectuée 15 à 20 jours après la contamination, en comparaison avec les plants témoins. Dans ces conditions, à une dose de 500 g/ha, une bonne protection (au moins 50%) est observée avec les composés décrits comme exemple : 108.

## Revendications

1. Composés de formule générale (I) : 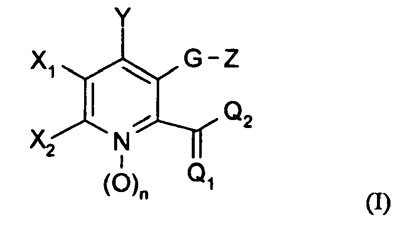 dans lesquels :
• G représente un atome d'oxygène ou de soufre,
• n représente 0 ou 1,
• Q₁ est un atome d'oxygène,
• Q₂ est le groupe -NR₄R₅,
• Z est choisi parmi l'atome d'hydrogène, le radical cyano, un radical alkyle, allyle, aryle, arylalkyle, propargyle, cycloalkyle, halocycloalkyle, alcényle, alcynyle, cyanoalkyle, haloalkyle, alkoxyalkyle, haloalkoxyalkyle, alkylthioalkyle, haloalkylthioalkyle, N-alkylaminoalkyle, N,N-dialkylaminoalkyle, acylaminoalkyle, alkoxycarbonylaminoalkyle, aminocarbonylaminoalkyle, alkoxycarbonyle, N-alkylamino-carbonyle, N,N-dialkylaminocarbonyle, acyle, thioacyle, alkoxythiocarbonyle, N-alkylaminothiocarbonyle, N,N-dialkylaminothiocarbonyle, alkylsulfinyle, haloalkylsulfinyle, alkylsulfonyle, haloalkylsulfonyle, alkoxysulfonyle, aminosulfonyle, N-alkylaminosulfonyle, N,N-dialkylaminosulfonyle, arylsulfinyle, arylsulfonyle, aryloxysulfonyle, N-arylaminosulfonyle, N,N-diarylaminosulfonyle, et N,N-arylalkylaminosulfonyle ;
• Y est choisi parmi un atome d'halogène, le radical hydroxy, mercapto, nitro, thiocyanato, azido, cyano, pentafluorosulfonyle, un radical alkyle, haloalkyle, alkylthio, haloalkylthio, alkoxyalkyle, haloalkoxyalkyle, alkylthioalkyle, haloalkylthioalkyle, cyanoalkyle, cyanoalkoxy, cyanoalkylthio, alkylsulfinyle, haloalkylsulfinyle, alkylsulfonyle, haloalkylsulfonyle, alkoxysulfonyle,
un groupe cycloalkyle, halocycloalkyle, alcényle, alcynyle, alcényloxy, alcynyloxy, alcénylthio, alcynylthio,
un groupe amino, N-alkylamino, N,N-dialkylamino, -NHCOR₁₀, -NHCSR₁₀, N-alkylaminocarbonylamino, N,N-dialkylaminocarbonylamino, aminoalkyle, N-alkyl-aminoalkyle, N,N-dialkylaminoalkyle, acylaminoalkyle, thioacylamino, alkoxythiocarbonylamino, N-alkylaminothiocarbonylamino, N,N-dialkylaminothiocarbonylamino, N,N-arylalkylaminocarbonylamino, N-alkylsulfinylamino, N-alkylsulfonylamino, N-alkyl-(alkylsulfonyl)amino, N-arylsulfinylamino, N-arylsulfonylamino, N-alkoxysulfonylamino, N-alkoxysulfinylamino, N-haloalkoxysulfinyl-amino, N-haloalkoxysulfonylamino, N-arylamino, N,N-diarylamino, arylcarbonylamino, alkoxycarbonylamino, N-arylaminocarbonylamino, N,N-diarylaminocarbonylamino, arylthiocarbonylamino, aryloxythiocarbonylamino, N-arylaminothiocarbonylamino, N,N-diarylaminothiocarbonylamino, N,N-arylalkylaminothiocarbonylamino,
un radical acyle, carboxy, carbamoyle, N-alkylcarbamoyle, N,N-dialkylcarbamoyle, alkoxycarbonyle inférieur, N-arylcarbamoyle, N,N-diarylcarbamoyle, aryloxycarbonyle, N,N-arylalkylcarbamoyle, et un groupe imino de formule : 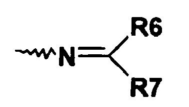
• X₁ et X₂ sont identiques ou différents et sont choisis indépendamment l'un de l'autre parmi l'atome d'hydrogène, un atome d'halogène, le radical hydroxy, mercapto, nitro, thiocyanato, azido, cyano, pentafluorosulfonyle, un radical alkyle, haloalkyle, alkoxy, haloalkoxy, alkylthio, haloalkylthio, alkoxyalkyle, haloalkoxyalkyle, alkylthioalkyle, haloalkylthioalkyle, cyanoalkyle, cyanoalkoxy, cyanoalkylthio, alkylsulfinyle, haloalkylsulfinyle, alkylsulfonyle, haloalkylsulfonyle, et alkoxysulfonyle, ou bien
• X₁ et X₂ peuvent également être joints ensemble, formant ainsi un cycle de 4 à 8 chaînons, saturé, partiellement insaturé ou totalement insaturé, et comportant éventuellement un ou plusieurs hétéroatomes choisis parmi le soufre, l'oxygène, l'azote et le phosphore,
• R₄ et R₅ sont identiques ou différents et sont choisis indépendamment l'un de l'autre parmi l'atome d'hydrogène et un radical aryle éventuellement substitué par un ou plusieurs radicaux choisi indépendamment les uns des autres parmi R₉, aryle, arylalkyle et un groupement -T-R₈,
• T représente une liaison directe ou un radical divalent choisi parmi un radical -(CH₂)ₘ-, m prenant une valeur comprise entre 1 et 12, bornes incluses, le dit radical étant éventuellement interrompu ou borné par un ou deux hétéroatomes choisis parmi l'azote, l'oxygène et/ou le soufre, un radical oxyalkylène, alkoxyalkylène, carbonyle (-CO-), oxycarbonyle(-O-CO-), carbonyloxy(-CO-O-), sulfinyle (-SO-), sulfonyle (-SO₂-), oxysulfonyle (-O-SO₂-), sulfonyloxy (-SO₂-O-), oxysulfinyle (-O-SO-), sulfinyloxy (-SO-O-), thio (-S-), oxy (-O-), vinyle (-C=C-), éthinyle (-C≡C-), -NR₉-, -NR₉O-, -ONR₉-, -N=N-, -NR₉-NR₁₀-, -NR₉-S-, -NR₉-SO-, -NR₉-SO₂-, -S-NR₉-, -SO-NR₉-, -SO₂-NR₉-, -CO-NR₉-O-, et -O-NR₉-CO-,
• R₈ est choisi parmi l'atome d'hydrogène, un radical aryle ou hétérocyclyle,
• R₉ et R₁₀, identiques ou différents, sont choisis indépendamment l'un de l'autre parmi l'atome d'hydrogène, un atome d'halogène, le radical hydroxy, mercapto, nitro, thiocyanato, azido, cyano ou pentafluorosulfonyle, un radical alkyle, haloalkyle, alkoxy, haloalkoxy, alkylthio, haloalkylthio, alkoxyalkyle, haloalkoxyalkyle, alkylthioalkyle, haloalkylthioalkyle, arylalkyle, cyanoalkyle, cyanoalkoxy, cyanoalkylthio, alkylsulfinyle, haloalkylsulfinyle, alkylsulfonyle, haloalkylsulfonyle, et alkoxysulfonyle,
ainsi que les éventuels N-oxydes, isomères géométriques et/ou optiques, énantiomères et/ou diastéréoisomères, formes tautomères, les sels, complexes métalliques et métalloïdiques des composés de formule (I) tels qu'ils viennent d'être définis,
avec la restriction que lorsque G-Z est un groupe hydroxy, R₄ est un atome d'hydrogène et R₅ représente le groupe 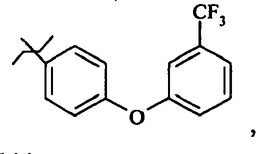 alors Y ne peut être ni un groupe methyl ni un groupe methylthio.

2. Composés selon la revendication 1 pour lesquels X₁ et X₂ représentent chacun un atome d'hydrogène.

3. Composés selon la revendication 1 pour lesquels Q₁ est choisi parmi l'atome d'oxygène et de soufre.

4. Composés selon la revendication 1 pour lesquels Z est choisi parmi un radical alkyle et l'atome d'hydrogène ou un radical alkoxyalkyle, haloalkoxy-alkyle, alkylthioalkyle, haloalkylthioalkyle, N-alkylamino-alkyle, N,N-dialkylamino-alkyle, acylamino-alkyle, acyle, thioacyle, cyanoalkyle, alkoxythiocarbonyle, N-alkylaminothiocarbonyle, N,N-dialkylaminothiocarbonyle, ou alkylsulfinyle.

5. Composés selon la revendication 1 pour lesquels Y est choisi parmi un atome d'halogène, le radical hydroxy, mercapto, nitro, thiocyanato, azido, cyano, pentafluorosulfonyle, un radical alkyle, haloalkyle, alkylthio, haloalkylthio, alkoxyalkyle, haloalkoxyalkyle, alkylthioalkyle, haloalkylthioalkyle, alkylsulfinyle, haloalkylsulfinyle, alkylsulfonyle, haloalkylsulfonyle, alkoxysulfonyle, un groupe amino, N-alkylamino, N,N-dialkylamino, -NHCOR₁₀, -NHCSR₁₀, N-arylamino, N,N-diarylamino, arylcarbonylamino, arylthiocarbonylamino, aryloxythiocarbonylamino, et N,N-arylalkylaminothiocarbonylamino.

6. Composés selon la revendication 1 possédant les caractéristiques suivantes prises isolément ou en combinaison :
• X₁ et X₂ représentent chacun un atome d'hydrogène,
• Z est choisi parmi un radical alkyle et l'atome d'hydrogène ou un radical clivable susceptible de redonner l'hydrogène, par exemple, alkoxyalkyle, haloalkoxy-alkyle, alkylthioalkyle, haloalkylthioalkyle, N-alkylaminoalkyle, N,N-dialkylaminoalkyle, acylaminoalkyle, acyle, thioacyle, cyanoalkyle, alkoxythiocarbonyle, N-alkylaminothiocarbonyle, N,N-dialkylaminothiocarbonyle, ou alkylsulfinyle,
• Y est choisi parmi un atome d'halogène, le radical hydroxy, mercapto, nitro, thiocyanato, azido, cyano, pentafluorosulfonyle, un radical alkyle, haloalkyle, alkylthio, haloalkylthio, alkoxyalkyle, haloalkoxyalkyle, alkylthioalkyle, haloalkylthioalkyle, alkylsulfinyle, haloalkylsulfinyle, alkylsulfonyle, haloalkylsulfonyle, alkoxysulfonyle, un groupe amino, N-alkylamino, N,N-dialkylamino, -NHCOR₁₀, -NHCSR₁₀, N-arylamino, N,N-diarylamino, arylcarbonylamino, arylthiocarbonylamino, aryloxythiocarbonylamino, N,N-arylalkylaminothiocarbonylamino,
• Q₁ est choisi parmi l'atome d'oxygène et de soufre.

7. Composés selon la revendication 1 ou la revendication 6 possédant les caractéristiques suivantes :
• X₁ et X₂ représentent chacun un atome d'hydrogène,
• Z est choisi parmi un radical alkyle et l'atome d'hydrogène ou un radical clivable susceptible de redonner l'hydrogène, par exemple, alkoxyalkyle, haloalkoxy-alkyle, alkylthioalkyle, haloalkylthioalkyle, N-alkylaminoalkyle, N,N-dialkylaminoalkyle, acylaminoalkyle, acyle, thioacyle, cyanoalkyle, alkoxythiocarbonyle, N-alkylaminothiocarbonyle, N,N-dialkylaminothiocarbonyle, ou alkylsulfinyle,
• Y est choisi parmi un atome d'halogène, le radical hydroxy, mercapto, nitro, thiocyanato, azido, cyano, pentafluorosulfonyle, un radical alkyle, haloalkyle, alkylthio, haloalkylthio, alkoxyalkyle, haloalkoxyalkyle, alkylthioalkyle, haloalkylthioalkyle, alkylsulfinyle, haloalkylsulfinyle, alkylsulfonyle, haloalkylsulfonyle, alkoxysulfonyle, un groupe amino, N-alkylamino, N,N-dialkylamino, -NHCOR₁₀, -NHCSR₁₀, N-arylamino, N,N-diarylamino, arylcarbonylamino, arylthiocarbonylamino, aryloxythiocarbonylamino, N,N-arylalkylaminothiocarbonylamino,
• Q₁ est choisi parmi l'atome d'oxygène et de soufre.

8. Composés selon l'une des revendications précédentes possédant les caractéristiques suivantes :
• X₁ et X₂ représentent chacun un atome d'hydrogène,
• Z est choisi parmi un radical alkyle et l'atome d'hydrogène ou un radical clivable susceptible de redonner l'hydrogène, par exemple, alkoxyalkyle, haloalkoxy-alkyle, alkylthioalkyle, haloalkylthioalkyle, N-alkylaminoalkyle, N,N-dialkylaminoalkyle, acylaminoalkyle, acyle, thioacyle, cyanoalkyle, alkoxythiocarbonyle, N-alkylaminothiocarbonyle, N,N-dialkylaminothiocarbonyle, ou alkylsulfinyle,
• Y est choisi parmi un atome d'halogène, le radical hydroxy, azido, alkylthio, alkylsulfonyle, un groupe amino, -NHCOR₁₀, et -NHCSR₁₀,
• Q₁ représente l'atome d'oxygène.

9. Composés selon l'une des revendications précédentes qui sont :
• le 4-amino-3-hydroxy-N-[4-(4-méthylphénoxy)phényl]-2-pyridine carboxamide,
• le 4-(formylamino)-3-hydroxy-N-{4-[3-(trifluorométhyl)phénoxy] phényl}-2-pyridine carboxamide,
• le 4-amino-3-hydroxy-N-{4-[4-(trifluorométhyl)phénoxy]phényl}-2-pyridine carboxamide,
• le N-[4-(4-chlorophénoxy)phényl]-4-(formylamino)-3-hydroxy-2-pyridine carboxamide,
• le 4-(formylamino)-3-hydroxy-N-{4-[4-(trifluorométhyl)phénoxy] phényl}-2-pyridine carboxamide, et
• le N-[4-(benzyloxy)phényl]-4-(formylamino)-3-hydroxy-2-pyridine carboxamide.

10. Procédé de préparation des composés selon l'une des revendications 1 à 9, dans lesquels G représente l'oxygène, **caractérisé en ce que** l'on fait réagir un composé de formule (IIa) : 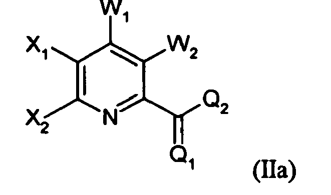 dans lesquels X₁, X₂, Q₁ et Q₂ sont tels que définis dans la revendication 1, et W₁ et W₂, identiques ou différents, représentent indépendamment l'un de l'autre un atome d'halogène choisi parmi fluor, chlore, brome ou iode,
avec un sel de l'acide azothydrique, cette réaction étant conduite dans un solvant polaire aprotique, au reflux ou à une température comprise entre 20°C et 200°C, pour conduire aux composés de formule (IIIa) : 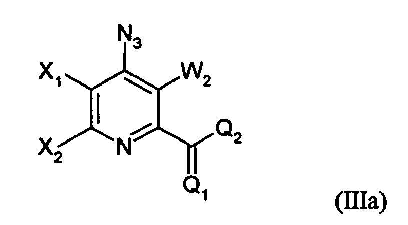 dans lesquels X₁, X₂, Q₁ et Q₂ sont tels que définis précédemment, et W₂ représente un atome d'halogène choisi parmi fluor, chlore, brome ou iode,
qui sont alors éventuellement réduits en dérivés aminés correspondants de formule (IVa) : 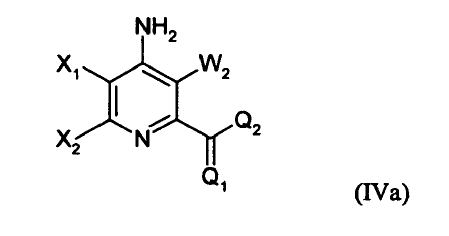 dans lesquels X₁, X₂, Q₁ et Q₂ sont tels que définis précédemment, et W₂ représente un atome d'halogène choisi parmi fluor, chlore, brome ou iode,
par action d'un agent réducteur, en présence d'un catalyseur,
les dérivés halogénés de formule (IVa) pouvant être alors hydrolysés en dérivés 3-hydroxypicoliniques de formule (Va) : 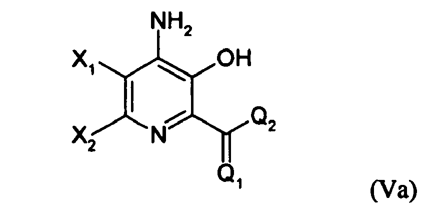 dans lesquels X₁, X₂, Q₁ et Q₂ sont tels que définis précédemment,
par action d'une base inorganique, cette réaction est généralement effectuée à une température comprise entre 0°C et la température d'ébullition du solvant, dans un solvant polaire aprotique dipolaire,
les composés de formule (Va) pouvant ensuite être soumis à diverses réactions d'alkylation bien connues de l'homme du métier afin de conduire aux composés de formule (VIa) : 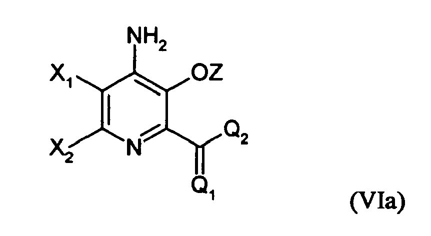 dans lesquels X₁, X₂, Z, Q₁ et Q₂ sont tels que définis dans la revendication 1.

11. Procédé de préparation des composés selon l'une des revendications 1 à 9, dans lesquels G représente le soufre, **caractérisé en ce que** l'on fait réagir un composé de formule (IIb) : 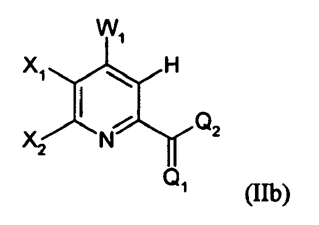 dans lesquels X₁, X₂, Q₁ et Q₂ sont tels que définis dans la revendication 1, et W₁ représente un atome d'halogène choisi parmi fluor, chlore, brome ou iode,
avec une base organique, pour conduire aux composés de formule (IIIb) : 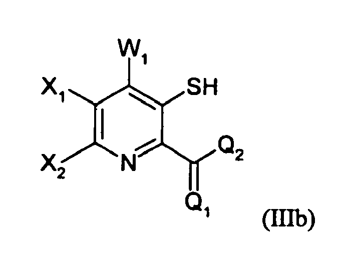 dans lesquels X₁, X₂, W₁ Q₁ et Q₂ sont tels que définis dans la revendication 1,
les thiols de formule (IIIb) pouvant être ensuite transformés en composés de formule (IVb) : 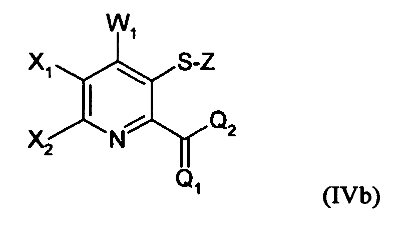 dans lesquels X₁, X₂, W₁, Q₁ et Q₂ sont tels que définis dans la revendication 1,
par réaction avec un agent alkylant, les composés de formule (IVb) pouvant ensuite être transformés en composés 4-aminés de formule (Vb) 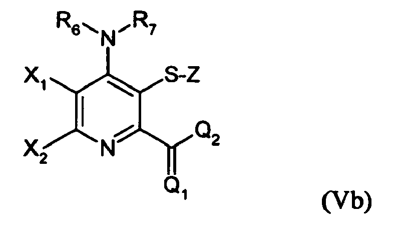 dans lesquels X₁, X₂, Q₁, Q₂, Z, R₆ et R₇ sont tels que définis dans la revendication 1, en faisant réagir un composé de formule R₆R₇NH, ou un sel alcalin ou alcalino-terreux correspondant, en l'absence de solvant ou dans un solvant aprotique polaire, à une température comprise entre 0°C et la température d'ébullition du solvant, les composés de formules (Vb) dans lesquels X₁, X₂, Q₁, Q₂, R₆ et R₇ sont tels que définis dans la revendication 1, et Z représente un groupement 4-méthoxybenzilique, pouvant être transformés en 3-thiopyridine correspondant par réaction avec un acide organique, cette réaction étant conduite dans un solvant polaire protique, au reflux ou à une température comprise entre 20°C et 200°C,
pour conduire aux composés de formule (VIb) : 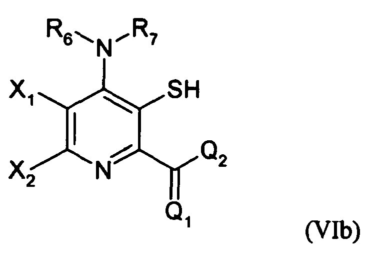 dans lesquels X₁, X₂, Q₁, Q₂, R₆ et R₇ sont tels que définis dans la revendication 1, les composés de formule (IVb) pouvant être transformés également en azotures de formule (VIIb) : 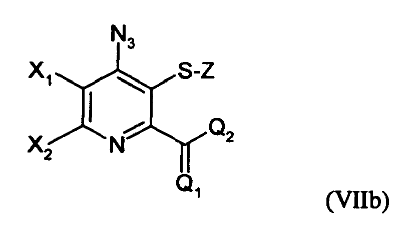 dans lesquels X₁, X₂, Q1, Q2, et Z sont tels que définis dans la revendication 1,
par réaction avec un sel de l'acide azothydrique, dans un solvant polaire aprotique, au reflux ou à une température comprise entre 20°C et 200°C,
les composés de formule (VIIb) pouvant être ensuite hydrolysés en composés de formule (VIIIb) : 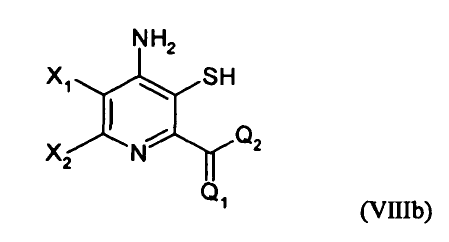 dans lesquels X₁, X₂, Q₁ et Q₂ sont tels que définis dans la revendication 1,
par action d'un agent réducteur, en présence d'un catalyseur.

12. Procédé de préparation des composés selon l'une des revendications 1 à 9, **caractérisé en ce que** les composés de formules (Va), (VIa) et (VIIIb) selon les revendications 10 et 11 sont mis en contact d'un agent acylant en présence d'un solvant et éventuellement d'une base, afin de conduire aux composés de formules (IX₁) et (IX₂) : 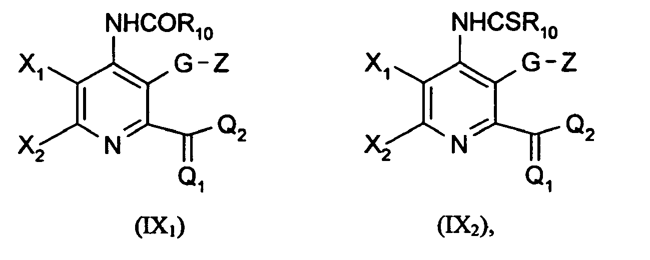 dans lesquels G, X₁, X₂, Q₁, Q₂, Z et R₁₀ sont tels que définis dans la revendication 1, les composés de formules (VIa) et (IVb) pouvant également être éventuellement soumis à diverses réactions de substitution et/ou d'addition bien connues de l'homme du métier pour conduire à l'ensemble des composés de formule (X) : 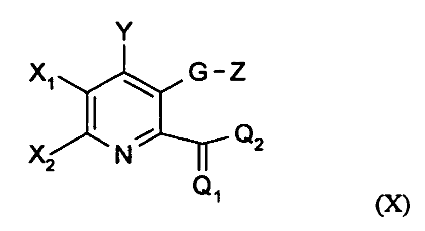 dans lesquels G, X₁, X₂, Q₁, Q₂, Y et Z sont tels que définis dans la revendication 1, cas particulier des composés de formule (I) pour lesquels n représente zéro,
les composés de formule générale (XI) : 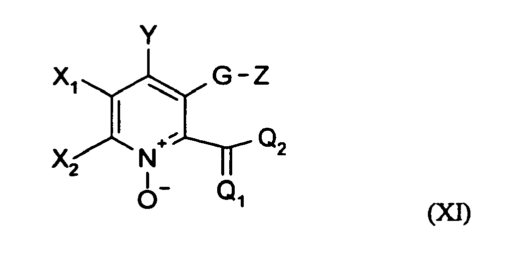 cas particuliers des composés selon la revendication 1 pour lesquels n est égal à 1, pouvant être obtenus par un procédé consistant à mettre en contact un composé de formule (X), avec un agent oxydant.

13. Compositions fongicides comprenant comme matière active une quantité efficace d'au moins un composé selon l'une quelconque des revendications 1 à 9 ou un de leurs N-oxydes, isomères géométriques et/ou optiques, énantiomères et/ou diastéréoisomères, formes tautomères, sels, complexes métalliques et métalloïdiques, acceptables en agriculture.

14. Compositions fongicides selon la revendication 13 comprenant, outre la matière active selon l'une quelconque des revendications 1 à 9 ou un de leurs N-oxydes, isomères géométriques et/ou optiques, énantiomères et/ou diastéréoisomères, formes tautomères, sels, complexes métalliques et métalloïdiques, acceptables en agriculture, un support solide ou liquide, acceptable en agriculture et/ou un agent tensioactif également acceptable en agriculture, ainsi qu'éventuellement un ou plusieurs autres fongicides, insecticides, herbicides, acaricides attractants ou phéromones et autres composés à activité biologique.

15. Procédé de lutte à titre préventif ou curatif contre les champignons phytopathogènes des cultures, **caractérisé en ce que** l'on applique sur le sol où poussent ou où sont susceptibles de pousser les végétaux, sur les feuilles, les troncs et/ou les fruits des végétaux ou sur les semences des végétaux, une quantité efficace (agronomiquement efficace) et non phytotoxique d'une matière active de formule (I) selon l'une des revendications 1 à 9, ou d'une composition fongicide comprenant une matière active de formule (I).

## Claims

1. Compounds of general formula (I): 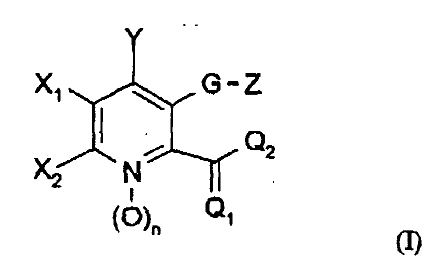 in which:
• G represents an oxygen or sulphur atom,
• n represents 0 or 1,
• Q₁ is an oxygen atom,
• Q₂ is the group -NR₄R₅,
• Z is chosen from a hydrogen atom, a cyano radical and an alkyl, allyl, aryl, arylalkyl, propargyl, cycloalkyl, halocycloalkyl, alkenyl, alkynyl, cyanoalkyl, haloalkyl, alkoxyalkyl, haloalkoxyalkyl, alkylthioalkyl, haloalkylthioalkyl, N-alkylaminoalkyl, N,N-dialkylaminoalkyl, acylaminoalkyl, alkoxycarbonylaminoalkyl, aminocarbonylaminoalkyl, alkoxycarbonyl, N-alkylaminocarbonyl, N,N-dialkylaminocarbonyl, acyl, thioacyl, alkoxythiocarbonyl, N-alkylaminothiocarbonyl, N,N-dialkylaminothiocarbonyl, alkylsulphinyl, haloalkylsulphinyl, alkylsulphonyl, haloalkylsulphonyl, alkoxysulphonyl, aminosulphonyl, N-alkylaminosulphonyl, N,N-dialkylaminosulphonyl, arylsulphinyl, arylsulphonyl, aryloxysulphonyl, N-arylaminosulphonyl, N,N-diarylaminosulphonyl or N,N-arylalkylaminosulphonyl radical;
• Y is chosen from a halogen atom, a hydroxyl, mercapto, nitro, thiocyanato, azido, cyano or pentafluorosulphonyl radical, an alkyl, haloalkyl, alkylthio, haloalkylthio, alkoxyalkyl, haloalkoxyalkyl, alkylthioalkyl, haloalkylthioalkyl, cyanoalkyl, cyanoalkoxy, cyanoalkylthio, alkylsulphinyl, haloalkylsulphinyl, alkylsulphonyl, haloalkylsulphonyl or alkoxysulphonyl radical, a cycloalkyl, halocycloalkyl, alkenyl, alkynyl, alkenyloxy, alkynyloxy, alkenylthio or alkynylthio group, an amino, N-alkylamino, N,N-dialkylamino, -NHCOR₁₀, -NHCSR₁₀, N-alkylaminocarbonylamino, N,N-dialkylaminocarbonylamino, aminoalkyl, N-alkylaminoalkyl, N,N-dialkylaminoalkyl, acylaminoalkyl, thioacylamino, alkoxythiocarbonylamino, N-alkylaminothiocarbonylamino, N,N-dialkylaminothiocarbonylamino, N,N-arylalkylaminocarbonylamino, N-alkylsulphinylamino, N-alkylsulphonylamino, N-alkyl(alkylsulphonyl)amino, N-arylsulphinylamino, N-arylsulphonylamino, N-alkoxysulphonylamino, N-alkoxysulphinylamino, N-haloalkoxysulphinylamino, N-haloalkoxysulphonylamino, N-arylamino, N,N-diarylamino, arylcarbonylamino, alkoxycarbonylamino, N-arylaminocarbonylamino, N,N-diarylaminocarbonylamino, arylthiocarbonylamino, aryloxythiocarbonylamino, N-arylaminothiocarbonylamino, N,N-diarylaminothiocarbonylamino or N,N-arylalkylaminothiocarbonylamino group, an acyl, carboxyl, carbamoyl, N-alkylcarbamoyl, N,N-dialkylcarbamoyl, lower alkoxycarbonyl, N-arylcarbamoyl, N,N-diarylcarbamoyl, aryloxycarbonyl or N,N-arylalkylcarbamoyl group, and an imino group of formula: 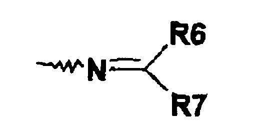
• X₁ and X₂ are identical or different and chosen, independently of each other, from a hydrogen atom, a halogen atom, a hydroxyl, mercapto, nitro, thiocyanato, azido, cyano or pentafluorosulphonyl radical, and an alkyl, haloalkyl, alkoxy, haloalkoxy, alkylthio, haloalkylthio, alkoxyalkyl, haloalkoxyalkyl, alkylthioalkyl, haloalkylthioalkyl, cyanoalkyl, cyanoalkoxy, cyanoalkylthio, alkylsulphinyl, haloalkylsulphinyl, alkylsulphonyl, haloalkylsulphonyl or alkoxysulphonyl radical, or
• X₁ and X₂ may also be joined together, thus forming a saturated, partially unsaturated or totally unsaturated 4- to 8-membered ring optionally comprising one or more hetero atoms chosen from sulphur, oxygen, nitrogen and phosphorus,
• R₄ and R₅ are identical or different and chosen, independently of each other, from a hydrogen atom and an aryl radical chosen, independently from one another, optionally substituted with one or more radicals from R₉, aryl, arylalkyl and a group -T-R₈,
• T represents a direct bond or a divalent radical chosen from a radical -(CH₂)ₘ-, m taking a value between 1 and 12, limits included, the said radical optionally being interrupted or ending with one or two hetero atoms chosen from nitrogen, oxygen and/or sulphur, and an oxyalkylene, alkoxyalkylene, carbonyl (-CO-), oxycarbonyl (-O-CO-), carbonyloxy (-CO-O-), sulphinyl (-SO-), sulphonyl (-SO₂-), oxysulphonyl (-O-SO₂-), sulphonyloxy (-SO₂-O-), oxysulphinyl (-O-SO-), sulphinyloxy (-SO-O-), thio (-S-), oxy (-O-), vinyl (-C=C-), ethynyl (-C≡C-), -NR₉-, -NR₉O-, -ONR₉-, -N=N-, -NR₉-NR₁₀-, -NR₉-S-, -NR₉-SO-, -NR₉-SO₂-, -S-NR₉-, -SO-NR₉-, -SO₂-NR₉-, -CO-NR₉-O- or -O-NR₉-CO- radical,
• R₈ is chosen from a hydrogen atom and an aryl or heterocyclyl radical,
• R₉ and R₁₀, which may be identical or different, are chosen, independently of each other, from a hydrogen atom, a halogen atom, a hydroxyl, mercapto, nitro, thiocyanato, azido, cyano or pentafluorosulphonyl radical, and an alkyl, haloalkyl, alkoxy, haloalkoxy, alkylthio, haloalkylthio, alkoxyalkyl, haloalkoxyalkyl, alkylthioalkyl, haloalkylthioalkyl, arylalkyl, cyanoalkyl, cyanoalkoxy, cyanoalkylthio, alkylsulphinyl, haloalkylsulphinyl, alkylsulphonyl, haloalkylsulphonyl or alkoxysulphonyl radical,
as well as the optional N-oxides, geometrical and/or optical isomers, enantiomers and/or diastereoisomers, tautomeric forms, salts and metal and metalloid complexes of the compounds of formula (I) as have just been defined,
with the restriction that when G-Z is a hydroxyl group, R4 is a hydrogen atom and R5 represents the group 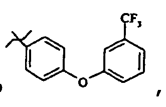 then Y cannot be either a methyl group or a methylthio group.

2. Compounds according to Claim 1 for which X₁ and X₂ each represent a hydrogen atom.

3. Compounds according to Claim 1, for which Q₁ is chosen from an oxygen atom and a sulphur atom.

4. Compounds according to Claim 1, for which Z is chosen from an alkyl radical and a hydrogen atom or an alkoxyalkyl, haloalkoxyalkyl, alkylthioalkyl, haloalkylthioalkyl, N-alkylaminoalkyl, N,N-dialkylaminoalkyl, acylaminoalkyl, acyl, thioacyl, cyanoalkyl, alkoxythiocarbonyl, N-alkylaminothiocarbonyl, N,N-dialkylaminothiocarbonyl or alkylsulphinyl radical.

5. Compounds according to Claim 1, for which Y is chosen from a halogen atom, a hydroxyl, mercapto, nitro, thiocyanato, azido, cyano or pentafluorosulphonyl radical, an alkyl, haloalkyl, alkylthio, haloalkylthio, alkoxyalkyl, haloalkoxyalkyl, alkylthioalkyl, haloalkylthioalkyl, alkylsulphinyl, haloalkylsulphinyl, alkylsulphonyl, haloalkylsulphonyl or alkoxysulphonyl radical, an amino group, and an N-alkylamino, N,N-dialkylamino, -NHCOR₁₀, -NHCSR₁₀, N-arylamino, N,N-diarylamino, arylcarbonylamino, arylthiocarbonylamino, aryloxythiocarbonylamino or N,N-arylalkylaminothiocarbonylamino group.

6. Compounds according to Claim 1 having the following characteristics, taken individually or in combination:
• X₁ and X₂ each represent a hydrogen atom,
• Z is chosen from an alkyl radical and a hydrogen atom or a cleavable radical capable of donating hydrogen, for example an alkoxyalkyl, haloalkoxyalkyl, alkylthioalkyl, haloalkylthioalkyl, N-alkylaminoalkyl, N,N-dialkylaminoalkyl, acylaminoalkyl, acyl, thioacyl, cyanoalkyl, alkoxythiocarbonyl, N-alkylaminothiocarbonyl, N,N-dialkylaminothiocarbonyl or alkylsulphinyl radical,
• Y is chosen from a halogen atom, a hydroxyl, mercapto, nitro, thiocyanato, azido, cyano or pentafluorosulphonyl radical, an alkyl, haloalkyl, alkylthio, haloalkylthio, alkoxyalkyl, haloalkoxyalkyl, alkylthioalkyl, haloalkylthioalkyl, alkylsulphinyl, haloalkylsulphinyl, alkylsulphonyl, haloalkylsulphonyl or alkoxysulphonyl radical, an amino group, and an N-alkylamino, N,N-dialkylamino, -NHCOR₁₀, -NHCSR₁₀, N-arylamino, N,N-diarylamino, arylcarbonylamino, arylthiocarbonylamino, aryloxythiocarbonylamino or N,N-arylalkylaminothiocarbonylamino group,
• Q₁ is chosen from an oxygen atom and a sulphur atom.

7. Compounds according to Claim 1 or Claim 6, having the following characteristics:
• X₁ and X₂ each represent a hydrogen atom,
• Z is chosen from an alkyl radical and a hydrogen atom or a cleavable radical capable of donating hydrogen, for example an alkoxyalkyl, haloalkoxyalkyl, alkylthioalkyl, haloalkylthioalkyl, N-alkylaminoalkyl, N,N-dialkylaminoalkyl, acylaminoalkyl, acyl, thioacyl, cyanoalkyl, alkoxythiocarbonyl, N-alkylaminothiocarbonyl, N,N-dialkylaminothiocarbonyl or alkylsulphinyl radical,
• Y is chosen from a halogen atom, a hydroxyl, mercapto, nitro, thiocyanato, azido, cyano or pentafluorosulphonyl radical, an alkyl, haloalkyl, alkylthio, haloalkylthio, alkoxyalkyl, haloalkoxyalkyl, alkylthioalkyl, haloalkylthioalkyl, alkylsulphinyl, haloalkylsulphinyl, alkylsulphonyl, haloalkylsulphonyl or alkoxysulphonyl radical, an amino group, and an N-alkylamino, N,N-dialkylamino, -NHCOR₁₀, -NHCSR₁₀, N-arylamino, N,N-diarylamino, arylcarbonylamino, arylthiocarbonylamino, aryloxythiocarbonylamino or N,N-arylalkylaminothiocarbonylamino group,
• Q₁ is chosen from an oxygen atom and a sulphur atom.

8. Compounds according to one of the preceding claims, having the following characteristics:
• X₁ and X₂ each represent a hydrogen atom,
• Z is chosen from an alkyl radical and a hydrogen atom or a cleavable radical capable of donating hydrogen, for example an alkoxyalkyl, haloalkoxyalkyl, alkylthioalkyl, haloalkylthioalkyl, N-alkylaminoalkyl, N,N-dialkylaminoalkyl, acylaminoalkyl, acyl, thioacyl, cyanoalkyl, alkoxythiocarbonyl, N-alkylaminothiocarbonyl, N,N-dialkylaminothiocarbonyl or alkylsulphinyl radical,
• Y is chosen from a halogen atom, a hydroxyl, azido, alkylthio and alkylsulphonyl radical and an amino, -NHCOR₁₀ and -NHCSR₁₀ group,
• Q₁ represents an oxygen atom.

9. Compounds according to one of the preceding claims, which are:
• 4-amino-3-hydroxy-N-[4-(4-methylphenoxy)phenyl]-2-pyridinecarboxamide,
• 4-(formylamino)-3-hydroxy-N-{4-[3-(trifluorometh yl)phenoxy]phenyl}-2-pyridinecarboxamide,
• 4-amino-3-hydroxy-N-{4-[4-(trifluoromethyl)pheno xy]phenyl}-2-pyridinecarboxamide,
• N-[4-(4-chlorophenoxy)phenyl]-4-(formylamino) -3-hydroxy-2-pyridinecarboxamide,
• 4-(formylamino)-3-hydroxy-N-{4-[4-(trifluorometh yl)phenoxy]phenyl}-2-pyridinecarboxamide and
• N-[4-(benzyloxy)phenyl]-4-(formylamino)-3-hydrox y-2-pyridinecarboxamide.

10. Process for preparing the compounds according to one of Claims 1 to 9, in which G represents oxygen, **characterized in that** a compound of formula (IIa): 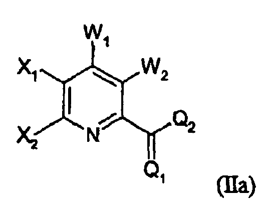 in which X₁, X₂, Q₁ and Q₂ are as defined in Claim 1, and W₁ and W₂, which may be identical or different, represent, independently of each other, a halogen atom chosen from fluorine, chlorine, bromine and iodine, is reacted with an azothydric acid salt, this reaction being carried out in a polar aprotic solvent, at reflux or at a temperature of between 20°C and 200°C, to give the compounds of formula (IIIa): 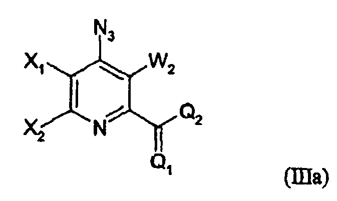 in which X₁, X₂, Q₁ and Q₂ are as defined above, and W₂ represents a halogen atom chosen from fluorine, chlorine, bromine and iodine,
which products are then optionally reduced to the corresponding amine derivatives of formula (IVa): 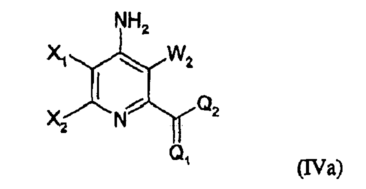 in which X₁, X₂, Q₁ and Q₂ are as defined above and W₂ represents a halogen atom chosen from fluorine, chlorine, bromine and iodine,
by the action of a reducing agent, in the presence of a catalyst,
the halo derivatives of formula (IVa) then possibly being hydrolysed to 3-hydroxypicolinic derivatives of formula (Va): 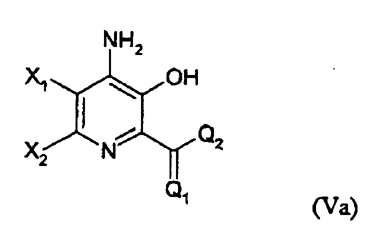 in which X₁, X₂, Q₁ and Q₂ are as defined above,
by the action of an inorganic base, this reaction generally being carried out at a temperature of between 0°C and the boiling point of the solvent, in a polar aprotic dipolar solvent,
the compounds of formula (Va) then possibly being subjected to various alkylation reactions that are well known to those skilled in the art, so as to give the compounds of formula (VIa): 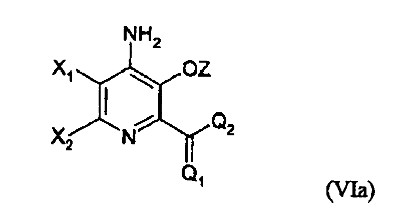 in which X₁, X₂, Z, Q₁ and Q₂ are as defined in Claim 1.

11. Process for preparing the compounds according to one of Claims 1 to 9, in which G represents sulphur, **characterized in that** a compound of formula (IIb) : 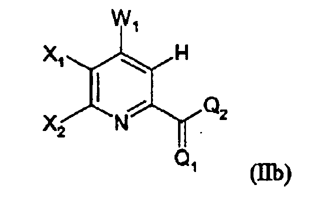 in which X₁, X₂, Q₁ and Q₂ are as defined in Claim 1, and W₁ represents a halogen atom chosen from fluorine, chlorine, bromine and iodine,
is reacted with an organic base, to give the compounds of formula (IIIb): 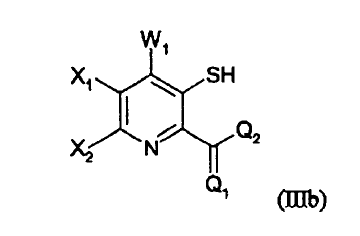 in which X₁, X₂, W₁, Q₁ and Q₂ are as defined in Claim 1, the thiols of formula (IIIb) then possibly being converted into compounds of formula (IVb): 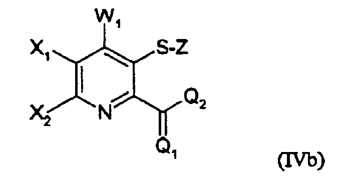 in which X₁, X₂, W₁, Q₁ and Q₂ are as defined in Claim 1, by reaction with an alkylating agent, the compounds of formula (IVb) then possibly being converted into 4-amino compounds of formula (Vb): 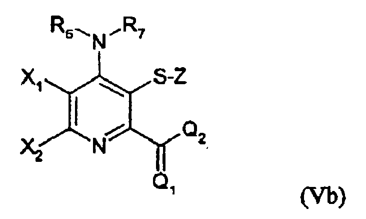 in which X₁, X₂, Q₁, Q₂, Z, R₆ and R₇ are as defined in Claim 1,
by reacting a compound of formula R₆R₇NH, or a corresponding alkali metal or alkaline-earth metal salt, in the absence of solvent or in a polar aprotic solvent, at a temperature of between 0°C and the boiling point of the solvent,
the compounds of formula (Vb) in which X₁, X₂, Q₁, Q₂, R₆ and R₇ are as defined in Claim 1, and Z represents a 4-methoxybenzilic group possibly being converted into the corresponding 3-thiopyridine by reaction with an organic acid, this reaction being carried out in a polar protic solvent, at reflux or at a temperature of between 20°C and 200°C,
to give the compounds of formula (VIb): 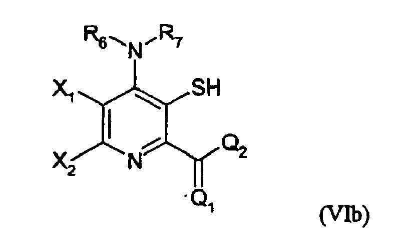 in which X₁, X₂, Q₁, Q₂, R₆ and R₇ are as defined in Claim 1,
the compounds of formula (IVb) also possibly being converted into azides of formula (VIIb): 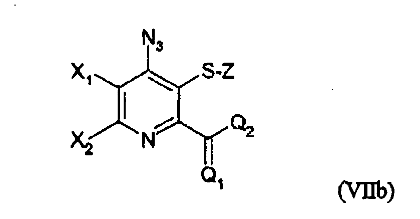 in which X₁, X₂, Q₁, Q₂ and Z are as defined in Claim 1, by reaction with an azothydric acid salt, in a polar aprotic solvent, at reflux or at a temperature of between 20°C and 200°C,
the compounds of formula (VIIb) then possibly being hydrolysed into compounds of formula (VIIIb): 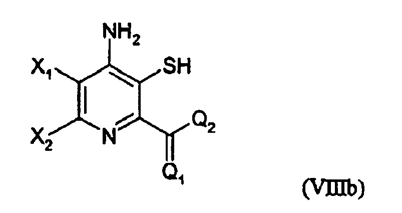 in which X₁, X₂, Q₁ and Q₂ are as defined in Claim 1, by the action of a reducing agent, in the presence of a catalyst.

12. Process for preparing the compounds according to one of Claims 1 to 9, **characterized in that** the compounds of formulae (Va), (VIa) and (VIIIb) according to Claims 10 and 11 are placed in contact with an acylating agent in the presence of a solvent and optionally of a base, in order to give the compounds of formulae (IX₁) and (IX₂) : 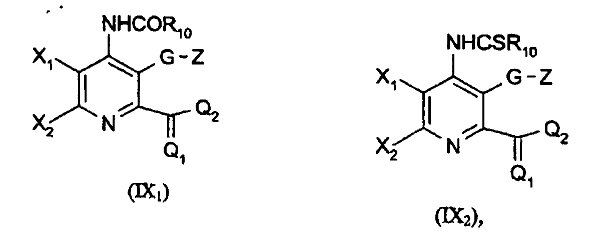 in which G, X₁, X₂, Q₁, Q₂, Z and R₁₀ are as defined in Claim 1,
the compounds of formulae (VIa) and (IVb) also possibly being optionally subjected to various substitution and/or addition reactions that are well known to those skilled in the art, to give the set of compounds of formula (X) : 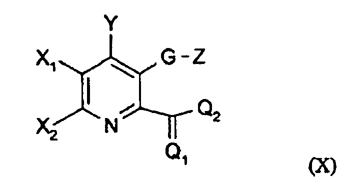 in which G, X₁, X₂, Q₁, Q₂, Y and Z are as defined in Claim 1,
which is a special case of the compounds of formula (I) for which n represents zero,
the compounds of general formula (XI): 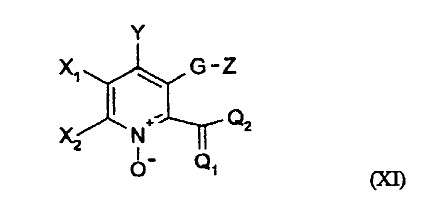 which are special cases of the compounds according to Claim 1 for which n is equal to 1,
possibly being obtained by a process which consists in placing a compound of formula (X) in contact with an oxidizing agent.

13. Fungicidal compositions, comprising, as active material, an effective amount of at least one compound according to any one of Claims 1 to 9 or one of the N-oxides, geometrical and/or optical isomers, enantiomers and/or diastereoisomers, tautomeric forms, salts and metal and metalloid complexes thereof, that are agriculturally acceptable.

14. Fungicidal compositions according to Claim 13, comprising, besides the active material according to any one of Claims 1 to 9 or one of the N-oxides, geometrical and/or optical isomers, enantiomers and/or diastereoisomers, tautomeric forms, salts and metal and metalloid complexes thereof, that are agriculturally acceptable, an agriculturally acceptable solid or liquid support and/or a surfactant which is also agriculturally acceptable, as well as, optionally, one or more other fungicides, insecticides, herbicides, acaricides, attractants or pheromones and other compounds with biological activity.

15. Process for preventively or curatively controlling the phytopathogenic fungi of crops, **characterized in that** an effective (agronomically effective) and non-phytotoxic amount of an active material of formula (I) according to one of Claims 1 to 9, or of a fungicidal composition comprising an active material of formula (I), is applied to the soil in which the plants grow or in which they are liable to grow, to the leaves, the trunks and/or the fruit of the plants or to the plant seeds.

## Patentansprüche

1. Verbindungen der allgemeinen Formel (I): 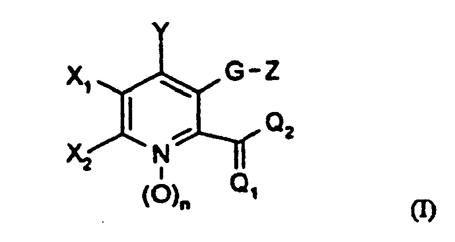 worin:
• G für ein Sauerstoff- oder Schwefelatom steht,
• n für 0 oder 1 steht,
• Q₁ ein Sauerstoffatom ist,
• Q₂ die Gruppe -NR₄R₅ ist,
• Z ausgewählt ist aus einem Wasserstoffatom, einem Cyanorest, einem Alkyl-, Allyl-, Aryl-, Arylalkyl-, Propargyl-, Cycloalkyl-, Halogencycloalkyl-, Alkenyl-, Alkinyl-, Cyanoalkyl-, Halogenalkyl-, Alkoxyalkyl-, Halogenalkoxyalkyl-, Alkylthioalkyl-, Halogenalkylthioalkyl-, N-Alkylaminoalkyl-, N,N-Dialkylaminoalkyl-, Acylaminoalkyl-, Alkoxycarbonylaminoalkyl-, Aminocarbonylaminoalkyl-, Alkoxycarbonyl-, N-Alkylaminocarbonyl-, N,N-Dialkylaminocarbonyl-, Acyl-, Thioacyl-, Alkoxythiocarbonyl-, N-Alkylaminothiocarbonyl-, N,N-Dialkylaminothiocarbonyl-, Alkylsulfinyl-, Halogenalkylsulfinyl-, Alkylsulfonyl-, Halogenalkylsulfonyl-, Alkoxysulfonyl-, Aminosulfonyl-, N-Alkylaminosulfonyl-, N,N-Dialkylaminosulfonyl-, Arylsulfinyl-, Arylsulfonyl-, Aryloxysulfonyl-, N-Arylaminosulfonyl-, N,N-Diarylaminosulfonyl- und N,N-Arylalkylaminosulfonylrest;
• Y ausgewählt ist aus einem Halogenatom, einem Hydroxy-, Mercapto-, Nitro-, Thiocyanato-, Azido-, Cyano-, Pentafluorsulfonylrest, einem Alkyl-, Halogenalkyl-, Alkylthio-, Halogenalkylthio-, Alkoxyalkyl-, Halogenalkoxyalkyl-, Alkylthioalkyl-, Halogenalkylthioalkyl-, Cyanoalkyl-, Cyanoalkoxy-, Cyanoalkylthio-, Alkylsulfinyl-, Halogenalkylsulfinyl-, Alkylsulfonyl-, Halogenalkylsulfonyl-, Aoxysulfonylrest, einer Cycloalkyl-, Halogencycloalkyl-, Alkenyl-, Alkinyl-, Alkenyloxy-, Alkinyloxy-, Alkenylthio-, Akinylthiogruppe, einer Amino-, N-Alkylamino-, N,N-Dialkylamino-, -NHCOR₁₀-, -NHCSR₁₀-, N-Alkylaminocarbonylamino-, N,N-Dialkylaminocarbonylamino-, Aminoalkyl-, N-Alkylaminoalkyl-, N,N-Dialkylaminoalkyl-, Acylaminoalkyl-, Thioacylamino-, Alkoxythiocarbonylamino-, N-Alkylaminothiocarbonylamino-, N,N-Dialkylaminothiocarbonylamino-, N,N-Arylalkylaminocarbonylamino-, N-Alkylsulfinylamino-, N-Alkylsulfonylamino-, N-Alkyl(alkylsulfonyl)amino-, N-Arylsulfinylamino-, N-Arylsulfonylamino-, N-Alkoxysulfonylamino-, N-Alkoxysulfinylamino-, N-Halogenalkoxysulfinylamino-, N-Halogenalkoxysulfonylamino-, N-Arylamino-, N,N-Diarylamino-, Arylcarbonylamino-, Alkoxycarbonylamino-, N-Arylaminocarbonylamino-, N,N-Diarylaminocarbonylamino-, Arylthiocarbonylamino-, Aryloxythiocarbonylamino-, N-Arylaminothiocarbonylamino-, N,N-Diarylaminothiocarbonylamino-, N,N-Arylalkylaminothiocarbonylaminogruppe, einem Acyl-, Carboxy-, Carbamoyl-, N-Alkylcarbamoyl-, N,N-Dialkylcarbamoyl-, Niederalkoxycarbonyl-, N-Arylcarbamoyl-, N,N-Diarylcarbamoyl-, Aryloxycarbonyl-, N,N-Arylalkylcarbamoylrest und einer Iminogruppe der Formel: 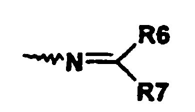
• X₁ und X₂ gleich oder verschieden sind und unabhängig voneinander ausgewählt sind aus einem Wasserstoffatom, einem Halogenatom, einem Hydroxy-, Mercapto-, Nitro-, Thiocyanato-, Azido-, Cyano-, Pentafluorsulfonylrest, einem Alkyl-, Halogenalkyl-, Alkoxy-, Halogenalkoxy-, Alkylthio-, Halogenalkylthio-, Alkoxyalkyl-, Halogenalkoxyalkyl-, Alkylthioalkyl-, Halogenalkylthioalkyl-, Cyanoalkyl-, Cyanoalkoxy-, Cyanoalkylthio-, Alkylsulfinyl-, Halogenalkylsulfinyl-, Alkylsulfonyl-, Halogenalkylsulfonyl- und Alkoxysulfonylrest oder
• X₁ und X₂ auch miteinander verbunden sein können, so dass ein 4- bis 8-gliedriger gesättigter, teilweise ungesättigter oder vollständig ungesättigter Ring gebildet wird, der gegebenenfalls ein oder mehrere Heteroatome enthält, die aus Schwefel, Sauerstoff, Stickstoff und Phosphor ausgewählt sind,
• R₄ und R₅ gleich oder verschieden sind und unabhängig voneinander ausgewählt sind aus einem Wasserstoffatom und einem Arylrest, der gegebenenfalls mit einem oder mehreren Resten substituiert ist, die unabhängig voneinander aus R₉, Aryl, Arylalkyl und einer Gruppe -T-R₈ ausgewählt sind,
• T für eine direkte Bindung oder einen zweiwertigen Rest, der ausgewählt ist aus einem Rest -(CH₂)ₘ-, wobei m einen Wert zwischen 1 und 12, einschließlich der Grenzen, annimmt, wobei der Rest gegebenenfalls durch ein oder zwei Heteroatome, die aus Stickstoff, Sauerstoff und/oder Schwefel ausgewählt sind, unterbrochen oder begrenzt sein kann, einen Oxyalkylen-, Alkoxyalkylen-, Carbonyl- (-CO-), Oxycarbonyl- (-O-CO-), Carbonyloxy- (-CO-O-), Sulfinyl- (-SO-), Sulfonyl- (-SO₂-), Oxysulfonyl- (-O-SO₂-), Sulfonyloxy- (-SO₂-O-), Oxysulfinyl- (-O-SO-), Sulfinyloxy- (-SO-O-), Thio- (-S-), Oxy- (-O-), Vinyl- (-C=C-), Ethinyl (-C≡C-), -NR₉-, -NR₉O-, -ONR₉-, -N=N-, -NR₉-NR₁₀-, -NR₉-S-, -NR₉-SO-, -NR₉-SO₂-, -S-NR₉-, -SO-NR₉-, -SO₂-NR₉-, -CO-NR₉-O- und -O-NR₉-CO-Rest steht,
• R₈ aus einem Wasserstoffatom, einem Arylrest oder einem Heterocyclus ausgewählt ist,
• R₉ und R₁₀, die gleich oder verschieden sind, unabhängig von einander ausgewählt sind aus einem Wasserstoffatom, einem Halogenatom, einem Hydroxy-, Mercapto-, Nitro-, Thiocyanato-, Azido-, Cyano- oder Pentafluorsulfonylrest, einem Alkyl-, Halogenalkyl-, Alkoxy-, Halogenalkoxy-, Alkylthio-, Halogenalkylthio-, Alkoxyalkyl-, Halogenalkoxyalkyl-, Alkylthioalkyl-, Halogenalkylthioalkyl-, Arylalkyl-, Cyanoalkyl-, Cyanoalkoxy-, Cyanoalkylthio-, Alkylsulfinyl-, Halogenalkylsulfinyl-, Alkylsulfonyl-, Halogenalkylsulfonyl- und Alkoxysulfonylrest,
sowie gegebenenfalls die N-Oxide, geometrischen und/oder optischen Isomere, Enantiomere und/oder Diastereoisomere, tautomeren Formen, die Salze, Metall- und metallartigen Komplexe der Verbindungen der Formel (I), wie definiert,
mit der Beschränkung, dass, wenn G-Z eine Hydroxygruppe ist, R₄ ein Wasserstoffatom ist und R₅ die Gruppe 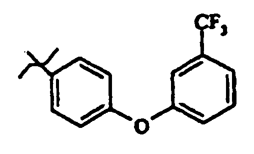 darstellt, Y weder eine Methylnoch eine Methylthiogruppe sein kann.

2. Verbindungen nach Anspruch 1, wobei X₁ und X₂ jeweils ein Wasserstoffatom darstellen.

3. Verbindungen nach Anspruch 1, wobei Q₁ aus einem Sauerstoff- und einem Schwefelatom ausgewählt ist.

4. Verbindungen nach Anspruch 1, wobei Z aus einem Alkylrest und einem Wasserstoffatom oder einem Alkoxyalkyl-, Halogenalkoxyalkyl-, Alkylthioalkyl-, Halogenalkylthioalkyl-, N-Alkylaminoalkyl-, N,N-Dialkylaminoalkyl-, Acylaminoalkyl-, Acyl-, Thioacyl-, Cyanoalkyl-, Alkoxythiocarbonyl-, N-Alkylaminothiocarbonyl-, N,N-Dialkylaminothiocarbonyl- oder Alkylsulfinylrest ausgewählt ist.

5. Verbindungen nach Anspruch 1, wobei Y aus einem Halogenatom, einem Hydroxy-, Mercapto-, Nitro-, Thiocyanato-, Azido-, Cyano-, Pentafluorsulfonyrest, einem Alkyl-, Halogenalkyl-, Alkylthio-, Halogenalkylthio-, Alkoxyalkyl-, Halogenalkoxyalkyl-, Alkylthioalkyl-, Halogenalkylthioalkyl-, Alkylsulfinyl-, Halogenalkylsulfinyl-, Alkylsulfonyl-, Halogenalkylsulfonyl-, Alkoxysulfonylrest, einer Amino-, N-Alkylamino-, N,N-Dialkylamino-, -NHCOR₁₀-, -NHCSR₁₀-, N-Arylamino-, N,N-Diarylamino-, Arylcarbonylamino-, Arylthiocarbonylamino-, Aryloxythiocarbonylamino- und N,N-Arylalkylaminothiocarbonylaminogruppe ausgewählt ist.

6. Verbindungen nach Anspruch 1 mit den folgenden Merkmalen allein oder in Kombination:
• X₁ und X₂ stehen jeweils für ein Wasserstoffatom,
• Z ist aus einem Alkylrest und einem Wasserstoffatom oder einem spaltbaren Rest, der wieder Wasserstoff ergeben kann ausgewählt, zum Beispiel Alkoxyalkyl, Halogenalkoxyalkyl, Alkylthioalkyl, Halogenalkylthioalkyl, N-Alkylaminoalkyl, N,N-Dialkylaminoalkyl, Acylaminoalkyl, Acyl, Thioacyl, Cyanoalkyl, Alkoxythiocarbonyl, N-Alkylaminothiocarbonyl, N,N-Dialkylaminothiocarbonyl oder Alkylsulfinyl,
• Y ist aus einem Halogenatom, einem Hydroxy-, Mercapto-, Nitro-, Thiocyanato-, Azido-, Cyano-, Pentafluorsulfonylrest, einem Alkyl-, Halogenalkyl-, Alkylthio-, Halogenalkylthio-, Alkoxyalkyl-, Halogenalkoxyalkyl-, Alkylthioalkyl-, Halogenalkylthioalkyl-, Alkylsulfinyl-, Halogenalkylsulfinyl-, Alkylsulfonyl-, Halogenalkylsulfonyl-, Alkoxysulfonylrest, einer Amino-, N-Alkylamino-, N,N-Dialkylamino-, -NHCOR₁₀-, -NHCSR₁₀-, N-Arylamino-, N,N-Diarylamino-, Arylcarbonylamino-, Arylthio-carbonylamino-, Aryloxythiocarbonylamino-, N,N-Arylalkylaminothiocarbonylaminogruppe ausgewählt,
• Q₁ ist aus einem Sauerstoff- und einem Schwefelatom ausgewählt.

7. Verbindungen nach Anspruch 1 oder Anspruch 6 mit den folgenden Merkmalen:
• X₁ und X₂ stehen jeweils für ein Wasserstoffatom,
• Z ist aus einem Alkylrest und einem Wasserstoffatom oder einem spaltbaren Rest, der wieder Wasserstoff ergeben kann, ausgewählt, zum Beispiel Alkoxyalkyl, Halogenalkoxyalkyl, Alkylthioalkyl, Halogenalkylthioalkyl, N-Alkylaminoalkyl, N,N-Dialkylaminoalkyl, Acylaminoalkyl, Acyl, Thioacyl, Cyanoalkyl, Alkoxythiocarbonyl, N-Alkylaminothiocarbonyl, N,N-Dialkylaminothiocarbonyl oder Alkylsulfinyl,
• Y ist aus einem Halogenatom, einem Hydroxy-, Mercapto-, Nitro-, Thiocyanato-, Azido-, Cyano-, Pentafluorsulfonylrest, einem Alkyl-, Halogenalkyl-, Alkylthio-, Halogenalkylthio-, Alkoxyalkyl-, Halogenalkoxyalkyl-, Alkylthioalkyl-, Halogenalkylthioalkyl-, Alkylsulfinyl-, Halogenalkylsulfinyl-, Alkylsulfonyl-, Halogenalkylsulfonyl-, Alkoxysulfonylrest, einer Amino-, N-Alkylamino-, N,N-Dialkylamino-, -NHCOR₁₀-, -NHCSR₁₀-, N-Arylamino-, N,N-Diarylamino-, Arylcarbonylamino-, Arylthio-carbonylamino-, Aryloxythiocarbonylamino-, N,N-Arylalkylaminothiocarbonylaminogruppe ausgewählt,
• Q₁ ist aus einem Sauerstoff- und einem Schwefelatom ausgewählt.

8. Verbindungen nach einem der vorhergehenden Ansprüche mit den folgenden Merkmalen:
• X₁ und X₂ stehen jeweils für ein Wasserstoffatom,
• Z ist aus einem Alkylrest und einem Wasserstoffatom oder einem spaltbaren Rest ausgewählt, der wieder Wasserstoff ergeben kann, zum Beispiel Alkoxyalkyl, Halogenalkoxyalkyl, Alkylthioalkyl, Halogenalkylthio-alkyl, N-Alkylaminoalkyl, N,N-Dialkylamino-alkyl, Acylaminoalkyl, Acyl, Thioacyl, Cyano-alkyl, Alkoxythiocarbonyl, N-Alkylaminothio-carbonyl, N,N-Dialkylaminothiocarbonyl oder Alkylsulfinyl,
• Y ist ausgewählt aus einem Halogenatom, einem Hydroxy-, Azido-, Alkylthio-, Alkylsulfonylrest, einer Aminogruppe, -NHCOR₁₀ und -NHCSR₁₀,
• Q₁ steht für ein Sauerstoffatom.

9. Verbindungen nach einem der vorhergehenden Ansprüche, die die folgenden sind:
• 4-Amino-3-hydroxy-N-[4-(4-methylphenoxy)-phenyl]-2-pyridincarboxamid,
• 4-(Formylamino)-3-hydroxy-N-{4-[3-(trifluormethyl) phenoxy] phenyl}-2-pyridincarboxamid,
• 4-Amino-3-hydroxy-N-{4-[4-(trifluormethyl)-phenoxy]phenyl}-2-pyridincarboxamid,
• N-[4-(4-Chlorphenoxy)phenyl]-4-(formylamino)-3-hydroxy-2-pyridincarboxamid,
• 4-(Formylamino)-3-hydroxy-N-{4-[4-(trifluormethyl)phenoxy]phenyl}-2-pyridincarboxamid und
• N-[4-(Benzyloxy)phenyl]-4-(formylamino)-3-hydroxy-2-pyridincarboxamid.

10. Verfahren zur Herstellung der Verbindungen nach einem der Ansprüche 1 bis 9, wobei G für Sauerstoff steht, **dadurch gekennzeichnet, dass** eine Verbindung der Formel (IIa): 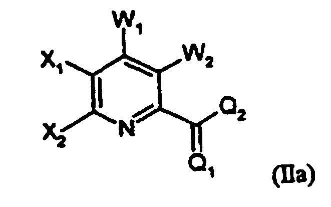 worin X₁, X₂, Q₁ und Q₂ wie in Anspruch 1 definiert sind und W₁ und W₂, die gleich oder verschieden sind, unabhängig voneinander ein Halogenatom darstellen, das aus Fluor, Chlor, Brom oder Iod ausgewählt ist,
mit einem Hydrazonsäuresalz umgesetzt wird, wobei die Umsetzung in einem polaren aprotischen Lösungsmittel unter Rückfluss oder bei einer Temperatur zwischen 20°C und 200°C durchgeführt wird, um die Verbindungen der Formel (IIIa) zu erhalten: 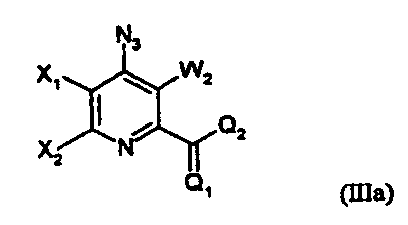 worin X₁, X₂, Q₁ und Q₂ wie vorstehend definiert sind und W₂ ein Halogenatom darstellt, das aus Fluor, Chlor, Brom oder Iod ausgewählt ist,
die anschließend gegebenenfalls durch Einwirkung eines Reduktionsmittels in Gegenwart eines Katalysators zu den Aminoderivaten der Formel (IVa) reduziert werden: 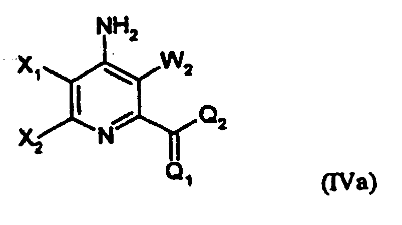 worin X₁, X₂, Q₁ und Q₂ wie vorstehend definiert sind und W₂ ein Halogenatom darstellt, das aus Fluor, Chlor, Brom oder Iod ausgewählt ist,
wobei die Halogenderivate der Formel (IVa) dann durch Einwirkung einer anorganischen Base zu den 3-Hydroxypicolinsäurederivaten der Formel (Va) hydrolysiert werden können: 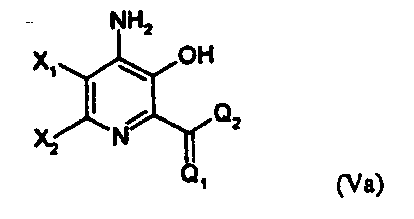 worin X₁, X₂, Q₁ und Q₂ wie vorstehend definiert sind, wobei diese Umsetzung gewöhnlich bei einer Temperatur zwischen 0°C und der Siedetemperatur des Lösungsmittels in einem dipolaren aprotischen polaren Lösungsmittel durchgeführt wird,
wobei die Verbindungen der Formel (Va) anschließend verschiedenen, dem Fachmann bekannten Alkylierungsreaktionen unterzogen werden können, um die Verbindungen der Formel (VIa) zu erhalten: 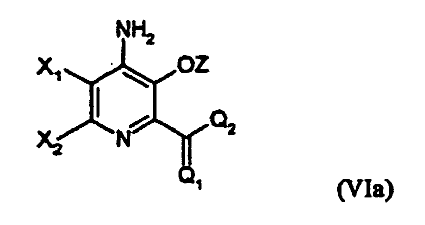 worin X₁, X₂, Z, Q₁ und Q₂ wie im Anspruch 1 definiert sind.

11. Verfahren zur Herstellung der Verbindungen nach einem der Ansprüche 1 bis 9, wobei G für Schwefel steht, **dadurch gekennzeichnet, dass** eine Verbindung der Formel (IIb): 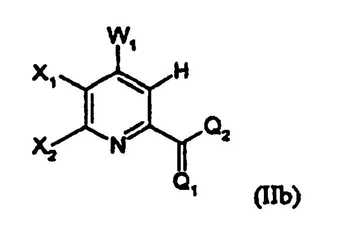 worin X₁, X₂, Q₁ und Q₂ wie in Anspruch 1 definiert sind und W₁ ein Halogenatom darstellt, das aus Fluor, Chlor, Brom oder Iod ausgewählt ist,
mit einer organischen Base umgesetzt wird, um die Verbindungen der Formel (IIIb) zu erhalten: 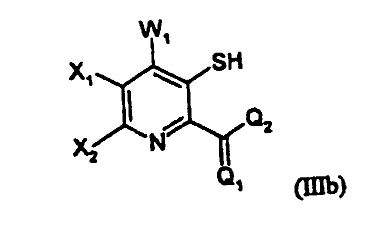 worin X₁, X₂, W₁, Q₁ und Q₂ wie in Anspruch 1 definiert sind,
die Thiole der Formel (IIIb) dann durch Umsetzung mit einem Alkylierungsmittel in Verbindungen der Formel (IVb) umgewandelt werden können: 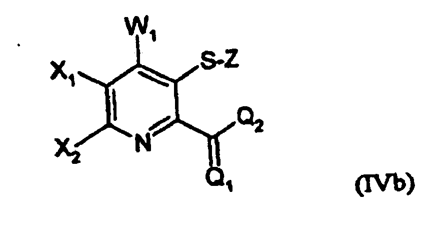 worin X₁, X₂, W₁, Q₁ und Q₂ wie in Anspruch 1 definiert sind,
die Verbindungen der Formel (IVb) dann durch Umsetzen einer Verbindung der Formel R₆R₇NH oder eines entsprechenden Alkali- oder Erdalkalisalzes in Abwesenheit von Lösungsmittel oder in einem aprotischen polaren Lösungsmittel bei einer Temperatur zwischen 0°C und der Siedetemperatur des Lösungsmittels in 4-Aminoverbindungen der Formel (Vb) umgewandelt werden können: 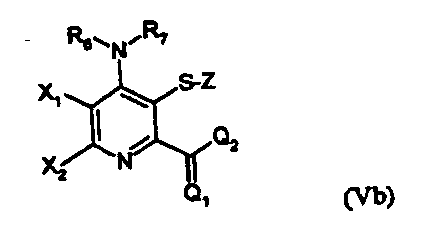 worin X₁, X₂, Q₁, Q₂, Z, R₆ und R₇ wie in Anspruch 1 definiert sind,
die Verbindungen der Formel (Vb), worin X₁, X₂, Q₁, Q₂, R₆ und R₇ wie in Anspruch 1 definiert sind und Z für eine 4-Methoxybenzilgruppe steht, durch Umsetzung mit einer organischen Säure in das entsprechende 3-Thiopyridin umgewandelt werden können, wobei diese Umsetzung in einem polaren protischen Lösungsmittel unter Rückfluss oder bei einer Temperatur zwischen 20°C und 200°C durchgeführt wird,
so dass die Verbindungen der Formel (VIb) erhalten werden: 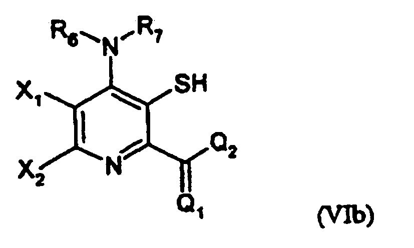 worin X₁, X₂, Q₁, Q₂, R₆ und R₇ wie in Anspruch 1 definiert sind,
die Verbindungen der Formel (IVb) auch durch Umsetzung mit einem Hydrazonsäuresalz in einem polaren aprotischen Lösungsmittel unter Rückfluss oder bei einer Temperatur zwischen 20°C und 200°C in Nitride der Formel (VIIb) umgewandelt werden können: 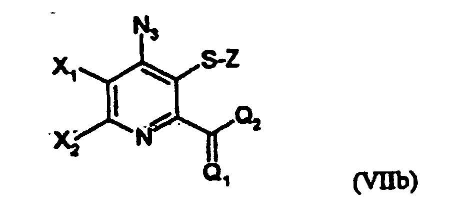 worin X₁, X₂, Q₁, Q₂ und Z wie in Anspruch 1 definiert sind,
wobei die Verbindungen der Formel (VIIb) anschließend durch Einwirkung eines Reduktionsmittels in Gegenwart eines Katalysators zu den Verbindungen der Formel (VIIIb) hydrolysiert werden können: 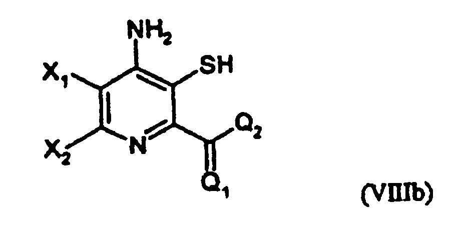 worin X₁, X₂, Q₁ und Q₂ wie in Anspruch 1 definiert sind.

12. Verfahren zur Herstellung der Verbindungen nach einem der Ansprüche 1 bis 9, **dadurch gekennzeichnet, dass** die Verbindungen der Formeln (Va), (VIa) und (VIIIb) nach den Ansprüchen 10 und 11 mit einem Acylierungsmittel in Gegenwart eines Lösungsmittels und gegebenenfalls einer Base in Kontakt gebracht werden, um die Verbindungen der Formeln (IX₁) und (IX₂) zu erhalten: 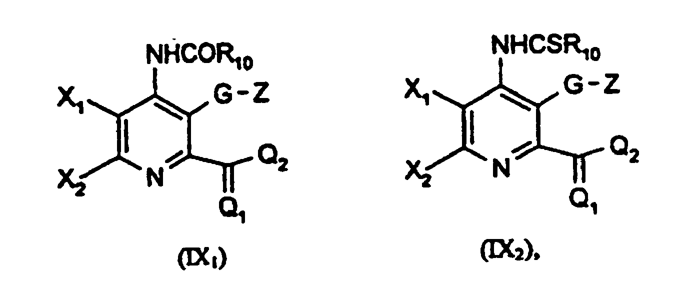 worin G, X₁, X₂, Q₁, Q₂, Z und R₁₀ wie in Anspruch 1 definiert sind,
wobei die Verbindungen der Formeln (VIa) und (IVb) auch gegebenenfalls verschiedenen, dem Fachmann bekannten Substitutions- und/oder Additionsreaktionen unterzogen werden können, um die Gruppe der Verbindungen der Formel (X) zu erhalten: 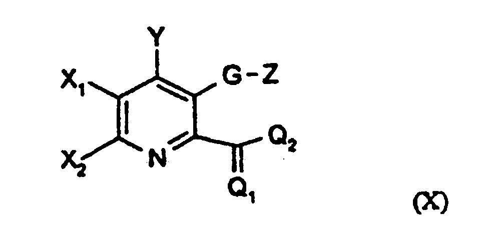 worin G, X₁, X₂, Q₁, Q₂, Y und Z wie in Anspruch 1 definiert sind, die einen Spezialfall der Verbindungen der Formel (I) darstellen, bei denen n für null steht,
wobei die Verbindungen der allgemeinen Formel (XI) : 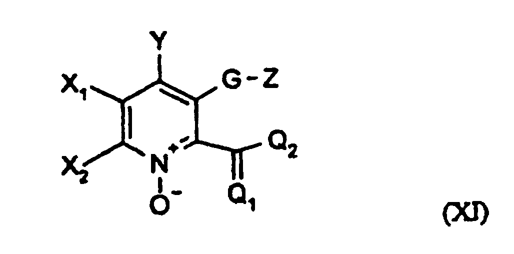 die Spezialfälle der Verbindungen nach Anspruch 1 darstellen, bei denen n gleich 1 ist, durch ein Verfahren erhalten werden können, bei dem eine Verbindung der Formel (X) mit einem Oxidationsmittel in Kontakt gebracht wird.

13. Fungizide Zusammensetzungen, die als Wirkstoff eine wirksame Menge mindestens einer Verbindung nach einem der Ansprüche 1 bis 9 oder ihrer N-Oxide, geometrischen und/oder optischen Isomere, Enantiomere und/oder Diasterisomere, tautomeren Formen, Salze, Metall- oder metallartigen Komplexe, die für die Landwirtschaft annehmbar sind, enthalten.

14. Fungizide Zusammensetzungen nach Anspruch 13, die außer dem Wirkstoff nach einem der Ansprüche 1 bis 9 oder einem seiner N-Oxide, geometrischen und/oder optischen Isomere, Enantiomere und/oder Diastereoisomere, tautomeren Formen, Salze, Metall- oder metallartigen Komplexe, die für die Landwirtschaft annehmbar sind, einen für die Landwirtschaft annehmbaren festen oder flüssigen Träger und/oder ein ebenfalls für die Landwirtschaft annehmbares Tensid sowie gegebenenfalls ein oder mehrere andere Fungizide, Insektizide, Herbizide, Akarizide, Attraktante oder Pheromone und andere Verbindungen mit biologischer Aktivität enthalten.

15. Bekämpfungsverfahren zur Vorbeugung oder Heilung gegen phytopathogene Pilze von Kulturpflanzen, **dadurch gekennzeichnet, dass** man auf den Boden, in dem die Pflanzen wachsen oder wachsen können, die Blätter, die Stengel und/oder die Früchte der Pflanzen oder auf die Samen der Pflanzen eine wirksame (agronomisch wirksame) und nicht phytotoxische Menge eines Wirkstoffs der Formel (I) nach einem der Ansprüche 1 bis 9 oder einer fungiziden Zusammensetzung, die einen Wirkstoff der Formel (I) enthält, aufbringt.
